# EUROPEAN PATENT APPLICATION

(11) **EP 1 972 349 A1**
(43) Date of publication of application: **24.09.2008**
(21) Application number: 07005831.8
(22) Date of filing: 21.03.2007
(51) Int. Cl.: A61K 47/48, C07K 14/605, C07K 19/00

(54) **GLP-1 fusion peptides conjugated to polymer(s), their production and use**

(71) Applicant: Biocompatibles UK Limited, Farnham Surrey GU9 8QL (GB)
(72) Inventor: Geigle, Peter, Dr., 63755 Alzenau (DE); Wallrapp, Christine, Dr., 63762 Großostheim (DE); Thoenes, Eric, 63654 Büdingen (DE)
(74) Representative: Graf von Stosch, Andreas

(57) **Abstract**

The present invention provides fusion peptides having GLP-1 activity and enhanced stability in vivo, in particular resistancy to dipeptidyl peptidase IV conjugated to polymers, thereby forming conjugate molecules. The fusion peptide of the conjugate molecule comprises as component (I) N-terminally a GLP-1(7-35, 7-36 or 7-37) sequence and as component (II) Gterminally a peptide sequence of at least 9 amino acids or a functional fragment, variant or derivative thereof. A synthetic polymer and/or a protein, e.g transferrin or albumin, is covalently or non-covalently bound to the fusion peptide to form the conjugate molecule. Component (II) is preferably a full or partial version of IP2 (intervening peptide 2). A preferred embodiment comprises the sequence GLP-1(7-35, 36 or 37)/IP2/GLP-1(7-35, 36 or 37) or GLP-2 and a polymeric component, e.g. a natural or non-natural polymer. The fusion peptide may be produced in engineered cells or synthetically and is e.g. conjugated to the polymeric component by chemical synthesis. The conjugate molecule may be used for the preparation of a medicament for treating various diseases or disorders, e.g. diabetes type 1 or 2, apoptosis related diseases or neurodegenerative disorders.

SEQUENCE LISTING

<110>  Biocompatibles UK Limited

<120>  GLP-1 fusion peptides conjugated to polymer(s), their production and use

<130>  BI07P004EP

<140>  EP07005831.8
<141>  2007-03-21

<160>  45

<170>  PatentIn version 3.3

<210>  1
<211>  31
<212>  PRT
<213>  Artificial

<220>
<223>  Description of sequence:  synthetic peptide corresponding to
       GLP-1(7-37) (see description p. 20 and 69)

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  15
<212>  PRT
<213>  Artificial

<220>
<223>  Description of sequence: full-length IP-2 sequence having all 15
       amino acids of the naturally occurring IP-2 sequence, human;
       (see description p. 21 and 69)

<400>  2

Arg Arg Asp Phe Pro Glu Glu Val Ala Ile Val Glu Glu Leu Gly
1               5                   10                  15

<210>  3
<211>  15
<212>  PRT
<213>  Artificial

<220>
<223>  Description of sequence: full-length IP-2 sequence having all 15
       amino acids of the naturally occurring IP-2 sequence, murine;
       (see description p. 21 and 69)

<400>  3

Arg Arg Asp Phe Pro Glu Glu Val Ala Ile Ala Glu Glu Leu Gly

1                   5                   10                  15


<210>   4
<211>   35
<212>   PRT
<213>   Artificial

<220>
<223>   Description of sequence: murine isoform of GLP-2
        (see description p. 24 and 69)

<400>   4

His Ala Asp Gly Ser Phe Ser Asp Glu Met Asn Thr Ile Leu Asp Asn
1               5                   10                  15


Leu Ala Ala Arg Asp Phe Ile Asn Trp Leu Ile Gln Thr Lys Ile Thr
            20                  25                  30


Asp Arg Lys
        35


<210>   5
<211>   35
<212>   PRT
<213>   Artificial

<220>
<223>   Description of sequence: human isoform of GLP-2
        (see description p. 24 and 69)

<400>   5

His Ala Asp Gly Ser Phe Ser Asp Glu Met Ser Thr Ile Leu Asp Asn
1               5                   10                  15


Leu Ala Thr Arg Asp Phe Ile Asn Trp Leu Ile Gln Thr Lys Ile Thr
            20                  25                  30


Asp Lys Lys
        35


<210>   6
<211>   79
<212>   PRT
<213>   Artificial

<220>
<223>   Description of sequence: SEQ ID No: 6 (ID6syn, CM1) corresponds
        to GLP-1(7-37)-IP2-RR-GLP1(7-37), 79 aa, 8,7 kD
        (see description p. 25 and 69)

<400>   6

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  7
<211>  83
<212>  PRT
<213>  Artificial

<220>
<223>  Description of sequence: SEQ ID No: 7 (ID7rec, CM2) corresponds
       to GLP-1(7-37)-IP2-RR-GLP2, 83 aa, 9,4 kD
       (see description p. 25 and 69)

<400>  7

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Asp Gly Ser Phe Ser Asp Glu Met Ser Thr Ile Leu Asp Asn
    50                  55                  60

Leu Ala Thr Arg Asp Phe Ile Asn Trp Leu Ile Gln Thr Lys Ile Thr
65                  70                  75                  80

Asp Lys Lys

<210>  8
<211>  46
<212>  PRT
<213>  Artificial

<220>
<223>  Description of sequence: SEQ ID No:8 (ID8 syn, CM3) corresponds
       to GLP-1(7-37)-IP2, 46 aa, 5,1 kD;(see description p. 22 and 69)

<400>  8

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly
        35                  40                  45

<210>  9
<211>  97
<212>  PRT
<213>  Artificial

<220>
<223>  Description of sequence: artificial (see description p. 69)

<400>  9

Met Ala Pro Ala Ala Trp Leu Arg Ser Ala Ala Ala Arg Ala Leu Leu
1               5                   10                  15

Pro Pro Met Leu Leu Leu Leu Leu Gln Pro Pro Pro Leu Leu Ala Arg
            20                  25                  30

Ala Leu Pro Pro Asp Val His His Leu His Ala Glu Arg Arg Gly Pro
        35                  40                  45

Gln Pro Trp His Ala Ala Leu Pro Ser Ser Pro Ala Pro Ala Pro Ala
    50                  55                  60

Thr Gln Glu Ala Pro Arg Pro Ala Ser Ser Leu Arg Pro Pro Arg Cys
65                  70                  75                  80

Gly Val Pro Asp Pro Ser Asp Gly Leu Ser Ala Arg Asn Arg Gln Lys
                85                  90                  95

Arg

<210>  10
<211>  79
<212>  PRT
<213>  Artificial

<220>
<223>  Description of sequence: SEQ ID No: 10
       (N-GLP-1(7-37)-IP2(human)-RR-GLP-1(7-37)-C, also designated human
       CM1), (see description p. 25 and 69)

<400>  10

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  11
<211>  83
<212>  PRT
<213>  Artificial

<220>
<223>  Description of sequence: SEQ ID No: 11
       (N-GLP-1(7-37)-IP2(human)-RR-GLP-2-C), also designated human CM2
       herein), (see description p. 25 and 69)

<400>  11

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Asp Gly Ser Phe Ser Asp Glu Met Asn Thr Ile Leu Asp Asn
    50                  55                  60

Leu Ala Ala Arg Asp Phe Ile Asn Trp Leu Ile Gln Thr Lys Ile Thr
65                  70                  75                  80

Asp Arg Lys

<210>  12
<211>  46
<212>  PRT
<213>  Artificial

<220>
<223>  Description of sequence: SEQ ID No: 12, GLP-1(7-37) linked
       without any linker sequence via its C-terminus to human IP2
       (see description p. 22 and 69)

<400>  12

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly
        35                  40                  45

<210>  13
<211>  815
<212>  DNA
<213>  Artificial

<220>
<223>  Description of sequence: (SEQ ID No:13) represents the
       translated peptide sequence of the construct according to Fig. 1k
       (SEQ ID No: 14), (see description p. 52 and 70)

<220>
<221>  CDS
<222>  (11)..(118)

<220>
<221>  CDS
<222>  (387)..(809)

<400>  13
gatatccacc atg gcc ccc gcc gcc tgg ctg agg agc gcc gcc gcc agg        49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg       97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct         148
Leu Ala Arg Ala Leu Pro Pro
30                  35

cgctgagggg gcgccgggca cgcgggctgg gcccagcggc gtatccggac gccaagaaac    208

cagagagcca gccagatgcc aaagggccct gccatgtgcc ggtgcccttt ccctctccat    268

ttgccctgcc acacagtggg ctggggttgc acgtgtgttt gctgacaggc cacatctcta    328

actgtgggcc atgtggacct taggcctgac cagaccctca tgtcttcctc cttcccag      386

gac gtg cac cac ctg cac gcc gag agg cgc ggc cct cag ccc tgg cac      434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc      482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat      530
Pro Arg Pro Ala Ser Ser Leu Arg Pro Pro Arg Cys Gly Val Pro Asp

70                  75                  80

ccc tcc gat ggc ctg agc gct cgg aat cgg cag aag agg cac gcc gag      578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc      626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc      674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gag      722
Pro Glu Glu Val Ala Ile Val Glu Glu Leu Gly Arg Arg His Ala Glu
        135                 140                 145

ggc acc ttc acc tcc gac gtg agc agc tac ctg gag ggc cag gcc gcc      770
Gly Thr Phe Thr Ser Asp Val Ser Ser Tyr Leu Glu Gly Gln Ala Ala
    150                 155                 160

aag gag ttc atc gcc tgg ctg gtg aag ggc agg ggc tga gcgcgc           815
Lys Glu Phe Ile Ala Trp Leu Val Lys Gly Arg Gly
165                 170                 175

<210>  14
<211>  815
<212>  DNA
<213>  Artificial

<220>
<223>  Description of sequence: DNA sequence of the construct according
       to Fig. 1k, (see description p. 52 and 70)

<400>  14
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

agggcacctt cacctccgac gtgagcagct acctggaggg ccaggccgcc aaggagttca     780

tcgcctggct ggtgaagggc aggggctgag cgcgc                                815

<210>  15
<211>  834
<212>  DNA
<213>  Artificial

<220>
<223>  Description of sequence: DNA sequence and sequence of the
       translated peptide according to the construct Fig. 1h, (see
       description p. 51 and 71)

<220>
<221>  CDS
<222>  (11)..(118)

<220>
<221>  CDS
<222>  (387)..(821)

<400>  15
gatatccacc atg gcc ccc gcc gcc tgg ctg agg agc gcc gcc gcc agg        49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg       97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct         148
Leu Ala Arg Ala Leu Pro Pro
30                  35

cgctgagggg gcgccgggca cgcgggctgg gcccagcggc gtatccggac gccaagaaac    208

cagagagcca gccagatgcc aaagggccct gccatgtgcc ggtgcccttt ccctctccat    268

ttgccctgcc acacagtggg ctggggttgc acgtgtgttt gctgacaggc cacatctcta    328

actgtgggcc atgtggacct taggcctgac cagaccctca tgtcttcctc cttcccag      386

gac gtg cac cac ctg cac gcc gag agg cgc ggc cct cag ccc tgg cac      434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc      482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat      530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag      578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc      626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc      674

Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac      722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg      770
Gly Ser Phe Ser Asp Glu Met Asn Thr Ile Leu Asp Asn Leu Ala Ala
    150                 155                 160

cgc gac ttc atc aac tgg ctg atc cag acc aag atc acc gat cgg aag      818
Arg Asp Phe Ile Asn Trp Leu Ile Gln Thr Lys Ile Thr Asp Arg Lys
165                 170                 175                 180

tga gcgcgctgat atc                                                   834


<210>  16
<211>  834
<212>  DNA
<213>  Artificial

<220>
<223>  Description of sequence: DNA sequence of the construct according
       to Fig. 1h, (see description p. 51 and 71)

<400>  16
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

acggcagctt cagcgacgag atgaacacca tcctggacaa cctggccgcg cgcgacttca     780

tcaactggct gatccagacc aagatcaccg atcggaagtg agcgcgctga tatc           834


<210>  17
<211>  780
<212>  DNA
<213>  Artificial

<220>
<223>  Description of sequence: DNA sequence sequence and translated

peptide sequence of the construct according to Fig. 11,
(see description p. 52 and 71)

<220>
<221> CDS
<222> (11)..(118)

<220>
<221> CDS
<222> (387)..(776)

<400> 17
gatatccacc atg gcc ccc gcc gcc tgg ctg agg agc gcc gcc gcc agg 49
Met Ala Pro Ala Ala Trp Leu Arg Ser Ala Ala Ala Arg
1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac 722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg 770
Gly Ser Phe Ser Asp Glu Met Asn Thr Ile Leu Asp Asn Leu Ala Ala
    150                 155                 160

cgc tga tatc                                                          780
Arg
165


<210>  18
<211>  720
<212>  DNA
<213>  Artificial

<220>
<223>  Description of sequence: DNA sequence of the construct according
       to Fig. 1l, (see description p. 52 and 72)

<400>  18
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

acggcagctt cagcgacgag atgaacacca tcctggacaa cctggccgcg cgctgatatc     720


<210>  19
<211>  716

<212>  DNA
<213>  Artificial

<220>
<223>  Description of sequence: DNA sequence and sequence of the
       translated peptide according to Fig. 1m., (see description p.
       52 and 72)

<220>
<221>  CDS
<222>  (11)..(118)

<220>
<221>  CDS
<222>  (387)..(710)

<400>  19
gatatccacc atg gcc ccc gcc gcc tgg ctg agg agc gcc gcc gcc agg         49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc tga gcgcgc 716
Pro Glu Glu Val Ala Ile Val Glu Glu Leu Gly
        135                 140

<210> 20
<211> 716
<212> DNA
<213> Artificial

<220>
<223> Description of sequence: DNA sequence of the construct
according to Fig. 1m, (see description p. 52 and 73)

<400> 20
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac    300

gtgtgtttgc tgacaggcca catctctaac tgtgggccat gtggacctta ggcctgacca    360

gaccctcatg tcttcctcct tcccaggacg tgcaccacct gcacgccgag aggcgcggcc    420

ctcagccctg gcacgccgcc ctgccaagca gccctgcccc tgccccagcc acccaggagg    480

cccccaggcc tgccagcagc ctgaggccac ccaggtgcgg cgtgcctgat ccctccgatg    540

gcctgagcgc tcggaatcgg cagaagaggc acgccgaggg caccttcacc tccgacgtga    600

gcagctacct ggagggccag gccgccaagg agttcatcgc ctggctggtg aagggcaggg    660

gccgcaggga cttccctgag gaggtggcca tcgtggagga gctgggctga gcgcgc        716

<210>  21
<211>  31
<212>  PRT
<213>  Artificial

<220>
<223>  Description of sequence: artificial, (see description p. 73)

<400>  21

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  22
<211>  10
<212>  PRT
<213>  Artificial

<220>
<223>  Description of sequence: artificial, (see description p. 21 and 73)

<400>  22

Arg Arg Asp Phe Pro Glu Glu Val Ala Ile
1               5                   10

<210>  23
<211>  14
<212>  PRT
<213>  Artificial

<220>
<223>  Description of sequence: artificial, (see description p. 21 and 73)

<400>  23

Arg Arg Asp Phe Pro Glu Glu Val Ala Ile Val Glu Glu Leu
1               5                   10

<210>  24
<211>  14
<212>  PRT
<213>  Artificial

<220>
<223>  Description of sequence: artificial, (see description p. 21 and 73)

<400>  24

Arg Arg Asp Phe Pro Glu Glu Val Ala Ile Ala Glu Glu Leu
1               5                   10


<210>  25
<211>  7
<212>  PRT
<213>  Artificial

<220>
<223>  Description of sequence: partial sequence derived from IP2
        (see description p. 21 and 74)

<400>  25

Asp Phe Pro Glu Glu Val Ala
1               5


<210>  26
<211>  10
<212>  PRT
<213>  Artificial

<220>
<223>  Description of sequence: sequence derived from SEQ ID NO: 22,
       wherein the first two arginines have been replaced by two alanines
        (see description p. 21 and 74)

<400>  26

Ala Ala Asp Phe Pro Glu Glu Val Ala Ile
1               5                   10


<210>  27
<211>  14
<212>  PRT
<213>  Artificial

<220>
<223>  Description of sequence: sequence derived from SEQ ID NO: 23,
       wherein the first two arginines have been replaced by two
       alanines (see description p. 21 and 74)

<400>  27

Ala Ala Asp Phe Pro Glu Glu Val Ala Ile Val Glu Glu Leu
1               5                   10


<210>  28
<211>  14

<212>  PRT
<213>  Artificial

<220>
<223>  Description of sequence: sequence derived from SEQ ID NO: 24,
       wherein the first two arginines have been replaced by two
       alanines(see description p. 21 and 74)

<400>  28

Ala Ala Asp Phe Pro Glu Glu Val Ala Ile Ala Glu Glu Leu
1               5                   10


<210>  29
<211>  15
<212>  PRT
<213>  Artificial

<220>
<223>  Description of sequence: sequence derived from SEQ ID NO: 2,
       wherein the first two arginines have been replaced by two
       alanines (see description p. 21 and 74)

<400>  29

Ala Ala Asp Phe Pro Glu Glu Val Ala Ile Val Glu Glu Leu Gly
1               5                   10                  15


<210>  30
<211>  15
<212>  PRT
<213>  Artificial

<220>
<223>  Description of sequence: sequence derived from SEQ ID NO: 3,
       wherein the first two arginines have been replaced by two
       alanines (see description p. 21 and 74)

<400>  30

Ala Ala Asp Phe Pro Glu Glu Val Ala Ile Ala Glu Glu Leu Gly
1               5                   10                  15


<210>  31
<211>  46
<212>  PRT
<213>  Artificial

<220>
<223>  Description of sequence: sequence derived from GLP-1-IP2-fragment
       (murine) (see description p. 22 and 74)

<400>  31

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly Ala
            20                  25                  30

Ala Asp Phe Pro Glu Glu Val Ala Ile Ala Glu Glu Leu Gly
        35                  40                  45

<210>  32
<211>  46
<212>  PRT
<213>  Artificial

<220>
<223>  Description of sequence: sequence derived from GLP-1-IP2-fragment
       (murine) (see description p. 22 and 74)

<400>  32

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Ala Glu Glu Leu Gly
        35                  40                  45

<210>  33
<211>  46
<212>  PRT
<213>  Artificial

<220>
<223>  Description of sequence: sequence derived from GLP-1-IP2-fragment
       (murine) (see description p. 22 and 74)

<400>  33

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Ala Glu Glu Leu Gly
        35                  40                  45

<210>  34
<211>  46
<212>  PRT
<213>  Artificial

<220>
<223>  Description of sequence: sequence derived from GLP-1-IP2-fragment
       (murine) (see description p. 22 and 75)

<400>  34

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Ala Ala Ala Leu Gly
        35                  40                  45

<210>  35
<211>  36
<212>  PRT
<213>  Artificial

<220>
<223>  Description of sequence: sequence derived from GLP-1-IP2-fragment
       (murine) (see description p. 22 and 75)

<400>  35

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro
        35

<210>  36
<211>  40
<212>  PRT
<213>  Artificial

<220>
<223>  Description of sequence: sequence derived from GLP-1-IP2-fragment
       (murine) (see description p. 22 and 75)

<400>  36

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala
        35                  40

<210>  37
<211>  51
<212>  PRT
<213>  Artificial

<220>
<223>  Description of sequence: sequence derived from GLP-1-IP2-fragment
       (murine) (see description p. 22 and 75)

<400>  37

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Cys
    50

<210>  38
<211>  46
<212>  PRT
<213>  Artificial

<220>
<223>  Description of sequence: sequence derived from GLP-1-IP2-fragment
       (human) (see description p. 22 and 75)

<400>  38

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Phe Pro Glu Glu Val Ala Ile Val Glu Glu Leu Gly
        35                  40                  45

<210>  39
<211>  46
<212>  PRT
<213>  Artificial

<220>
<223>  Description of sequence: sequence derived from GLP-1-IP2-fragment
       (human) (see description p. 22 and 75)

<400>  39

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Val Glu Glu Leu Gly
        35                  40                  45

<210>  40
<211>  46
<212>  PRT
<213>  Artificial

<220>
<223>  Description of sequence: sequence derived from GLP-1-IP2-fragment
       (human) (see description p. 22 and 76)

<400>  40

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Val Glu Glu Leu Gly
        35                  40                  45

<210>  41
<211>  46
<212>  PRT
<213>  Artificial

<220>
<223>  Description of sequence: sequence derived from GLP-1-IP2-fragment
       (human) (see description p. 22 and 76)

<400>  41

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Val Ala Ala Leu Gly
        35                  40                  45

<210>  42
<211>  51
<212>  PRT
<213>  Artificial

<220>
<223>  Description of sequence: sequence derived from GLP-1-IP2-fragment
       (human) (see description p. 22 and 76)

<400>  42

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Cys
    50

<210>  43
<211>  31
<212>  PRT
<213>  Artificial

<220>
<223>  Description of sequence: sequence according to formula (I)
       (see description, p. 1 and 76)

<220>
<221>  misc_feature
<222>  (31)..(31)
<223>  Xaa = NH2, when sequence is GLP-1(7-36) amide, or Xaa = Gly-OH,
       when sequence is GLP-1(7-37) amide

<400>  43

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Xaa
            20                  25                  30

<210>  44
<211>  31
<212>  PRT
<213>  Artificial

<220>
<223>  Description of sequence:  sequence according to formula (II)
       (see description p. 28 and 76)

<220>
<221>  VARIATION
<222>  (1)..(1)
<223>  /replace="D-histidine"
       /replace="desamino-histidine"
       /replace="2-amino-histidine"
       /replace="3-hydroxy-histidine"
       /replace="homohistidine"
       /replace="N-acetyl-histidine"
       /replace="alpha-fluoromethyl-histidine"
       /replace="alpha-methyl-histidine"
       /replace="3-pyridylalanine"
       /replace="2-pyridylalanine"
       /replace="4-pyridylalanine"

<220>
<221>  VARIATION
<222>  (2)..(2)

<223>  /replace="Ala"
      /replace="Val"
      /replace="Leu"
      /replace="Ile"
      /replace="Lys"
      /replace="Aib"
      /replace="(1-aminocyclopropyl) carboxylic acid"
      /replace="(1-aminocyclobutyl) carboxylic acid"
      /replace="(1-aminocyclopentyl) carboxylic acid"
      /replace="(1-aminocyclohexyl) carboxylic acid"
      /replace="(1-aminocycloheptyl) carboxylic acid"
      /replace="(1-aminocyclooctyl) carboxylic acid"

<220>
<221>  VARIATION
<222>  (10)..(10)
<223>  /replace="Leu"

<220>
<221>  VARIATION
<222>  (12)..(12)
<223>  /replace="Lys"
      /replace="Arg"

<220>
<221>  VARIATION
<222>  (13)..(13)
<223>  /replace="Gln"

<220>
<221>  VARIATION
<222>  (14)..(14)
<223>  /replace="Met"

<220>
<221>  VARIATION
<222>  (16)..(16)
<223>  /replace="Glu"
      /replace="Aib"

<220>
<221>  VARIATION
<222>  (17)..(17)
<223>  /replace="Glu"
      /replace="Lys"
      /replace="Arg"

<220>
<221>  VARIATION
<222>  (19)..(19)
<223>  /replace="Val"

<220>
<221>  VARIATION
<222>  (20)..(20)
<223>  /replace="Glu"
      /replace="Arg"

<220>
<221>  VARIATION
<222>  (21)..(21)
<223>  /replace="Leu"

<220>

<221>  VARIATION
<222>  (24)..(24)
<223>  /replace="Glu"
       /replace="Arg"

<220>
<221>  VARIATION
<222>  (27)..(27)
<223>  /replace="Lys"

<220>
<221>  VARIATION
<222>  (28)..(28)
<223>  /replace="Glu"
       /replace="Asn"
       /replace="Arg"

<220>
<221>  VARIATION
<222>  (29)..(29)
<223>  /replace="Aib"

<220>
<221>  VARIATION
<222>  (30)..(30)
<223>  /replace="Gly"
       /replace="Lys"
       /replace="amide"
       /replace=""

<220>
<221>  VARIATION
<222>  (31)..(31)
<223>  /replace="Ala"
       /replace="Glu"
       /replace="Pro"
       /replace="Lys"
       /replace="amide"
       /replace=""

<400>  44

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  45
<211>  31
<212>  PRT
<213>  Artificial

<220>
<223>  Description of sequence: sequence according to formula (III)
       (see description p. 29 and 76)

<220>
<221>  VARIATION
<222>  (1)..(1)
<223>  /replace="D-histidine"
       /replace="desamino-histidine"

      /replace="2-amino-histidine"
      /replace="3-hydroxy-histidine"
      /replace="homohistidine"
      /replace="N-acetyl-histidine"
      /replace="alpha-fluoromethyl-histidine"
      /replace="alpha-methyl-histidine"
      /replace="3-pyridylalanine"
      /replace="2-pyridylalanine"
      /replace="4-pyridylalanine"

<220>
<221>  VARIATION
<222>  (2)..(2)
<223>  /replace="Gly"
      /replace="Val"
      /replace="Leu"
      /replace="Ile"
      /replace="Lys"
      /replace="Aib"
      /replace="(1-aminocyclopropyl) carboxylic acid"
      /replace="(1-aminocyclobutyl) carboxylic acid"
      /replace="(1-aminocyclopentyl) carboxylic acid"
      /replace="(1-aminocyclohexyl) carboxylic acid"
      /replace="(1-aminocycloheptyl) carboxylic acid"
      /replace="(1-aminocyclooctyl) carboxylic acid"

<220>
<221>  VARIATION
<222>  (12)..(12)
<223>  /replace="Lys"
      /replace="Arg"

<220>
<221>  VARIATION
<222>  (16)..(16)
<223>  /replace="Glu"
      /replace="Aib"

<220>
<221>  VARIATION
<222>  (17)..(17)
<223>  /replace="Glu"
      /replace="Lys"
      /replace="Arg"

<220>
<221>  VARIATION
<222>  (20)..(20)
<223>  /replace="Glu"
      /replace="Arg"

<220>
<221>  VARIATION
<222>  (24)..(24)
<223>  /replace="Glu"
      /replace="Arg"

<220>
<221>  VARIATION
<222>  (28)..(28)
<223>  /replace="Glu"
      /replace="Arg"

<220>

<221>  VARIATION
<222>  (29)..(29)
<223>  /replace="Aib"

<220>
<221>  VARIATION
<222>  (30)..(30)
<223>  /replace="Lys"
       /replace="amide"
       /replace=""

<220>
<221>  VARIATION
<222>  (31)..(31)
<223>  /replace="Ala"
       /replace="Glu"
       /replace="Lys"
       /replace="amide"
       /replace=""

<400>  45

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

Sequence listing

HAEGTFTSDV SSYLEGQAAK EFIAWLVKGR G
Sequence ID No.:1

RRDFPEEVAI VEELG
Sequence ID No.:2

RRDFPEEVAI AEELG
Sequence ID No.:3

HADGSFSDEM NTILDNLAAR DFINWLIQTK ITDRK
Sequence ID No.:4

HADGSFSDEM STILDNLATR DFINWLIQTK ITDKK
Sequence ID No.:5

HAEGTFTSDV SSYLEGQAAK EFIAWLVKGR GRRDFPEEVA IAEELGRRHA
EGTFTSDVSS YLEGQAAKEF IAWLVKGRG
Sequence ID No.:6

HAEGTFTSDV SSYLEGQAAK EFIAWLVKGR GRRDFPEEVA IAEELGRRHA
DGSFSDEMST ILDNLATRDF INWLIQTKIT DKK
Sequence ID No.:7

HAEGTFTSDV SSYLEGQAAK EFIAWLVKGR GRRDFPEEVA IAEELG
Sequence ID No.:8

MAPAAWLRSA AARALLPPML LLLLQPPPLL ARALPPDVHH LHAERRGPQP
WHAALPSSPA PAPATQEAPR PASSLRPPRC GVPDPSDGLS ARNRQKR
Sequence ID No.:9

HAEGTFTSDV SSYLEGQAAK EFIAWLVKGR GRRDFPEEVA IVEELGRRHA
EGTFTSDVSS YLEGQAAKEF IAWLVKGRG
Sequence ID No.:10

HAEGTFTSDV SSYLEGQAAK EFIAWLVKGR GRRDFPEEVA IVEELGRRHA
DGSFSDEMNT ILDNLAARDF INWLIQTKIT DRK
Sequence ID No.:11

HAEGTFTSDV SSYLEGQAAK EFIAWLVKGR GRRDFPEEVA IVEELG
Sequence ID No.:12

1 gatatccacc atggcccccg ccgcctggct gaggagcgcc gccgccaggg
M A P A A W L R S A A A R A
51 ccctgctgcc acccatgctg ctgctgctgc tgcagccccc acctctgctg
L L P P M L L L L L Q P P P L L
101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L
401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggccgg cgacacgccg agggcacctt cacctccgac gtgagcagct
L G R R H A E G T F T S D V S S Y
751 acctggaggg ccaggccgcc aaggagttca tcgcctggct ggtgaagggc
L E G Q A A K E F I A W L V K G
801 aggggctgag cgcgc
R G *

Sequence ID No.:13

1 gatatccacc atggcccccg ccgcctggct gaggagcgcc gccgccaggg ccctgctgcc
61 acccatgctg ctgctgctgc tgcagccccc acctctgctg gcccgggccc tgcccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
661 gccgcaggga cttccctgag gaggtggcca tcgtggagga gctgggccgg cgacacgccg
721 agggcacctt cacctccgac gtgagcagct acctggaggg ccaggccgcc aaggagttca
781 tcgcctggct ggtgaagggc aggggctgag cgcgc

Sequence ID No.:14

1 gatatccacc atggcccccg ccgcctggct gaggagcgcc gccgccaggg
M A P A A W L R S A A A R A
51 ccctgctgcc acccatgctg ctgctgctgc tgcagccccc acctctgctg
L L P P M L L L L Q P P P L L
101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L
401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggccgg cgacacgccg acggcagctt cagcgacgag atgaacacca
L G R R H A D G S F S D E M N T I
751 tcctggacaa cctggccgcg cgcgacttca tcaactggct gatccagacc
L D N L A A R D F I N W L I Q T
801 aagatcaccg atcggaagtg agcgcgctga tatc
K I T D R K *

Sequence ID No.:15

1 gatatccacc atggcccccg ccgcctggct gaggagcgcc gccgccaggg ccctgctgcc
61 acccatgctg ctgctgctgc tgcagccccc acctctgctg gcccgggccc tgcccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
661 gccgcaggga cttccctgag gaggtggcca tcgtggagga gctgggccgg cgacacgccg
721 acggcagctt cagcgacgag atgaacacca tcctggacaa cctggccgcg cgcgacttca
781 tcaactggct gatccagacc aagatcaccg atcggaagtg agcgcgctga tatc

Sequence ID No.:16

1 gatatccacc atggcccccg ccgcctggct gaggagcgcc gccgccaggg
M A P A A W L R S A A A R A
51 ccctgctgcc acccatgctg ctgctgctgc tgcagccccc acctctgctg
L L P P M L L L L Q P P P L L

101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L
401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggccgg cgacacgccg acggcagctt cagcgacgag atgaacacca
L G R R H A D G S F S D E M N T I
751 tcctggacaa cctggccgcg cgctga tat c
L D N L A A R *

Sequence ID No.:17

1 gatatccacc atggcccccg ccgcctggct gaggagcgcc gccgccaggg ccctgctgcc
61 acccatgctg ctgctgctgc tgcagccccc acctctgctg gcccgggccc tgcccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
721 acggcagctt cagcgacgag atgaacacca tcctggacaa cctggccgcg cgctgatatc

Sequence ID No.:18

1 gatatccacc atggcccccg ccgcctggct gaggagcgcc gccgccaggg
M A P A A W L R S A A A R A
51 ccctgctgcc acccatgctg ctgctgctgc tgcagccccc acctctgctg
L L P P M L L L L Q P P P L L
101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L

401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggctga gcgcgc
L G *

Sequence ID No.:19

1 gatatccacc atggcccccg ccgcctggct gaggagcgcc gccgccaggg ccctgctgcc
61 acccatgctg ctgctgctgc tgcagccccc acctctgctg gcccgggccc tgcccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
661 gccgcaggga cttccctgag gaggtggcca tcgtggagga gctgggctga gcgcgc

Sequence ID No.:20

HGEGTFTSDV SSYLEGQAAK EFIAWLVKGR G
Sequence ID No.:21

RRDFPEEVAI
Sequence ID No.:22

RRDFPEEVAI VEEL
Sequence ID No.:23

RRDFPEEVAI AEEL
Sequence ID No.:24

Asp Phe Pro Glu Glu Val Ala

Sequence ID No: 25

Ala Ala Asp Phe Pro Glu Glu Val Ala Ile

Sequence ID No: 26

Ala Ala Asp Phe Pro Glu Glu Val Ala Ile Val Glu Glu Leu

Sequence ID No: 27

Ala Ala Asp Phe Pro Glu Glu Val Ala Ile Ala Glu Glu Leu

Sequence ID No: 28

Ala Ala Asp Phe Pro Glu Glu Val Ala Ile Val Glu Glu Leu Gly

Sequence ID No: 29

Ala Ala Asp Phe Pro Glu Glu Val Ala Ile Ala Glu Glu Leu Gly

Sequence ID No: 30

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Phe Pro Glu Glu Val Ala Ile Ala Glu Glu Leu Gly

Sequence ID No: 31

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Ala Glu Glu Val Ala Ile Ala Glu Glu Leu Gly

Sequence ID No:32

His Ala Glu Gly Thr Phe Thr Ser Asp Val Ser Ser Tyr Leu Glu Gly

Gln Ala Ala Lys Glu Phe Ile Ala Trp Leu Val Lys Gly Arg Gly Arg
Arg Asp Ala Ala Ala Ala Val Ala Ile Ala Glu Glu Leu Gly

Sequence ID No: 33

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Ala Ala Ala Ala Val Ala Ile Ala Ala Ala Leu Gly

Sequence ID No: 34

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro

Sequence ID No: 35

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro Glu Glu Val Ala

Sequence ID No: 36

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro Glu Glu Val Ala Ile Ala Glu Glu Leu Gly Arg Arg
His Ala Cys

Sequence ID No: 37

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Phe Pro Glu Glu Val Ala Ile Val Glu Glu Leu Gly

Sequence ID No: 38

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Ala Glu Glu Val Ala Ile Val Glu Glu Leu Gly

Sequence ID No: 39

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Ala Ala Ala Ala Val Ala Ile Val Glu Glu Leu Gly

Sequence ID No: 40

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Ala Ala Ala Ala Val Ala Ile Val Ala Ala Leu Gly

Sequence ID No: 41

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro Glu Glu Val Ala Ile Val Glu Glu Leu Gly Arg Arg
His Ala Cys

Sequence ID No: 42

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Xaa

Sequence ID No: 43 (formula (I))

Xaa Xaa Glu Gly Thr Phe Thr Ser Asp Xaa Ser Xaa Xaa Xaa Glu Xaa
Xaa Ala Xaa Xaa Xaa Phe Ile Xaa Trp Leu Xaa Xaa Xaa Xaa Xaa

Sequence ID No: 44 (formula (II))

Xaa Xaa Glu Gly Thr Phe Thr Ser Asp Val Ser Xaa Tyr Leu Glu Xaa
Xaa Ala Ala Xaa Glu Phe Ile Xaa Trp Leu Val Xaa Xaa Xaa Xaa

Sequence ID No: 45 (formula (III))

SEQUENCE LISTING

<110> Biocompatibles UK Limited

<120> GLP-1 fusion peptides, their production and use

<130> BI07P001WO

<140>
<141>

<150> EP050207.18.2
<151> 2005-09-22

<160> 45

<170> PatentIn version 3.3

<210> 1
<211> 31
<212> PRT
<213> Artificial

<220>
<223> Description of sequence: synthetic peptide corresponding to
GLP-1(7-37) (see description p. 36)

<400> 1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 2
<211> 15
<212> PRT
<213> Artificial

<220>
<223> Description of sequence: full-length IP-2 sequence having all 15
amino acids of the naturally occurring IP-2 sequence, human;
(see description p. 36)

<400> 2

Arg Arg Asp Phe Pro Glu Glu Val Ala Ile Val Glu Glu Leu Gly
1               5                   10                  15

<210> 3
<211> 15
<212> PRT
<213> Artificial

<220>
<223> Description of sequence: full-length IP-2 sequence having all 15
amino acids of the naturally occurring IP-2 sequence, murine;
(see description p. 36)

<400> 3

Arg Arg Asp Phe Pro Glu Glu Val Ala Ile Ala Glu Glu Leu Gly

1               5                   10                  15

<210>  4
<211>  35
<212>  PRT
<213>  Artificial

<220>
<223>  Description of sequence: murine isoform of GLP-2
       (see description p. 36)

<400>  4

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp Arg Lys
        35

<210>  5
<211>  35
<212>  PRT
<213>  Artificial

<220>
<223>  Description of sequence: human isoform of GLP-2
       (see description p. 36)

<400>  5

His Ala Asp Gly Ser Phe Ser Asp Glu Met Ser Thr Ile Leu Asp Asn
1               5                   10                  15

Leu Ala Thr Arg Asp Phe Ile Asn Trp Leu Ile Gln Thr Lys Ile Thr
            20                  25                  30

Asp Lys Lys
        35

<210>  6
<211>  79
<212>  PRT
<213>  Artificial

<220>
<223>  Description of sequence: SEQ ID No: 6 (ID6syn, CM1) corresponds
       to GLP-1(7-37)-IP2-RR-GLP1(7-37), 79 aa, 8,7 kD
       (see description p. 36)

<400>  6

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  7
<211>  83
<212>  PRT
<213>  Artificial

<220>
<223>  Description of sequence: SEQ ID No: 7 (ID7rec, CM2) corresponds
       to GLP-1(7-37)-IP2-RR-GLP2, 83 aa, 9,4 kD
       (see description p. 36)

<400>  7

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Asp Gly Ser Phe Ser Asp Glu Met Ser Thr Ile Leu Asp Asn
    50                  55                  60

Leu Ala Thr Arg Asp Phe Ile Asn Trp Leu Ile Gln Thr Lys Ile Thr
65                  70                  75                  80

Asp Lys Lys

<210>  8
<211>  46
<212>  PRT
<213>  Artificial

<220>
<223>  Description of sequence: SEQ ID No:8 (ID8 syn, CM3) corresponds
       to GLP-1(7-37)-IP2, 46 aa, 5,1 kD;

<400>  8

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly
        35                  40                  45

<210>  9
<211>  97
<212>  PRT
<213>  Artificial

<220>
<223>  Description of sequence: artificial

<400>  9

Met Ala Pro Ala Ala Trp Leu Arg Ser Ala Ala Ala Arg Ala Leu Leu
1               5                   10                  15

Pro Pro Met Leu Leu Leu Leu Leu Gln Pro Pro Pro Leu Leu Ala Arg
            20                  25                  30

Ala Leu Pro Pro Asp Val His His Leu His Ala Glu Arg Arg Gly Pro
        35                  40                  45

Gln Pro Trp His Ala Ala Leu Pro Ser Ser Pro Ala Pro Ala Pro Ala
    50                  55                  60

Thr Gln Glu Ala Pro Arg Pro Ala Ser Ser Leu Arg Pro Pro Arg Cys
65                  70                  75                  80

Gly Val Pro Asp Pro Ser Asp Gly Leu Ser Ala Arg Asn Arg Gln Lys
                85                  90                  95

Arg

<210>  10
<211>  79
<212>  PRT
<213>  Artificial

<220>
<223>  Description of sequence: SEQ ID No: 10
       (N-GLP-1(7-37)-IP2(human)-RR-GLP-1(7-37)-C, also designated human
       CM1), (see description p. 36)

<400>  10

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  11
<211>  83
<212>  PRT
<213>  Artificial

<220>
<223>  Description of sequence: SEQ ID No: 11
       (N-GLP-1(7-37)-IP2(human)-RR-GLP-2-C), also designated human CM2
       herein), (see description p. 36)

<400>  11

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Asp Gly Ser Phe Ser Asp Glu Met Asn Thr Ile Leu Asp Asn
    50                  55                  60

Leu Ala Ala Arg Asp Phe Ile Asn Trp Leu Ile Gln Thr Lys Ile Thr
65                  70                  75                  80

Asp Arg Lys

<210>  12
<211>  46
<212>  PRT
<213>  Artificial

<220>
<223>  Description of sequence: SEQ ID No: 12, GLP-1(7-37) linked
       without any linker sequence via its C-terminus to human IP2
       (see description p. 36)

<400>  12

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly
        35                  40                  45

<210>  13
<211>  815

<212>  DNA
<213>  Artificial

<220>
<223>  Description of sequence: (SEQ ID No:13) represents the
      translated peptide sequence of the construct according to Fig. 1k
      (SEQ ID No: 14), (see description p. 37);


<220>
<221>  CDS
<222>  (11)..(118)

<220>
<221>  CDS
<222>  (387)..(809)

<400>  13
gatatccacc atg gcc ccc gcc gcc tgg ctg agg agc gcc gcc gcc agg        49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg        97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct          148
Leu Ala Arg Ala Leu Pro Pro
30                  35

cgctgagggg gcgccgggca cgcgggctgg gcccagcggc gtatccggac gccaagaaac     208

cagagagcca gccagatgcc aaagggccct gccatgtgcc ggtgcccttt ccctctccat     268

ttgccctgcc acacagtggg ctggggttgc acgtgtgttt gctgacaggc cacatctcta     328

actgtgggcc atgtggacct taggcctgac cagaccctca tgtcttcctc cttcccag       386

gac gtg cac cac ctg cac gcc gag agg cgc ggc cct cag ccc tgg cac       434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc       482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat       530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag       578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc       626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc       674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gag       722
Pro Glu Glu Val Ala Ile Val Glu Glu Leu Gly Arg Arg His Ala Glu
        135                 140                 145

ggc acc ttc acc tcc gac gtg agc agc tac ctg gag ggc cag gcc gcc       770
Gly Thr Phe Thr Ser Asp Val Ser Ser Tyr Leu Glu Gly Gln Ala Ala

150                 155                 160

aag gag ttc atc gcc tgg ctg gtg aag ggc agg ggc tga gcgcgc          815
Lys Glu Phe Ile Ala Trp Leu Val Lys Gly Arg Gly
165                 170                 175

<210>  14
<211>  815
<212>  DNA
<213>  Artificial

<220>
<223>  Description of sequence: DNA sequence of the construct according
       to Fig. 1k, (see description p. 37);

<400>  14
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

agggcacctt cacctccgac gtgagcagct acctggaggg ccaggccgcc aaggagttca     780

tcgcctggct ggtgaagggc aggggctgag cgcgc                                815

<210>  15
<211>  834
<212>  DNA
<213>  Artificial

<220>
<223>  Description of sequence: DNA sequence and sequence of the
       translated peptide according to the construct Fig. 1h, (see
       description p. 38);

<220>
<221>  CDS
<222>  (11)..(118)

<220>
<221>  CDS
<222>  (387)..(821)

<400>  15
gatatccacc atg gcc ccc gcc gcc tgg ctg agg agc gcc gcc gcc agg          49

Met Ala Pro Ala Ala Trp Leu Arg Ser Ala Ala Ala Arg
1               5                   10

gcc ctg ctg cca ccc atg ctg ctg ctg ctg ctg cag ccc cca cct ctg      97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct      148
Leu Ala Arg Ala Leu Pro Pro
30                  35

cgctgagggg gcgccgggca cgcgggctgg gcccagcggc gtatccggac gccaagaaac      208

cagagagcca gccagatgcc aaagggccct gccatgtgcc ggtgcccttt ccctctccat      268

ttgccctgcc acacagtggg ctggggttgc acgtgtgttt gctgacaggc cacatctcta      328

actgtgggcc atgtggacct taggcctgac cagaccctca tgtcttcctc cttcccag      386

gac gtg cac cac ctg cac gcc gag agg cgc ggc cct cag ccc tgg cac      434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc      482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat      530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag      578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc      626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc      674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac      722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg      770
Gly Ser Phe Ser Asp Glu Met Asn Thr Ile Leu Asp Asn Leu Ala Ala
    150                 155                 160

cgc gac ttc atc aac tgg ctg atc cag acc aag atc acc gat cgg aag      818
Arg Asp Phe Ile Asn Trp Leu Ile Gln Thr Lys Ile Thr Asp Arg Lys
165                 170                 175                 180

tga gcgcgctgat atc                                                  834

<210>  16
<211>  834
<212>  DNA
<213>  Artificial

<220>
<223>  Description of sequence: DNA sequence of the construct according
       to Fig. 1h, (see description p. 38);

<400>  16

gatatccacc atggcccccg ccgcctggct gaggagcgcc gccgccaggg ccctgctgcc 60
acccatgctg ctgctgctgc tgcagccccc acctctgctg gcccgggccc tgcccccggt 120
gagtgcccgc cactcgccgt ccgctcctcg ctgagggggc gccgggcacg cgggctgggc 180
ccagcggcgt atccggacgc caagaaacca gagagccagc cagatgccaa agggccctgc 240
catgtgccgg tgccctttcc ctctccattt gccctgccac acagtgggct ggggttgcac 300
gtgtgtttgc tgacaggcca catctctaac tgtgggccat gtggacctta ggcctgacca 360
gaccctcatg tcttcctcct tcccaggacg tgcaccacct gcacgccgag aggcgcggcc 420
ctcagccctg gcacgccgcc ctgccaagca gccctgcccc tgccccagcc acccaggagg 480
cccccaggcc tgccagcagc ctgaggccac ccaggtgcgg cgtgcctgat ccctccgatg 540
gcctgagcgc tcggaatcgg cagaagaggc acgccgaggg caccttcacc tccgacgtga 600
gcagctacct ggagggccag gccgccaagg agttcatcgc ctggctggtg aagggcaggg 660
gccgcaggga cttccctgag gaggtggcca tcgtggagga gctgggccgg cgacacgccg 720
acggcagctt cagcgacgag atgaacacca tcctggacaa cctggccgcg cgcgacttca 780
tcaactggct gatccagacc aagatcaccg atcggaagtg agcgcgctga tatc 834

<210> 17
<211> 780
<212> DNA
<213> Artificial

<220>
<223> Description of sequence: DNA sequence sequence and translated
      peptide sequence of the construct according to Fig. 11,
      (see description p. 35, 38);

<220>
<221> CDS
<222> (11)..(118)

<220>
<221> CDS
<222> (387)..(776)

<400> 17
gatatccacc atg gcc ccc gcc gcc tgg ctg agg agc gcc gcc gcc agg 49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac     434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc     482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat     530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag     578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc     626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc     674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac     722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg     770
Gly Ser Phe Ser Asp Glu Met Asn Thr Ile Leu Asp Asn Leu Ala Ala
    150                 155                 160

cgc tga tatc                                                        780
Arg
165


<210>  18
<211>  720
<212>  DNA
<213>  Artificial

<220>
<223>  Description of sequence: DNA sequence of the construct according
       to Fig. 11, (see description p. 39);

<400>  18
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg 660

acggcagctt cagcgacgag atgaacacca tcctggacaa cctggccgcg cgctgatatc 720

<210> 19
<211> 716

<212> DNA
<213> Artificial

<220>
<223> Description of sequence: DNA sequence and sequence of the
      translated peptide according to Fig. 1m., (see description p.
      39, 40);

<220>
<221> CDS
<222> (11)..(118)

<220>
<221> CDS
<222> (387)..(710)

<400> 19
gatatccacc atg gcc ccc gcc gcc tgg ctg agg agc gcc gcc gcc agg 49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe

            120                 125                 130

cct gag gag gtg gcc atc gtg gag gag ctg ggc tga gcgcgc 716
Pro Glu Glu Val Ala Ile Val Glu Glu Leu Gly
        135                 140

<210> 20
<211> 716
<212> DNA
<213> Artificial

<220>
<223> Description of sequence: DNA sequence of the construct
according to Fig. 1m, (see description p. 40);

<400> 20
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

gccgcaggga cttccctgag gaggtggcca tcgtggagga gctgggctga gcgcgc 716

<210> 21
<211> 31
<212> PRT
<213> Artificial

<220>
<223> Description of sequence: artificial, (see description p. 40);

<400> 21

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 22
<211> 10
<212> PRT
<213> Artificial

<220>
<223> Description of sequence: artificial, (see description p. 40);

<400>  22

Arg Arg Asp Phe Pro Glu Glu Val Ala Ile
1               5                   10


<210>  23
<211>  14
<212>  PRT
<213>  Artificial

<220>
<223>  Description of sequence: artificial, (see description p. 40);

<400>  23

Arg Arg Asp Phe Pro Glu Glu Val Ala Ile Val Glu Glu Leu
1               5                   10


<210>  24
<211>  14
<212>  PRT
<213>  Artificial

<220>
<223>  Description of sequence: artificial, (see description p. 40);

<400>  24

Arg Arg Asp Phe Pro Glu Glu Val Ala Ile Ala Glu Glu Leu
1               5                   10



<210>  25
<211>  7
<212>  PRT
<213>  Artificial

<220>
<223>  Description of sequence: partial sequence derived from IP2

<400>  25

Asp Phe Pro Glu Glu Val Ala
1               5


<210>  26
<211>  10
<212>  PRT
<213>  Artificial

<220>
<223>  Description of sequence: sequence derived from SEQ ID NO: 22,
       wherein the first two alanines have been replaced by two
       arginines

<400>  26

Ala Ala Asp Phe Pro Glu Glu Val Ala Ile
1               5                   10


<210>  27
<211>  14
<212>  PRT

<213>  Artificial

<220>
<223>  Description of sequence: sequence derived from SEQ ID NO: 23,
      wherein the first two alanines have been replaced by two
      arginines

<400>  27

Ala Ala Asp Phe Pro Glu Glu Val Ala Ile Val Glu Glu Leu
1               5                   10


<210>  28
<211>  14
<212>  PRT
<213>  Artificial

<220>
<223>  Description of sequence: sequence derived from SEQ ID NO: 24,
      wherein the first two alanines have been replaced by two
      arginines

<400>  28

Ala Ala Asp Phe Pro Glu Glu Val Ala Ile Ala Glu Glu Leu
1               5                   10


<210>  29
<211>  15
<212>  PRT
<213>  Artificial

<220>
<223>  Description of sequence: sequence derived from SEQ ID NO: 2,
      wherein the first two arginines have been replaced by two
      alanines

<400>  29

Ala Ala Asp Phe Pro Glu Glu Val Ala Ile Val Glu Glu Leu Gly
1               5                   10                  15


<210>  30
<211>  15
<212>  PRT
<213>  Artificial

<220>
<223>  Description of sequence: sequence derived from SEQ ID NO: 3,
      wherein the first two arginines have been replaced by two
      alanines

<400>  30

Ala Ala Asp Phe Pro Glu Glu Val Ala Ile Ala Glu Glu Leu Gly
1               5                   10                  15


<210>  31
<211>  46
<212>  PRT
<213>  Artificial

<220>
<223>  Description of sequence: sequence derived from GLP-1-IP2-fragment

(murine)

<400>  31

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Phe Pro Glu Glu Val Ala Ile Ala Glu Glu Leu Gly
        35                  40                  45

<210>  32
<211>  46
<212>  PRT
<213>  Artificial

<220>
<223>  Description of sequence: sequence derived from GLP-1-IP2-fragment
       (murine)

<400>  32

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Ala Glu Glu Leu Gly
        35                  40                  45

<210>  33
<211>  46
<212>  PRT
<213>  Artificial

<220>
<223>  Description of sequence: sequence derived from GLP-1-IP2-fragment
       (murine)

<400>  33

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Ala Glu Glu Leu Gly
        35                  40                  45

<210>  34
<211>  46
<212>  PRT
<213>  Artificial

<220>

<223>  Description of sequence: sequence derived from GLP-1-IP2-fragment
       (murine)

<400>  34

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Ala Ala Ala Leu Gly
        35                  40                  45

<210>  35
<211>  36
<212>  PRT
<213>  Artificial

<220>
<223>  Description of sequence: sequence derived from GLP-1-IP2-fragment
       (murine)

<400>  35

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro
        35

<210>  36
<211>  40
<212>  PRT
<213>  Artificial

<220>
<223>  Description of sequence: sequence derived from GLP-1-IP2-fragment
       (murine)

<400>  36

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala
        35                  40

<210>  37
<211>  51
<212>  PRT
<213>  Artificial

<220>
<223>  Description of sequence: sequence derived from GLP-1-IP2-fragment
      (murine)

<400>  37

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Cys
    50

<210>  38
<211>  46
<212>  PRT
<213>  Artificial

<220>
<223>  Description of sequence: sequence derived from GLP-1-IP2-fragment
      (human)

<400>  38

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Phe Pro Glu Glu Val Ala Ile Val Glu Glu Leu Gly
        35                  40                  45

<210>  39
<211>  46
<212>  PRT
<213>  Artificial

<220>
<223>  Description of sequence: sequence derived from GLP-1-IP2-fragment
      (human)

<400>  39

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Val Glu Glu Leu Gly
        35                  40                  45

<210>  40
<211>  46
<212>  PRT
<213>  Artificial

<220>
<223>  Description of sequence: sequence derived from GLP-1-IP2-fragment
       (human)

<400>  40

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Val Glu Glu Leu Gly
        35                  40                  45

<210>  41
<211>  46
<212>  PRT
<213>  Artificial

<220>
<223>  Description of sequence: sequence derived from GLP-1-IP2-fragment
       (human)

<400>  41

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Val Ala Ala Leu Gly
        35                  40                  45

<210>  42
<211>  51
<212>  PRT
<213>  Artificial

<220>
<223>  Description of sequence: sequence derived from GLP-1-IP2-fragment
       (human)

<400>  42

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Cys
    50

<210>  43
<211>  31
<212>  PRT
<213>  Artificial

<220>
<223>  Description of sequence: sequence according to formula (I)
       (see description, p. 1)

<220>
<221>  misc_feature
<222>  (31)..(31)
<223>  Xaa = NH2, when sequence is GLP-1(7-36) amide, or Xaa = Gly-OH,
       when sequence is GLP-1(7-37) amide,

<400>  43

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Xaa
            20                  25                  30

<210>  44
<211>  31
<212>  PRT
<213>  Artificial

<220>
<223>  Description of sequence:  sequence according to formula (II)
       (see description, p. 13)

<220>
<221>  misc_feature
<222>  (1)..(1)
<223>  Xaa is L-histidine, D-histidine, desamino-histidine,
       2-amino-histidine, 3-hydroxy-histidine, homohistidine,
       N-acetyl-histidine, a-fluoromethyl-histidine, a-methyl-histidine,
       3-pyridylalanine, 2-pyridylalanine or 4-pyridylalanine;

<220>
<221>  misc_feature
<222>  (2)..(2)
<223>  Xaa is Ala, Gly, Val, Leu, Ile, Lys, or Aib;

<220>
<221>  misc_feature
<222>  (2)..(2)
<223>  Xaa is alternatively (1-aminocyclopropyl) carboxylic acid, (1-
       aminocyclobutyl) carboxylic acid, (1-aminocyclopentyl) carboxylic
       acid, (1-aminocyclohexyl) carboxylic acid, (1-aminocycloheptyl)
       carboxylic acid, or (1-aminocyclooctyl) carboxylic acid,

<220>
<221>  misc_feature
<222>  (10)..(10)
<223>  Xaa is Val or Leu; Xaa is is Ser, Lys or Arg;

<220>
<221>  misc_feature
<222>  (12)..(12)
<223>  Xaa is Ser, Lys or Arg;

<220>
<221>  misc_feature
<222>  (13)..(13)
<223>  Xaa is Tyr or Gln

<220>
<221>  misc_feature
<222>  (14)..(14)
<223>  Xaa is Leu or Met;

<220>
<221>  misc_feature
<222>  (16)..(16)
<223>  Xaa is Gly, Glu or Aib;

<220>
<221>  misc_feature
<222>  (17)..(17)
<223>  Xaa is Gln, Glu, Lys or Arg ;

<220>
<221>  misc_feature
<222>  (19)..(19)
<223>  Xaa is Ala or Val ;

<220>
<221>  misc_feature
<222>  (20)..(20)
<223>  Xaa is Lys, Glu or Arg;

<220>
<221>  misc_feature
<222>  (21)..(21)
<223>  Xaa is Glu or Leu;

<220>
<221>  misc_feature
<222>  (24)..(24)
<223>  Xaa is Ala, Glu or Arg;

<220>
<221>  misc_feature
<222>  (27)..(27)
<223>  Xaa is Val or Lys;

<220>
<221>  misc_feature
<222>  (28)..(28)
<223>  Xaa is Lys, Glu, Asn or Arg;

<220>
<221>  misc_feature
<222>  (29)..(29)
<223>  Xaa is Gly or Aib;

<220>
<221>  misc_feature
<222>  (30)..(30)
<223>  Xaa is Arg, Gly or Lys or amide or absent;

<220>
<221>  misc_feature
<222>  (31)..(31)

<223>  Xaa is Gly, Ala, Glu, Pro, Lys, amide or is absent;

<400>  44

Xaa Xaa Glu Gly Thr Phe Thr Ser Asp Xaa Ser Xaa Xaa Xaa Glu Xaa
1               5                   10                  15

Xaa Ala Xaa Xaa Xaa Phe Ile Xaa Trp Leu Xaa Xaa Xaa Xaa Xaa
            20                  25                  30

<210>  45
<211>  31
<212>  PRT
<213>  Artificial

<220>
<223>  Description of sequence: sequence according to formula (III)
       (see description, p. 13)

<220>
<221>  misc_feature
<222>  (1)..(1)
<223>  Xaa is L-histidine, D-histidine, desamino-histidine,
       2-amino-histidine, -hydroxy-histidine, homohistidine,
       N-acetyl-histidine, a-fluoromethyl-histidine, a-methyl-histidine,
       3-pyridylalanine, 2-pyridylalanine or 4-pyridylalanine;

<220>
<221>  misc_feature
<222>  (2)..(2)
<223>  Xaa is Ala, Gly, Val, Leu, Ile, Lys or Aib;

<220>
<221>  misc_feature
<222>  (2)..(2)
<223>  Xaa is alternatively (1-aminocyclopropyl) carboxylic acid, (1-
       aminocyclobutyl) carboxylic acid, (1-aminocyclopentyl) carboxylic
       acid, (1-aminocyclohexyl) carboxylic acid, (1-aminocycloheptyl)
       carboxylic acid, or (1-aminocyclooctyl) carboxylic acid;

<220>
<221>  misc_feature
<222>  (12)..(12)
<223>  Xaa is Ala, Gly, Val, Leu, Ile, Lys or Aib;

<220>
<221>  misc_feature
<222>  (12)..(12)
<223>  Xaa is alternatively (1-aminocyclopropyl) carboxylic acid, (1-
       aminocyclobutyl) carboxylic acid, (1-aminocyclopentyl) carboxylic
       acid, (1-aminocyclohexyl) carboxylic acid, (1-aminocycloheptyl)
       carboxylic acid, or (1-aminocyclooctyl) carboxylic acid;

<220>
<221>  misc_feature
<222>  (16)..(16)
<223>  Xaa is Gly, Glu or Aib;

<220>
<221>  misc_feature
<222>  (17)..(17)
<223>  Xaa is Gln, Glu, Lys or Arg;

<220>
<221>  misc_feature

<222>  (20)..(20)
<223>  Xaa is Lys, Glu or Arg;

<220>
<221>  misc_feature
<222>  (24)..(24)
<223>  Xaa is Ala, Glu or Arg;

<220>
<221>  misc_feature
<222>  (28)..(28)
<223>  Xaa is Lys, Glu or Arg;

<220>
<221>  misc_feature
<222>  (29)..(29)
<223>  Xaa is Gly or Aib;

<220>
<221>  misc_feature
<222>  (30)..(30)
<223>  Xaa is Arg or Lys, amide or is absent;

<220>
<221>  misc_feature
<222>  (31)..(31)
<223>  Xaa is Gly, Ala, Glu or Lys, amide or is absent,

<400>  45

Xaa Xaa Glu Gly Thr Phe Thr Ser Asp Val Ser Xaa Tyr Leu Glu Xaa
1               5                   10                  15


Xaa Ala Ala Xaa Glu Phe Ile Xaa Trp Leu Val Xaa Xaa Xaa Xaa
            20                  25                  30

## Description

The present invention relates to novel GLP=1 fusion peptides *conjugated to polymer constituent(s)* which have extended C termini, are resistant to endopeptidase IV inactivation, may be expressed at high levels in transformed animal cells, and are e.g. useful in the treatment of type 2 diabetes.

The glucagon gene is well studied; see e.g White, J.W. et al., 1986 Nucleic Acid Res. 14(12) 4719-4730. The preproglucagon molecule as a high molecular weight precursor molecule is synthesised in pancreatic alpha cells and in the jejunum and colon :L cells. Preproglucagon is a 180 amino acid long prohormone and its sequence contains, in addition to glucagon, two sequences of related structure: glucagon like peptide-1 (GLP-1) and glucagon-like peptide 2 (GLP-2). In the preproglucagon molecule, between GLP-1 and GLP-2 is a 17 amino acid peptide sequence (or rather a 15 amino acid sequence plus the C-terminal RR cleavage site), intervening: peptide 2 (IP2). The IP2 sequence (located between GLP-1 and -2 in the precursor molecule) is normally cleaved proteolytically after aa 37 of GLP-1. The preproglucagon module is therefore cleaved into various peptides, depending on the cell; and the environment, including GLP-1 (1-37), a 37 amino, acid peptide in its unprocessed form. Generally, this processing occurs in the pancreas and the intestine. The GLP-1 (1-37) sequence can be further protib6lytically, processed into active GLP-1 (7-37), the 31 amino acid processed form, or GLP-1 (7-36) amide. Accordingly, the designation GLP-1(7-37) designates that the fragment in question comprises the amino acid residues from (and including) number 7 to (and including) number 37 when counted from the N-terminal end of the parent peptide, GLP-1. The amino acid sequence of GLP-1(7-36)amide and of GLP-1(7-37) is given in formula I (SEQ ID No.: 43):
His-Ala-Glu-Gly-Thr-Phe-Thr-Ser-Asp-Val-Ser-Ser-Tyr-Leu-Glu-Gly-Gln-Ala=Ala-Lys-Glu-Phe=Ile-Ala-Trp-Leu-Val-Lys-Gly-Arg-X (1), which shows GLP-1(7-36)amide, when X is NH2, and GLP-1(7-37), when X is Gly-OH.

GLP-1 is a gut hormone and is the most potent endogenous insulinotropic agent with actions that include stimulating adenylate cyclase and protein kinase activity in the beta-cell. Physiologically, together with gastric inhibitory polypeptide from the upper gut, it functions as an incretin hormone lowering the blood glucose level. Accordingly, GLP-1, secreted in response to food intake, has e.g. multiple effects on the stomach, liver, pancreas and brain that work in concert to regulate blood sugar. Conseqently, Glucagon-like peptide GLP-1(7-36) amide, and its non-amidated analogue GLP-1(7-37) have attracted considerable interest because of their potent actions on carbohydrate metabolism and its potential applicability to the treatment of diabetes, including type 2 diabetes. Type 2 diabetes is characterized by insulin resistance, since cells do not respond appropriately when insulin is present. This is a more complex problem than type 1 diabetes. Type 2 diabetes may go unnoticed for years in a patient before diagnosis, since the symptoms are typically milder (no ketoacidosis) and can be sporadic. However, severe complications can result from unnoticed type 2 diabetes, including renal failure, and coronary heart disease. This leads to increased morbidity and mortality.

GLP-1 (7-36) amide or GLP-1(7-37) is short-tived in serum. The peptide is cleaved, by dipeptidyl peptidase IV (DPP-IV) between residues 8 and 9. The cleaved peptide is inactive. Thus GLP-1, administered exogenously, is extremely short-lived and has limited utility in therapeutic applications.

Various attempts have been made to synthesise stabilised (against DPP-IV) analogues of naturally occurring GLP-1 (GLP-1(7-37)). In particular, the 8. residue, which in vivo is Ala, was replaced by another residue, for instance, Gly, Ser or Thr (Burcelin, R., et al. (1999) Metabolism 48, 252-258). The Gly8 or G8 analogue has been extensively tested, both as synthesised molecule, and produced by cell lines genetically engineered to secrete the mutant polypeptide (Burcelin, R., et a1 (1999) Annals of the New York Academy of Sciences 875: 277-285). Various other modifications have been introduced into GLP-1 (7-37) to enhance its in vivo stability without compromising its biological activity. However, all of these approaches did not achieve any significant therapeutic significance due to considerable problems involved.

In WO/9953064, Thorens, B. discloses a strategy for creating a multimeric GLP-1 expression cassette which can be incorporated into a variety of cell types which are publicly available immortalised cell lines and dividing primary cell cultures. Examples include EGF-responsive neurospheres, bFGF-responsive neural progenitor stem cells from the CNS of mammals, while the worked example uses baby hamster kidney, BHK cells. The implanted transfected cells were said to have been used to treat successfully diabetic mice, allowing glucose control equivalent substantially to non-diabetic controls. However, this techniques does not comply with the requirements of a treatment to be administered routinely to diabetes patients.

Another approach to stabilize the glucose level exogeneously is based on a new class of medicines known as incretin mimetics under investigation for the treatment of type 2 diabetes. Exenatide (Byetta®) is a synthetic version of a natural compound found in the saliva of the Gila monster lizard. In clinical trials, an incretin mimetic (exenatide) has demonstrated reductions in blood sugar and improvements in markers of beta cell function. However, Exenatide exhibits only certain effects of human incretin hormone glucagon-like peptide-1 (GLP-1).

In summary, at present there is no efficient diabetes type 2 therapy available, which allows to lower the blood glucose level on the basis of GLP-1, in other words to provide a therapy which reflects the entire spectrum of beneficial effects known for GLP-1, e.g. its activity in physiological concentrations to powerfully reduce the rate of entry of nutrients into the circulation by a reduction of gastric emptying rate in obese subjects or its insulin stimulating activity. Therefore, it is an object of the present invention to provide GLP-1 based peptide molecules which are biologically active and resistant towards proteolytic degradation. It is highly desirable to provide GLP-1 based peptide molecules with the highest in vivo stability possible.

The present invention relates to a fusion peptide comprising as component (I) N-terminally a GLP-1 (7-35, 7-36 or 7-37) sequence, as component (II) C-terminally a peptide sequence of at least 9 amino acids or a functional fragment, variant or derivative thereof, (hereinafter: "fusion peptide") and, in addition, at least one synthetic polymer or a natural polymer, e.g. polyamino acids. The at least one polymer constituent is typically covalently coupled to the fusion peptide subunit. "Conjugated" in the meaning of the present invention is intended to mean "chemically coupled". "Chemically coupled" is intended to mean coupled via covalent or non-covalent bondings. While covalent bonding is preferred, the polymer constituent may also be linked to the fusion peptide via complexation without covalent linkage, e.g. via hydrogen bonding or electrostatic, hydrophobic, etc. interaction. The entire complex containing the fusion peptide and the polymer is termed hereinafter "conjugate complex" or "conjugate molecule".

The present invention is based on the finding that the resulting inventive conjugate molecule is even further protected against the proteolytic degradation in vivo, mainly due to proteolytic endopeptidase IV activity. The inventive conjugate complex having an inventive peptide of at least two components (I) and (II) and the synthetic polymer exhibits GLP-1's biologically activity and, simultaneously, confers stability to the GLP-1 as its component (I) by a C-terminal elongation. Accordingly, by conjugating the fusion peptide to a polymer its in vivo stabilisation is increased considerably.

The polymer used herein can be a physiologically acceptable polymer which includes polymers which are soluble in an aqueous solution or suspension and have no negative impact, such as side effects, to mammals upon administration of the fusion peptide in a pharmaceutically effective amount. There is no particular limitation to the physiologically acceptable polymer used according to the present invention. The polymer may be of synthetic nature or may be naturally occurring polymer (natural polymer, e.g. a protein). Specific embodiments of polymers are disclosed hereinafter.

One, two, or three polymer constituents may be covalently attached to the fusion peptide subunit, with one polymer constituent being preferred, to form the inventive conjugate molecule. However, in specific embodiments more than three polymer constituents may be provided per fusion peptide subunit. The constituents may be covalently coupled to either component (I) or component (II) of the fusion peptide or both of them. It is preferred to couple at least one of the polymer constituents to component (II). If one or more polymer constituent(s) is/are coupled to component (I) it/they is/are preferred to be coupled to the N-terminus or to the side chains of serine, threonine, tyrosine, aspartate, glutamate, lysine or arginine residues. Preferably, the side chains of one or more of residues Thr 11, Thr 13, Asp15, Ser 17, Ser 18, Tyr 19, Glu 21, Lys 26, Glu 27, Lys 34, Arg 36 of component (I) are used for coupling purposes. If the naturally occurring sequence of IP2 is used as component (II) one or more of its Arg, Glu and Asp residues will modified at their side chains by a polymer constituent(s).

The N-terminal binding of a polymer, in particular PEG, may offer advantages in purification of the conjugate. It is also believed that the N-terminal binding of a polymer may better preserve the bioactivity compared with random polymer binding to any other residue, e.g. any other lysine residue. Accordingly, in a preferred embodiment, at least one polymer constituent is located at the N-terminus of the fusion peptide. If PEGylation is used, PEGylation of terminal or side chain carboxyl groups or the epsilon-amino group of lysine occurring in the inventive peptide, confers resistance to oxidation and is also within the scope of the present invention.

Polymers used in the fusion peptide according to the present invention can be prepared by polymerising in the usual way monomers of the following formula: 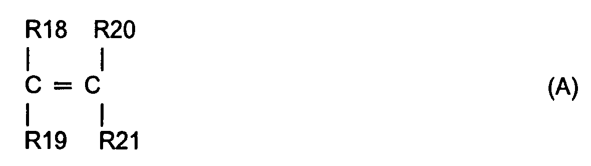 in which
R18 is selected from hydrogen, halogen, C1-4 alkyl and groups COOR22 in which R22 is hydrogen and C1-4 alkyl;
R19 is selected from hydrogen, halogen and C1-4 alkyl;
R20 is selected from hydrogen, halogen, C1-4 alkyl and groups COOR22 provided that R18 and R20 are not both COOR22; and
R21 is a C1-10 alkyl, a C1-20 alkoxycarbonyl, a mono- or di-(C1-20 alkyl) amino carbonyl, a C6-20 aryl (including alkaryl), a C7-20 aralkyl, a C6-20 aryloxycarbonyl, a C1-20 aralkyloxycarbonyl, a C6-20 arylamino carbonyl, a C7-20 aralkyl-amino, a hydroxyl or a C2-10 acyloxy group, any of which may have one or more substituents selected from halogen atoms, alkoxy, oligo-alkoxy, aryloxy, acyloxy, aclamino, amine (including mono and di-alkyl amino and trialkylammonium), carboxyl, sulphonyl, phosphoryl, phosphino, (including mono- and di-alkyl phosphine and tri-alkylphosphonium), zwitterionic and hydroxyl groups;
or R21 and R20 or R21 and R19 may together form -CONR23CO in which R23 is a C1-20 alkyl group.

The ethylenically unsaturated monomers may polymerise to form a polymer selected from polyalkylene glycol, polyvinylpyrrolidone, polyacrylate, poloyoxazoline, polyvinylalcohol, polacrylamid, polymethacrylmide or a HPMA copolymer. Alternatively, the polymer may be susceptible to enzymatic or hydrolytic degradation, such as polyesters, polyacetales, poly(orthoesters), polycarbonates and polyamides.

The polymer may be a homopolymer or a copolymer of at least two of the above mentioned components. The copolymer can be a block copolymer or a random copolymer.

Specific particularily preferred examples of the polymers include, but are not limited to, poly alkylene glycols such as polyethylene glycol (PEG), polypropylene glycol (PPG), copolymers of ethylene glycol and propylene glycol and the like, polyoxyethylated polyol, polyolefinic alcohol, polyvinylpyrrolidone, polyhydroxyalkyl methacrylamide, polyhydroxyalkyl methacrylate, such as polyhydroxyethylen methycrylate, polyacrylate, polysaccharides, poly([alpha]-hydroxy acid), polyvinyl alcohol, polyphosphazene, polyoxazoline, poly(N-acryloylmorpholine), polyvinylethyl ether, polyvinlyacetale, polylactic glycolic acid, polylactic acid , lipid polymer, chitin, hyaluronuic acid, polyurethyne, polysialic acid cellulose triacetat, cellulose nitrate and combinations of any of the foregoing.

Further specific polymers may be selected from N-(2-hydroxypropyl)methacrylamide (HPMA), and poly(lactide-co-glycolide) (PLGA) copolymers, poly(N-vinylpyrrolidone-co-acrylic acid-g-PEG) (VAP), a copolymer of N-vinylpyrrolidone with acrylic acid, and a copolymer of N-vinylpyrrolidone with maleic anhydride.

In another preferred embodiment of the present invention the polymer has the following general formula (B) or (C) 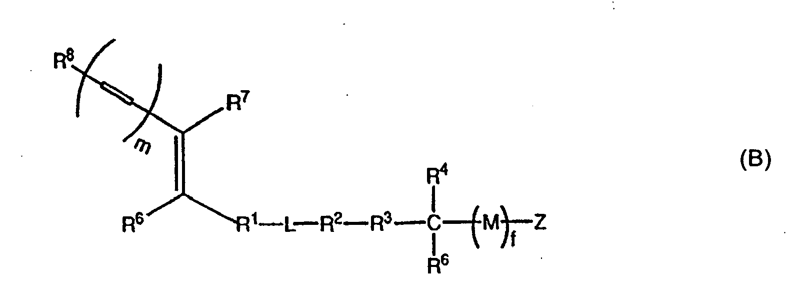 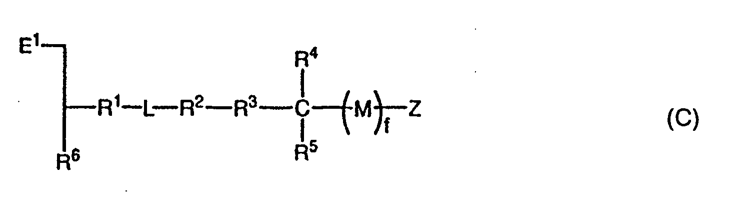 wherein
R1 is a electron withdrawing group known in the art;
R2 is selected from C=O, C(=O)NR9 or a bond; wherein R9 is H or a C1-4 alkyl;
R3 is selected from the group consisting of a C1-C20 alkylene; C3-C8 cycloalkylene; C(=C)R10, C(=O)NR11, C1-C20 alkoxy, C2-C20 alkenylene, C2-C20 alkenyl oxiranylene; arylene, heterocyclene, aralkylene; in which 1 to all of the hydrogen atoms are replaced with halogen; C1-C6 alkyl susbtituted with 1 to 2 substituents selected from the group consisting of C1-C4 alkoxy, aryl, heterocyclyl, C(=O)R10, C(=O)NR11R12, oxiranyl and glycidyl;
R10 is alkylene of from 1 to 20 carbon atoms, alkoxy from 1 to 20 carbon atoms, oligo(alkoxy) in which each alkoxy group has from 1 to 3 carbon atoms, aryloxy or heterocycyloxy; and of which groups may have substituents selected from optionally substituted alkoxy, oligoalkoxy, amino (including mono- and di-alkyl amino and trialkyl ammonium, which alkyl groups, in turn, may have substituents selected from acyl, alkoxycarbonyl, alkenoxycarbonyl, aryl and hydroxy) and hydroxyl groups;
R11 and R12 are independently H or alkyl of from 1 to 20 carbon atom or R11 and R12 may be joined together to form an alkanediyl group of from 2 to 5 carbon atoms, thus froming a 3- to 6-membered ring;
R4 and R5 are each independently selected from H, Z, halogen, C1-C20 alkyl, C3-C8 cycloalkyl, OH, CN, C2-C20 alkenyl, C2-C20 alkenyl oxiranyl, C(=O)R13, glycidyl, aryl, heterocyclyl, arylkyl, aralkenyl, in which 1 to all of the hydrogen atoms are replaced with halogen, C1-C6 alkyl substituted with 1 to 2 substituents selected from the group consisting of C1-C4 alkoxy, aryl, heterocyclyl, C(=O)R10, C(=O)NR11R12, oxiranyl and glycidyl;
where R13 is alkyl of from 1 to 20 carbon atoms, alkoxy of from 1 to 20 carbon atoms, oligo(alkoxy) in which each alkoxy group has 1 to 3 carbon atoms, aryloxy or heterocyclyloxy any of which groups may have substituents selected from optionally susbstituted alkoxy, oligoalkoxy, amino (including mono- and di-alkyl amino and trialkyl ammonium, which alkyl groups, in turn may have susbtituents selevted from acyl, alkoxycarbonyl, alkenoxycarbonyl, and aryl and hydroxy) and hydroxyl groups; and
L is a linking group;
Z is selected from the group consisting of Cl, Br, I, OR14, SR15, SeR15, OP(=O)R15, OP(=O)(=OR15)₂**,** O-N(R15)₂, and S-C(=S)N(R15)₂, where R14 is alkyl of from 1 to 20 carbon atoms in which each of the hydrogen atoms may be independently replaced by halide, R15 is aryl or a straight or branched C1-C20 alkyl group, and where an N(R15)₂ group is present, the two R15 groups may be joined to form a 5- or 6-membered heterocyclic ring;
R6 is CH₂E2 , H or C1-C4 alkyl;
R7 and R8 are each independently selected from H and C1-C4 alkyl;
m is 0-4;
f is 1 to 500, preferably 5 to 50; and
groups M are the same or different and are residues derived from ethylenically unsaturated monomers, in particular the monomers as defined in formula (A)

The polymers of formulas (B) and (C) can be prepared by a polymerisation process in which ethylenically unsaturated monomers, in particular the ethylenically unsaturated monomer of formula (A) are polymerised as known in the art by a living radical polymerisation process in the presence of an initiator of the general formula (D) and (E), 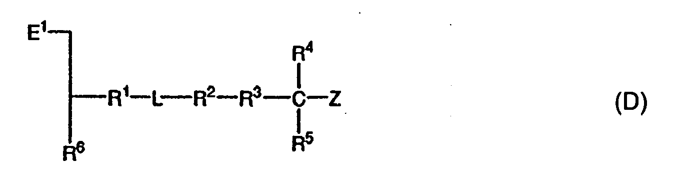 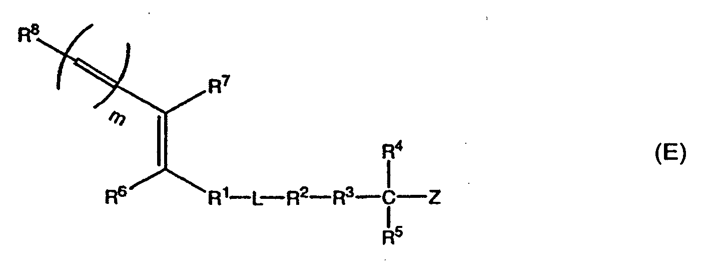 wherein R1 to R8, L, E1, Z and m are defined as above.

A preferred polymer to be used in the present invention is polyethylene glycol (PEG) and modified versions of PEG, such as methoxyPEG (mPEG). The conjugation of peptides with water-soluble PEG polymers slows down renal filtration, therefore increasing their residence time in circulation. This technology enlarges the size of an active molecule by attaching a web-like shield of hydrated PEG polymer chains around the molecule. One of the key benefits of this modification is to increase clearance half-life and provide the possibility of the drug staying in systemic circulation for longer. Furthermore, this technology increases molecular stability, changes the volume of distribution within the body and reduces immune reactions, making the drug more effective. Polymer molecules attached to the fusion peptide creates steric hindrances for the interaction of 'protected' polypeptides with active sites of proteases.

The physiologically acceptable polymer is not limited to a particular structure and can be linear (e.g. alkoxy PEG or bifunctional PEG), i.e. not branched, branched or multi-armed (e.g. forked PEG or PEG attached to a polyol core), dendritic, or with degradable linkages. Moreover, the internal structure of the polymer can be organized in any number of different patterns and can be selected from the group consisting of homopolymer, alternating copolymer, random copolymer, block copolymer, alternating tripolymer, random tripolymer, and block tripolymer.

As used herein, the term polyethylene glycol ("PEG") is inclusive and means any of a series of polymers having the general formula:

HO-CH₂CH₂O-(CH₂CH₂O)ₙ-CH₂CH₂-OH

wherein n ranges from about 5 to 4 000. PEG also refers to the structural unit:

-CH₂CH₂O-(CH₂CH₂O)ₙ-CH₂CH₂-

wherein n ranges from about 5 to about 4 000. Thus, by PEG is meant modified PEGs including methoxy-PEGs; PEGs having at least one terminal moiety other than a hydroxyl group which is reactive with another moiety; branched PEGs; pendent PEGs; forked PEGS; and the like.

The polyethylene glycol useful in the practice of this invention normally has an average molecular weight of from about 100 to 200 000 Daltons. Molecular weights of from about 1 000 to 40 000 Dalton are somewhat more commonly used. Molecular weights of from about 300 to 8 000, and in particular, from about 500 to about 5 000 daltons, are somewhat typical and, evetually, preferred.

PEG is useful in biological applications because it has properties that are highly desirable and is generally approved for biological or biotechnical applications. PEG typically is clear, colorless, odorless, soluble in water, stable to heat, inert to many chemical agents, does not hydrolyze or deteriorate, and is generally nontoxic. Polyethylene glycol is considered to be biocompatible, which is to say that PEG is capable of coexistence with living tissues or organisms without causing harm. More specifically, PEG, in itself, is normally considered nonimmunogenic, which is to say that PEG does not tend to produce an immune response in the body. Desirable terminal activating groups by which PEG can be attached to various peptides should not appreciably alter the nonimmunogenic character of the PEG, so as to avoid immunogenic effects. Desirable PEG conjugates tend not to produce a substantial immune response or cause clotting or other undesirable effects.

PEG is a highly hydrated random coil polymer that can shield proteins or peptides from enzymatic digestion, immune system molecules and cells, and can increase the hydrodynamic volume to slow reticuloendothelial system (RES) clearance. PEG is a useful polymer having the properties of water solubility as well as solubility in many organic solvents. The unique solubility properties of PEG allow conjugation (PEGylation) to certain compounds with low aqueous solubility, with the resulting conjugate being water-soluble.

The polymer used in this invention normally can be linear or branched. Branched polymer backbones are generally known in the art. Typically, a branched polymer has a central core moiety and a plurality of linear polymer chains linked to the central core. PEG is commonly used in branched forms that can be prepared by addition of ethylene oxide to various polyols, such as glycerol, pentaerythritol and sorbitol. For example, the four-arm, branched PEG prepared from pentaerythritol is shown below:

C(CH₂-OH)₄+nC₂H₄O -> C[CH₂O-(CH₂CH₂O)ₙ-CH₂CH₂-OH]₄

The central moiety can also be derived from several amino acids. An example is lysine.

The branched polyethylene glycols can be represented in general form as R(-PEG-OH)n in which R represents the core moiety, such as glycerol or pentaerythritol, and n represents the number of arms. Suitable branched PEGs can be prepared in accordance with U.S. Pat. No. 5,932,462, the contents of which are incorporated herein in their entirety by reference. These branched PEGs can then be used in accordance with the teachings herein.

Forked PEGs and related polymers may also be useful in the practice of the invention. The term "forked" is used to describe those PEGs that are branched adjacent at least one terminus thereof. The polymer has a branched moiety at one end of the polymer chain and two free reactive groups, one on each end of the branched moiety, for covalent attachment to another molecule. Each reactive moiety can have a tethering group, including, for example, an alkyl chain, linking a reactive group to the branched moiety. Thus, the branched terminus allows the polymer to react with two molecules to form conjugates. The forked PEGs can be either linear or branched in the backbone attached to the branched terminus.

Apart from the PEGylation technology, certain polymers that are not large enough to prevent the renal clearance, but attach themselves, together with the conjugated fusion peptide, to natural long-circulating blood plasma components have been used to prepare derivatives to extend circulation longevity. For example, conjugation with poly(styrene-co-maleic acid anhydride (SMA)) with a molecular weight (MW) as low as 1.5 kDa may increase the circulation time of the fusion peptide via binding to plasma albumin.

Moreover, various polymers based on saccharidic units may be utilized to stabilize the fusion peptide by conjugation, e..g. dextrans or certain acidic polysaccharides, such as pectin, algesic acid, hyaluronic acid and carrageenan, can be coupled to fusion peptide subunits to produce both soluble and insoluble, preferably soluble products. Such polysaccharides are polyanionic, typically derived from food plants.

In another embodiment of the present invention, the physiologically acceptable polymer is polysialic acid (PSA) and derivatives thereof. PSA can be bound to a fusion peptide by well known methods. In one embodiment of the invention the polysaccharide compound may be naturally occurring polysaccharide, a derivative of a naturally occurring polysaccharide, or a naturally occurring polysaccharide derivative. The polysaccharide portion of the compound has more than 5, typically at least 10, and in another embodiment at least 20 to 50 sialic acid residues in the polymer chain. Readily available polysaccharide compounds may have up to 500 saccharide residues in total, but usually have fewer than 300 residues in the polymer chain. Generally, all of the saccharide residues in the compound are sialic acid residues.

The polysialic acid portion at least of the polysaccharide compound, and in one embodiment the entire compound, is highly hydrophilic. Hydrophilicity is conferred primarily by the pendant carboxyl groups of the sialic acid units, as well as the hydroxyl groups. The saccharide unit may contain other functional groups, such as, amine, hydroxyl or sulphate groups, or combinations thereof. These groups may be present on naturally occurring saccharide compounds, or introduced into derivative polysaccharide compounds.

Polysaccharide compounds for use in the present invention are also those produced by bacteria. Some of these naturally occurring polysaccharides are known as glycolipids. It is particularly advantageous, if the polysaccharide compounds are substantially free of terminal galactose units, which tend to be recognized by galactose receptors of hepatocytes and Kupffer cells.

Still further, N-(hydroxy)succinimidyl ester-terminated glycopolymers obtained via copper(I)-catalysed living radical polymerisation may be used for conjugation purposes. Monomers employed may be based on protected glucose and galactose, glucofuranoside monomer (1) and galactopyranoside monomer (2). The corresponding polymers feature a relatively narrow molecular weight distribution (PDI=1.10-1.31) and Mₙ between 4.5 and 10.2 kDa. Protecting groups may be removed by treatment with formic acid. Primary amino groups of of the fusion peptide subunitmay react with the terminally functional sugar polymers.

Alternatively, fusion peptides of the inventive conjugate molecule may be stabilized by conjugating them with chondroitin sulfates. Products of this combination are usually polyanionic, and very hydrophilic.

The polymer used may be bound components (I) and/or (II) of the fusion peptide and may be a copolymer composed of amino acids (polyamino acids). The amino acids to be used can be the naturally occurring amino acids or synthetic modified amino acids which are well known in the art (see also below). Examples of proteins which can be used are albumin, globulin, glutelin, histone, prolamine, protamine, keratin, fibroin, elastin, collagen, chromatin, lectin, immune globulin, lipoproteines, casein, vitellin, hemoglobin, cytochrome, catalse, rhodopsin, caeruloplasmin, transferrin and ferredoxin, wherein albumin and transferrin are particularily preferred due to their stabilizing effect.

Finally, the fusion peptide may be conjugated with a polymer including (i) a linear polyalkylene glycol moiety and (ii) a lipophilic moiety, wherein the fusion peptide, the linear polyalkylene glycol moiety and the lipophilic moiety are conformationally arranged in relation to one another such that the resulting conjugate molecule has an enhanced in vivo resistance to enzymatic degradation, relative to fusion peptide alone (s. US 040). Alternatively, the physiologically active fusion peptide may be covalently coupled to a physiologically compatible polyethylene glycol modified glycolipid moiety. In such conjugate complex, the physiologically active peptide may be covalently coupled to the physiologically compatible polyethylene glycol modified glycolipid moiety by a labile covalent bond at a free amino acid group of the polypeptide, wherein the liable covalent bond is scissionable in vivo by biochemical hydrolysis and/or proteolysis. The physiologically compatible polyethylene glycol modified glycolipid moiety may advantageously comprise a polysorbate polymer, e.g., a polysorbate polymer comprising fatty acid ester groups selected from the group consisting of monopalmitate, dipalmitate, monolaurate, dilaurate, trilaurate, monoleate, dioleate, trioleate, monostearate, distearate, and tristearate. In such complex, the physiologically compatible polyethylene glycol modified glycolipid moiety may suitably comprise a polymer selected from the group consisting of polyethylene glycol ethers of fatty acids, and polyethylene glycol esters of fatty acids, wherein the fatty acids for example comprise a fatty acid selected from the group consisting of lauric, palmitic, oleic, and stearic acids.

### Polymer Activation

In order to conjugate the fusion peptide subunit of the inventive conjugate molecule to a polymer (which is either already polymerized or in its monomeric form at the time of conjugation), the polymer may first need to be activated. "Activated" means the preliminary attachment of a reactive group onto the polymer. A polymer which has been activated, as well as a polymer which already contains at least one reactive group is referred to as being "active". Many synthetic polymers do not normally contain reactive groups which will react with a protein's s pendant groups. For example, unsubstituted PEG has a hydroxyl group at each end of the linear polymer chain, one or both of which must first be activated before conjugation to a protein. To prevent the potential for cross-linking, it is desirable to activate only one of PEG's two hydroxyl termini to form a "monofunctional" substituted.

Techniques for activating polymers prior to protein conjugation are known to those skilled in the art. See, for example, Greg T. Hermanson, Bioconjugate Techniques, p. 605619 (1996) Academic Press. For example, activation of hydroxyl groups in PEG or mPEG as well as in other natural or synthetic polymers, can be accomplished using trichlorostriazine (TsT; cyanuric acid). (See, inter alia, Abuchowski et al., J. Biol. Chem.252:3582-3586 (1977) and Abuchowski et al., J. Biol. Chem. 252:3578-3581 (1977)). Another method of activating hydroxyl groups is through formation of an amine reactive N-hydroxyl succinimidyl-(NHS) or p-nitrophenyl (Np) carbonate active ester (See Zalipsky et al., Biotechnol. Appl. Biochem. 15:100-114 (1992)).

Similar activation can be achieved when the hydroxyl-containing polymer is first reacted with a cyclic anhydride (succinic or glutaric anhydride) and then the formed carboxyl modified product is coupled with N-hydroxyl succinimide in the presence of carbodiimides resulting in succinimidyl succinate or glutarate type active esters. (See Abuchowski et al., Cancer Biochem. Biophys.7:175-186 (1984).)

An alternative approach to conjugate the fusion peptide with a polymer, preferably PEG, is based on living radical polymerisation using N-succinimidyl ester functionalised polymers (from transition metal mediated living radical polymerisation).

A further method for activating a polymer's hydroxyl groups is through formation of an imidazolyl carbamate intermediate by reaction with N,N'-carbonyldiimidazole (CDI). The CDI-activated polymer reacts with amine groups of protein to form a stable N alkyl carbamate linkage identical to that formed with succinimidyl carbonate chemistry described above. (See Beauchamp petal., Anal. Biochem. 131:25-33 (1983).).

For additional methods on activating synthetic polymers, such as PEG, see inter alia, U.S. Patent No. 5,349,001; U.S. Patent No. 5,359,030; and U.S. Patent No. 5,446,090:

When the polymer to be conjugated to the fusion peptide subunit of the inventive conjugate molecule is a natural polymer such as a serum protein, it is generally preferred to activate the protein subunit by attachment of a multifunctional crosslinking agent as is more fully described in the following section.

### Polymer-Fusion Peptide Conjugation

After activation of the polymer (if necessary), the polymer is conjugated to the individual fusion peptide subunit. In general, polymers can be covalently attached to proteins (in the present case to the fusion peptide) via pendant groups in the protein chain, such as primary amino groups, carboxyl groups, aromatic rings, or thiol groups, all of which may already be present, or can be added by preliminary chemical modification of the protein or by modifying a protein's amino acid sequence, using known embodiments of the present invention.

The ratio of polymer to fusion peptide to be used to carry out the conjugation depends on the characteristics (structure, size, charge, reactivity) of the polymer, as well as the characteristics (number and location of pendant groups, nature of the biological activity) of the individual subunit. It would be a matter of routine experimentation to determine the appropriate ratio by varying the ratio to optimize biological activity and conjugate stability.

There are three major alternatives for a conjugation of PEG to a fusion peptide subunit, namely amin, thiol and hydroxyl PEGylation, which may be used to provide the present conjugate molecule of the invention.

For amine PEGylation the following methods may be employed by using N-Hydroxysuccinimid active esters (NHS-method, which is the most common method for protein PEGylation). By this method, all primary and secondary amino groups are addressed. Result is a stable amid bond. The modification is carried out under mild alkaline conditions e.g. in 50mM bi carbonate buffer pH.8.5 to 9.5. With these conditions the NHS active ester is very reactive. As a rule, the modification is finished after 1 hour at temperatures between 4°C and 25°C. The degree of modification can be easily controlled by the amount (equivalents) of PEG-NHS-reagent in relation to the number of modifiable groups in the fusion peptide.

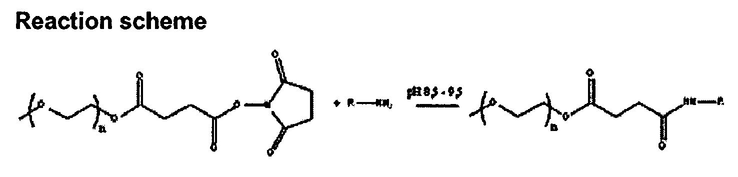

This method allows for quick and efficient coupling, is selective for amino groups and results in very good surface protection because of to the spreading of modified groups. However, it may be less preferable, if the fusion peptide is sensitive to alkaline reaction conditions and/or a homogenous final product is needed, since the NHS-method results in a statistical spreading of the modification on the fusion peptide surface. The pH value has an influence on the reaction. The higher the pH value, the more efficient is the modification of lysine residues of the fusion peptide subuni. At pH values below 7, a selective modification of hisitidin residues is possible. This modification, however, is less stable against alkaline conditions. Preferably, the equivalents of the PEG-reagents in relation to amino-groups range from a ratio of 1:1 to 100:1. Typically, the higher the fusion peptide concentration, the more efficient the PEGylation. Ideally, the fusion peptide concentration is chosen in a range from 0.1 mg/mL und 15 mg/mL.

Alternatively, the reductive amination may be employed. Reductive amination addresses the amino terminus of a fusion peptide and is conducted with PEG-aldehydes. The condensation of the aldehyde with the N-terminal amino acid leads to an instable intermediate (Schiff base). This is reduced to a stable secondary amine by NaCNBH₃. The modification is carried out at slightly acidic conditions e.g. in 50-100mM Na-acetate buffer at pH 5 to 6. The reaction may take 14h-24h at temperatures between 4°C and 25°C. The reaction buffer contains 25mM NaCNBH₃ for the reduction. In order to stop the reaction ethanol amine can be used. The degree of modification is mainly determined by the reaction time and, to a less extends, by temperature and equivalents of PEG-aldehyde.

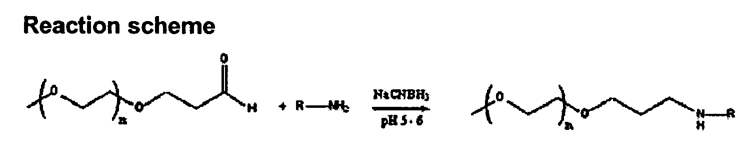

Reductive amination may lead to selective modification of the N-terminus. Preferably, mono-PEGylation is possible by this method. However, it may be less preferable, if short reaction times are required and/or chromatographic steps for the separation of mono-, di- and tri-PEGylated species are not desired. In a preferred embodiment of this method, the concentration of NaCNBH₃ is adjusted. The higher the concentration of NaCNBH₃, the faster the building of a stable end product. Preferably, the PEG-reagents in relation to amino-terminus range from a ratio of 1:1 to 10:1. With regard to the pH-value slightly acidic conditions between pH values of 4.5 and 6 are preferred. The pKs-value of the N-terminal amino group slightly differs from the pKs-value of epsilon amino groups of lysine. Thus, a selective PEGylation of the N-terminus is heavily dependent on the pH value. Again, the higher the fusion peptide concentration, the more efficient the PEGylation. Ideally, the fusion peptide concentration is chosen in a range from 2mg/mL to 15mg/mL.

For thiol PEGylation the following maleimid method may be employed. PEGylation of thiol group is the method of choice, if a protein contains a few or ideally only one sulfhydryl group in the form of a cystein. This method results in stabel thioether bonds. The maleimid method heaviliy depends on the pH value and is carried out at a pH value of 8 e.g. in 50mM bi carbonate buffer. At higher pH values side reactions occur with amine groups. The reaction is carried out for 2 h at 25°C. The reaction can be terminated by the addition of thiol containing reagents such as Ethanthiol, DTE and DTT.

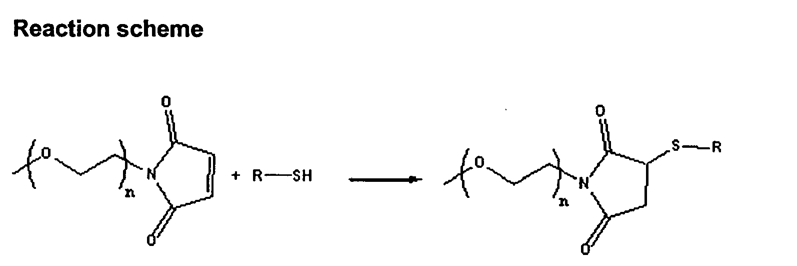

This method is particularly prefered for selective PEGylation of thiol groups, mild reaction conditions and/or for site specific mono PEGylation, if only one free thiol group is present in the (modified) fusion peptide, e.g. introduced by genetic methods. The method my be optimized by an advantageous choice of the reaction parameters, namely e.g. concentration conditions. The higher the fusion peptide concentration, the more efficient the PEGylation. Preferably, the fusion peptide concentration is chosen in a range from 2 mg/mL to 15 mg/mL.

Finally, PEGylation may be achieved by hydroxyl PEGylation, preferably on the basis of the epoxid method. PEG-epoxid reacts with hydroxyl groups under slightly alkaline conditions at pH values between 8.5 and 9.5 e.g. in 50mM bi carbonate buffer forming an ether bond. Frequently side reactions occur with amine and thiol groups. PEG epoxid is very stable in aqueous solution at alkaline pH-values. The coupling is carried out for 14h to 25 h at 4°C to 25°C. The reaction may be easily terminated by adding ethanol.

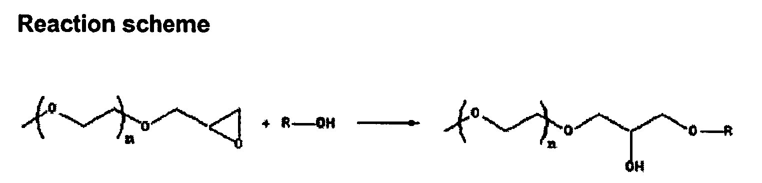

The epoxid methods is preferable, if mild reaction conditions for the reaction with hydroxyl groups are desired. This method may be less suited, if high specificity of the PEGylation and/or short reaction times is/are required. The higher the fusion peptide concentration, the more efficient the PEGylation. Preferably, fusion peptide concentration is chosen in a range from 1mg/mL und 10mg/mL.

In a preferred embodiment of the present invention, unbranched mPEG having an average molecular weight of about 40 000 being activated with a maleimido propionamide group is conjugated to a thiol group of the amino acid cysteine of the fusion peptide. Since the fusion peptide may not contain a cysteine residue in its sequence to which the mPEG shall be conjugated, it can be easily incorporated by standard methods in this art, e.g. at the N- and/or C-terminus of the peptide components (I) and/or (II), preferably the N-terminus of component (I) or the C-terminus of component (II). Alternatively, the cystein residue may also be introduced by an amino acid substitution, preferably a conservative substitution, e.g. substitution of a serine, glycine or alanine residue by a cystein. Conjugation of a fusion peptide via e.g. a thiol group is advantageous, since the fusion peptide typically contains no thiol groups (no cystein residues). Accordingly, the thiol group has to be introduced specifically into the fusion peptide, which in turn allows the conjugation to be located at a specific site of the conjugate molecule.

However, there may also be provided a linker sequence between component (I) and component (II). If necessary, the cysteine residue may be provided by a linker between components (I) and (II), for example a short amino acid sequence of 1 to 10, preferably about 4 amino acids containing at least one cystein residue.

The term "inventive (fusion) peptide" as used herein is a fusion peptide as defined herein, a variant, an analog, a fragment or a derivative thereof, including combinations e.g. a derivatized fragment, analog or variant of a fusion peptide, whereby the fusion peptide contains at least a component (I) and a component (II), which are directly or indirectly covalently linked to one another. The fusion peptide may contain further components, e.g. component (III). The fusion peptide is according to the invention linked to at least one polymer constituent forming the inventive conjugate molecule. The term "GLP-1 peptide" as used herein means GLP-1(7-35, 36 or 37), whereas "modified GLP-1 peptide" is intended to mean any GLP-1 analogue, a GLP-1 derivative, a GLP-1 variant or GLP-1 fragment, including a derivatized fragment, analog or variant of GLP-1 (7-35, 36 or 37), which may occur in either component (I) and (III) of the inventive peptide. The term "GLP-2 peptide" as used herein means GLP-2 (1-33, 34, or 35), whereas "modified GLP-2 peptide" is intended to mean any GLP-2 analogue, fragment or variant, a GLP-2 derivative or a derivative of a GLP-2 analogue, including a derivatized fragment, analog or variant of GLP-2(1-33, 34 or 35). Variants, analogs, fragments and derivatives are categorized as modifications of the unmodified sequence, e.g. GLP-1(7-35, 36 or 37) or GLP-2(1-33, 34 or 35). Within the meaning of the present invention any variant, analog, fragment or derivative has to be functional, e.g. has to exert the same or a similar biological effects as the unmodified GLP-1 peptide.

Preferably, the inventive peptide is a fusion peptide or a variant, analog, fragment or derivative thereof, wherein component (I) contains a sequence having at least 80 %, more preferably at least 85 % and even more preferably at least 90 % sequence homology with SEQ ID No.: 1. SEQ ID No.1 represents the native amino acid sequence of GLP-1(7-37) (length of 31 amino acids), which is strictly conserved among mammalians.

The second component (component (II)) of the fusion peptide according to the invention (or more generally any inventive peptide including analogs, fragments, variants or derivatives of fusion peptides) typically contains a sequence length of at least nine amino acids, which may or may not form a β-turn like structure. A β-turn structure is a typical secondary structure element of proteins or peptides. It is typically formed by four amino acids, which revert the direction of the peptide's or protein's backbone chain direction. The amino acid sequence of component (II) contains at least nine amino acids and, preferably, contains at least one proline or alanine residue in its sequence. Proline residues are common amino acids within the β turn forming tetrameric amino acid sequence. The proline residue is commonly is typically located at position 2 or 3, preferably 2, of the tetrameric β-turn sequence occurring in component (II) of the fusion peptide.

An inventive fusion may typically have in its component (II) sequence length of 9 to 30, preferably 9 to 20, and most preferably 9 to 15 amino acids. Generally spoken, shorter sequences in component (II) may be preferred due to their superior binding activity to the GLP receptor over longer sequences. The sequence of component (II), even though it is not a prerequisite, may preferably be neutral or may have a negative charge at pH7.

A fusion peptide according to the invention is preferred, if its component (II) contains a sequence motif selected from the group consisting of VAIA, IAEE, PEEV, AEEV, EELG, AAAA, AAVA, AALG, DFPE, AADX, AXDX, and XADX, wherein X represents any amino acid (naturally occurring or a modified non-natural amino acid). These tetrameric motifs may be located anywhere in the sequence of component (II). In a particularly preferred embodiment, the inventive fusion peptide component (II) is a peptide sequence being linked to the C-terminus of component (I) by its N-terminal sequence motif selected from the group consisting of AA, XA, AX, RR, RX, and XR, wherein X represents any amino acid (naturally occurring or a modified non-natural amino acid).

A preferred motif of component (II) in an inventive fusion peptide contains the sequence motif SEQ ID No.: 25 (DFPEEVA) or contains a sequence having at least 80% sequence homology with the sequence motif DFPEEVA, which corresponds to a partial sequence of human or murine IP-2.

Particularly preferred is a fusion peptide, wherein component (II) is a peptide sequence containing a sequence according to SEQ ID No.: 22 (RRDFPEEVAI) or SEQ ID No.: 26 (AADFPEEVAI) (all peptide sequences given in the one-letter-code) or a sequence having at least 80% sequence homology with SEQ ID No.: 22 or with SEQ ID No.: 26. SEQ ID No.: 22 is a partial sequence of the full-length (human or murine) IP-2 (intervening peptide 2) sequence, which contains the N-terminal 10 amino acids of the 15 amino acid long full-length IP-2 sequence. SEQ ID No.: 26 is derived from SEQ ID No.: 22 by substitution of the N-terminal (RR) residues by (AA). IP-2 is a preferred example of a β-turn containing peptide sequence. Accordingly, other stronger preferred sequences being contained in component (II) are longer partial amino acid sequences of IP-2, such as the N-terminal 14 amino acid sequence occurring in humans (SEQ ID No.: 23 (RRDFPEEVAIVEEL)) or its murine counterpart (SEQ ID No. 24 (RRDFPEEVAIAEEL)) or a sequence having at least 80% sequence homology with SEQ ID Nos.: 23 or 24. Most preferred as elements being contained in component (II) of the fusion peptide are full-length IP-2 sequences having all 15 amino acids of the natural occurring IP-2 sequence (SEQ ID No.: 2 (RRDFPEEVAIVEELG), human, or SEQ ID No. 3 (RRDFPEEVAIAEELG), murine) or a sequence having at least 80% sequence homology with SEQ ID Nos.: 2 or 3. Within the scope of the present invention are also all mammalian isoforms of IP2 (natural variants of IP2 among mammalians). All sequences mentioned in this paragraph may also be provided with the N-terminal motif (AA), (AX) or (XA) instead of the naturally occurring (RR), e.g. SEQ ID No.: 27 (AADFPEEVAIVEEL), SEQ ID No.: 28 (AADFPEEVAIAEEL), SEQ ID No.: 29 (AADFPEEVAIVEELG), and SEQ ID NO.: 30 (AADFPEEVAIAEELG). More than one copy of a sequence being included into component (II) may be provided, e.g. 2, 3 or even more copies of IP2 or a fragment, variant or analog or derivative of IP2.

Accordingly, an inventive peptide is preferred containing a sequences according to SEQ ID No.: 8 (HAEGTFTSDVSSYLEGQAAKEFIAWLVKGRGRRDFPEEVAIAEELG), i.e. GLP-1(7-37) linked without any linker sequence via its C-terminus to murine IP2 or according to SEQ ID No.: 12 (HAEGTFTSDVSSYLEGQAAKEFIAWLVKGRGRRDF-PEEVAIVEELG), i.e. GLP-1(7-37) linked without any linker sequence via its C-terminus to human IP2. Further preferred inventive variants of the inventive peptides of SEQ ID No.:8 and SEQ ID No.: 12 are SEQ ID No.: 31 (HAEGTFTSDVSSYLEGQAAKEFIAWLVKGRGAADFPEEVAIAEELG), SEQ ID No.: 32 (HAEGTFTSDVSSYLEGQAAKEFIAWLVKGRGRRDFAEEVAIAEELG), SEQ ID No.: 33 (HAEGTFTSDVSSYLEGQAAKEFIAWLVKGRGRRDAAAAVAIAEELG), SEQ ID No.: 34 (HAEGTFTSDVSSYLEGQA-AKEFIAWLVKGRGAADAAAAVAIAAALG), SEQ ID No.: 35 (HAEGTFTSDVSSYLEGQAAKEFIAWLVKGRGRRDFP), SEQ ID No.: 36 (HAEGTFTSDVSSYLEGQAAKEFIAWLVKGRGRRDFPEEVA), SEQ ID No.: 37 (HAEGTFTSDVSSYLEGQAAKEFIAWLVKGRGRRDFPEEVAIAEELGRRHAC), SEQ ID No.: 38 (HAEGTFTSDVSSYLEGQAAKEFIAWLVKGRGAADFPEEVAIVEELG), SEQ ID No.: 39 (HAEGTFTSDVSSYLEGQAAKEFIAWLVKGRGRRDFAEEVAIVEELG), SEQ ID No.: 40 (HAEGTFTSDVSSYLEGQAAKEFIAWLVKGRGRRDAAAAVAIVEELG), SEQ ID No.: 41 (HAEGTFTSDVSSYLEGQAAKEFIAWLVKGRGAADAAAAVAIVAALG), SEQ ID No.: 42 (HAEGTFTSDVSSYLEGQAAKEFIAWLVKGRGRRDFPEEVAIVEELGRRHAC), i.e. GLP-1 (7-37) linked without any linker sequence via its C-terminus to specific analogs or variants of the IP2 sequence. Variants, analogs or fragments thereof having a sequence homology of at least 80 % with SEQ ID Nos: 8 and 12 or derivatives thereof are encompassed as well and preferred.

Without being bound to any theory, it is concluded by the inventors of the present invention that the instability of GLP-1(7-35, 36 or 37), if administered to any patient in need thereof, is due to its unprotected 3-dimensional structure. Proteases may cleave the GLP-1 (7-35, 36 or 37) peptide and abolish its physiological activity rapidly in vivo. By linking a peptide sequence to the C-Terminus of GLP-1(7-35, 36 or 37) its structure gains stability towards enzymatic degradation. Gain in stability appears to be enhanced, if the additional C-terminal peptide sequence (being contained in component (II) of the fusion peptide according to the invention) folds back due to the presence of a β-turn structural element formed by its primary structure and providing rigidity to component (II). However, a β-turn structure in component (II) of the inventive peptide does not appear to be a prerequisite for stabilizing the GLP-1 sequence of component (I) towards enzymatic degradation. The inventive peptide, by virtue of its C-terminal peptide extension, e.g. containing a β-turn structural element, is found to have improved resistance to DPP-IV inactivation. The C-terminal peptide is either not cleaved from the GLP-1(7-35, 36 or 37) sequence prior to acting on its receptor in target cells or it may be cleaved enzymatically to form GLP-1 (7-35, 36 or 37) in vivo. Irrespective of the exact form of the inventive peptide bound at the site of the GLP-1 receptor, an inventive peptide exerts its function as an active insulinotropic compound.

Peptide sequences, which are considered to be suitable for being contained in component (II) due to a primary structure forming a f3-tum element may readily be identified by adequate e.g. spectroscopic methods, e.g. circular dichroism, or other methods known to the skilled person.

Component (II) and component (I) may be directly linked or linked via a linker sequence. Preferably, both components are directly linked with each other. In case they are linked via a linker (or spacer), the linker is preferably a peptide linker or an organic linker. A peptide linker typically has a length of 1 to 10 amino acids, preferably 1 to 5, even more preferably 1 to 3 amino acids, in some cases the linker sequence may be even longer comprising 11 to 50 amino acids. A peptide linker may be composed of various amino acid sequences. Preferably, a peptide linker will introduce some structural flexibility between components to be linked. Structural flexibility is achieved e.g. by having a peptide linker containing various glycine or proline residues, preferably at least 30%, more preferably at least 40% and even more preferably at least 60 % proline and glycine residues within the linker sequence. Irrespective of the specific sequence the peptide linker may preferably be immunologically inactive.

In a preferred embodiment of the present invention, an inventive peptide, i.e. a fusion peptide or its analogs, fragment, variants or derivatives, contains a fourth component (component (III)) which is either linked to the C-terminus of component (II) and/or to the N-terminus of component (I). Preferably, component (fit) is located at the C-terminus of component (II). Irrespective of whether component (III) is linked to N-terminus of component (I) (by its C-terminus) or to the C-terminus of component (II) (by its N-terminus), the coupling may be direct or indirect via a linker sequence. With regard to the linker sequence it is referred to the above disclosure for a linker connecting component (I) and component (II). Generally, component (III) comprises at least four amino acid residues, preferably at least 10 additional amino acid residues, more preferably at least 20, or at least 30. In functional terms, component (III) is provided to further enhance the stability of an inventive peptide. Component (III) is expected not to interfere with the biological function of the inventive peptide which is app. comparable to the biological activity of GLP-1 (7-37).

Preferably, component (III) of the inventive peptide comprises at least 4, preferably at least 10, more preferably at least 20 additional amino acid residues of the N-terminal sequence of an isoform of GLP-2 of any mammalian organism (other naturally occurring variant of GLP-2 among mammalian), e.g. murine or human isoforms as shown in SEQ ID Nos: 4 and 5. GLP-2 occurs in pro-glucagon and is also involved in carbohydrate metabolism. As with the biologically active sequence included in component (I) (GLP-1 peptide), component (III) may also comprise analogs, variants or derivatives of naturally occurring forms of GLP-2. Alternatively, component (III) may also comprise at least 4, preferably at least 10, more preferably at least 20 additional amino acid residues of the N-terminal sequence of GLP-1 (7-37), correspondingly including all mammalian isoforms or - as disclosed herein - all functional variants, analogs or derivatives thereof. Generally speaking, component (III) may contain any form of a GLP-1 peptide or a modified GLP-1 peptide, which is disclosed herein as suitable for component (I) of the inventive peptide. In a further alternative, component (III) may also contain chimeric forms of GLP-1 (7-37) and GLP-2. A chimeric form may be produced by coupling GLP-1(7-37) and GLP-2 (or fragments, analogs, variants or derivatives of both) with each other and by subsequently introducing this chimeric form as component (III) into the inventive peptide. Preferably, the chimeric form is composed of a partial sequence of GLP-1(7-37) and a partial sequence of GLP-2 linked together. E.g. the chimeric form may include the N-terminal 5 to 30 amino acids of GLP-1 and the C-terminal 5 to 30 amino acids of GLP-2 or vice versa, e.g amino acids 7 or 8 to 22, 23, 24, 25, 26, 27, or 28 of GLP-1(7-37) and amino acid sequence from position 15, 16, 17, 18, 19, 20, 21, 22, 23 or 24 to e.g. the C-terminus of GLP-2.

If modifications of naturally occurring forms of GLP-2 or GLP-1(7-37), respectively, are used as component (III), component (III) preferably contains the sequence of SEQ ID Nos.: 4 or 5 or SEQ ID No.: 1, respectively, or a sequence having at least 80% sequence homology with SEQ ID Nos.: 4 or 5 or SEQ ID No.: 1. Derivatives of these preferred sequences, e.g. due to side chain modifications or peptide backbone modifications etc. (as disclosed herein pertaining to "derivatives"), are also encompassed as component (III) by the present invention.

In another embodiment, component (III) may contain a plurality of sequences as described above. E.g. component (III) may contain at least two, preferably 2, 3, or 4 copies of GLP-1(7-37) and/or GLP-2 or at least two copies of sequences having at least 80% sequence homology with SEQ ID Nos: 1, 4 or 5. Also component (III) may contain more than copy of a chimeric version of GLP-1 (7-37) or GLP-2, as disclosed above, e.g. eventually forming a combination of chimeric version(s) together with GLP-1 (7-37) and/or GLP-2 or its modifications with at least 80 % sequence homology. Within the scope of the present invention are also two or more, preferably two component (III), which may e.g. be (1) linked by its N-terminus to the C-terminus of component (II) and (2) linked by its C-terminus to the N-terminus of component (I) via a linker or directly. If two components (III) are provided, these may be identical or different.

Accordingly, inventive conjugate molecules containing components (I), (II), the synthetic polymer and/or the protein and (III) are particularly preferred. Four specific embodiments containing all of these components are selected from a group consisting of: SEQ ID No. 6 (N-GLP-1(7-37)-IP2(murine)-RR-GLP-1(7-37)-C, also designated murine CM1 herein), SEQ ID No. 7 (N-GLP-1(7-37)-IP2(murineyRR-GLP2-C, also designated murine CM2 herein), SEQ ID No. 10 (N-GLP-1(7-37)-IP2(human)-RR-GLP-1(7-37)-C, also designated human CM1), and SEQ ID No. 11 (N-GLP-1(7-37)-IP2(human)-RR-GLP-2-C), also designated human CM2 herein) or a sequence having at least 80% sequence homology with SEQ ID Nos.: 6, 7, 10, or 11 or a derivative thereof. All sequences 6, 7, 10 and 11 contain an RR-Linker (two arginine residues) at the C-terminus of IP2 (component (II)), which may alternatively also be discarded. Component (I) in each of the embodiments according to SEQ ID Nos:6, 7, 10 or 11 is GLP-1(7-37), whereas component (III) (in each of these embodiments linked to the C-terminus of component (II)) is either GLP-1(7-37) or GLP-2.

The inventive peptides may occur in various modified forms. These modified forms are disclosed in the following and described in more detail.

The term "salts" herein refers to both salts of carboxyl groups and to acid addition salts of amino groups of the fusion peptides described above or analogs, fragments, derivatives or variants thereof. Salts of a carboxyl group may be formed by means known in the art and include inorganic salts, for example, sodium, calcium, ammonium, ferric or zinc salts, and the like, and salts with organic bases as those formed, for example, with amines, such ethanolamine, arginine or lysine, piperidine, procaine and the like. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of the inventive peptides relevant to the present invention, i.e. the ability to reduce the rate of entry of nutrients into the circulation. As disclosed below, the salt peptide forms may be contained in a pharmaceutical formulation.

A "fragment" of a fusion peptide according to the present invention refers to any subset of the molecules, that is, a shorter peptide which retains the desired biological activity. Fragments may readily be prepared by removing amino acids from either end of the molecule and testing the resultant for its properties as a incretin. Proteases for removing one amino acid at a time from either the N-terminal and/or the C-terminal of a polypeptide are known, and so determining fragments which retain the desired biological activity involves only routine experimentation. Conclusively, fragments may be due to deletions of amino acids at the peptide termini and/or of amino acids positioned within the peptide sequence.

Additionally, the inventive peptide which has anti-diabetes type 2 activity, be it a fusion peptide itself, an analog or variant, salt, functional derivative and/or fragment thereof, can also contain additional amino acid residues flanking the inventive peptide. As long as the resultant molecule retains its resistancy or stability towards proteases and its ability to act as incretin, one can determine whether any such flanking residues affect the basic and novel characteristics of the core peptide, e.g. by its effects on pancreas cells, by routine experimentation. The term "consisting essentially of, when referring to a specified sequence, means that additional flanking residues can be present which do not affect the basic and novel characteristic of the specified inventive peptide. This term does not comprehend substitutions, deletions or additions within the specified sequence.

A "variant" according to the present invention refers to a molecule which is substantially similar to either the entire inventive peptide defined above or a fragment thereof. Variant peptides may be conveniently prepared by direct chemical synthesis of the variant peptide, using methods well known in the art. Of course, such variant of an inventive peptide would have similar anti-diabetic, e.g. insulin stimulating activity as the corresponding naturally-occurring GLP-1 peptide.

Alternatively, amino acid sequence variants of the peptides defined above can be prepared by mutations in the DNAs which encode the synthesized derivatives. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure.

An "analog" of the peptides defined above, according to the present invention, refers to a non-natural molecule which is substantially similar to either the entire molecule or to an active fragment thereof. Such analog would exhibit the same activity as the corresponding naturally-occurring GLP-1 peptide.

The types of substitutions which may be made in the inventive peptide, according to the present invention, may be based on analysis of the frequencies of amino acid changes between a homologous protein/peptide of different species. Based upon such analysis, conservative substitutions may be defined herein as exchanges within one of the following five groups:
I. Small, aliphatic, non-polar or slightly polar residues: Ala, Ser, Thr, Pro, Gly; II. Polar, negatively-charged residues and their amides: Asp, Asn, Glu, Gin; III. Polar, positively-charged residues: His, Arg, Lys; IV. Large, aliphatic non-polar residues: Met, Leu, Ile, Val, Cys ; V. Large aromatic residues: Phe, Try, Trp.

Within the foregoing groups, the following substitutions are considered to be "highly conservative" : Asp/Glu; His/Arg/Lys; Phe/Tyr/Trp; Met/Leu/Ile/Val. Semi-conservative substitutions are defined to be exchanges between two of groups (I) - (IV) above which are limited to supergroup (A), comprising (I), (II), and (III) above, or to supergroup (B), comprising (IV) and (V) above. Substitutions are not limited to the genetically encoded or even the naturally-occurring amino acids.

In general, analogs or variants of the inventive peptide may also contain amino acid substitutions, made e.g. with the intention of improving solubility (replacement of hydrophobic amino acids with hydrophilic amino acids). In one embodiment of variants/analogs of the GLP-1 peptide of the inventive peptide (occurring in component (I) and/or (III) of the inventive peptide) the (modified) GLP-1 peptide is characterized by one or more substitution(s) at positions 7, 8, 11, 12, 16, 22, 23, 24, 25, 27, 30, 33, 34, 35, 36, or 37 of the GLP-1 peptide. As an example for the following nomenclature [Arg34-GLP-1 (7-37)] designates a GLP-1 analogue wherein the naturally occurring lysine at position 34 has been substituted with arginine.

Specifically, component (I) and/or (III) of an inventive peptide may comprise variants and analogs of GLP-1(7-35, 36 or 37) including, for example, Gln9-GLP-1 (7-37), D-Gln9-GLP-1(7-37), acetyl-Lys9-GLP-1 (7-37), Thr16-Lys18-GLP-1 (7-37), and Lys18-GLP-1 (7-37), Arg34-GLP-1 (7-37), Lys38-Arg26-GLP-1 (7-38)-OH, Lys36-Arg26-GLP-1 (7-36), Arg26,34-Lys38-GLP-1 (7-38), Arg26,34-Lys38-GLP-1(7-38), Arg26,34-Lys38-GLP-1 (7-38), Arg26,34-Lys38-GLP-1 (7-38), Arg26,34-Lys38-GLP-1 (7-38), Arg26-Lys38-GLP-1(7-38), Arg26-Lys38-GLP-1(7-38), Arg26-Lys38-GLP-1 (7-38), Arg34-Lys38-GLP-1 (7-38), Ala37-Lys38-GLP-1 (7-38), and Lys37-GLP-1 (7-37).

In another embodiment of the invention the inventive peptide contains as component (I) or (III) a modified GLP-1 peptide comprising the amino acid sequence of the following formula II (SEQ ID No.: 44):
Xaa7-XaaB-Glu-Gly-Thr-Phe-Thr-Ser-Asp-Xaa 16-Ser-Xaa 18-Xaa 19-Xaa20-Glu-Xaa22-Xaa23-Ala-Xaa25-Xaa26-Xaa27-Phe-Ile-Xaa3o-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is is Ser, Lys or Arg; Xaa19 is Tyr or Gln ; Xaa20 is Leu or Met; Xaa22 is Gly, Glu or Aib; Xaa23 is Gin, Glu, Lys or Arg ; Xaa25 is Ala or Val ; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent.

In another embodiment of the invention component (I) and/or (III) of the inventive peptide contains a modified GLP-1 peptide comprising the amino acid sequence of the following formula III (SEQ ID No.: 45):
Xaa7-XaaB-Glu-Gly-Thr-Phe-Thr-Ser-Asp-Val-Ser-XaaB-Tyr-Leu-Glu-Xaa22-Xaa23-Ala-Ala-Xaa26-Glu-Phe-Ile-Xaa30-Trp-Leu-Val-Xaa34-Xaa35-Xaa36-Xaa37,
wherein Xaa7 is L-histidine, D-histidine, desamino-histidine, 2-amino-histidine, -hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gin, Glu, Lys or Arg ; Xaa26 is Lys, Glu or Arg;Xaa30 isAla, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent.

In a particular preferred embodiment of the invention component (I) and/or (III) of the inventive peptide contain a (modified) GLP-1 peptide, which is selected from GLP-1 (7-35), GLP-1 (7-36), GLP-1 (7-36)-amide, GLP-1 (7-37) or an variant, analogue or derivative thereof. Also preferred are inventive peptides comprising in their components (I) and/or (III) a modified GLP-1 peptide having a Aib residue in position 8 or an amino acid residue in position 7 of said GLP-1 peptide, which is selected from the group consisting of D-histidine, desamino-histidine, 2-amino-histidine, hydroxy-histidine, homohistidine, N-acetyl-histidine, a-fluoromethyl-histidine, a-methyl-histidine, 3-pyridylalanine, 2-pyridylalanine and 4-pyridylalanine.

Examples of production of amino acid substitutions in proteins which can be used for obtaining analogs for use in the present invention include any known method steps, such as presented in U. S. patents RE 33,653; 4,959,314; 4,588,585 and 4,737,462, to Mark et al; 5,116,943 to Koths et al; 4,965,195 to Namen et al; and 5,017,691 to Lee, et al, and lysine substituted proteins presented in US patent 4,904,584 (Shaw et al).

Preferably, the variant or analog, as defined above and contained in component (I), (II) and/or (III), will have a core sequence, which is the same as that of the "native" sequence, e.g. GLP-1(7-37) or GLP-2 or biologically active fragment thereof or any IP2 isoform, which has an amino acid sequence having at least 70% identity to the native amino acid sequence and retains the biological activity thereof. More preferably, such a sequence has at least 80% identity, at least 90% identity, or most preferably at least 95% identity to the native sequence. Where a particular peptide is said to have a specific percent identity to a reference polypeptide of a defined length; the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Of course, other polypeptides will meet the same criteria. The term "sequence identity" as used herein means that the sequences are compared as follows. The sequences are aligned using Version 9 of the Genetic Computing Group's GAP (global alignment program), using the default (BLOSUM62) matrix (values-4 to +11) with a gap open penalty of-12 (for the first null of a gap) and a gap extension penalty of-4 (per each additional consecutive null in the gap). After alignment, percentage identity is calculated by expressing the number of matches as a percentage of the number of amino acids in the claimed sequence.

Derivatives of a fusion peptide or an analog, fragment or variant thereof are also encompassed by the present invention. The term "derivatives" of an inventive peptide is intended to include only those modified inventive peptides that do not change one amino acid to another of the twenty commonly-occurring natural amino acids. Correspondingly, a genetically encoded amino acid may be modified by reacting it with an organic derivatizing agent that is capable of reacting with selected side chains of residues or amino or carboxy groups of terminal residues (preferably by covalent modification) or by introducing non-natural amino acids (manufactured by chemical synthesis, i.e. D-isomers of the amino acids encoded by the genetic code, Aib (a-aminoisobutyric acid), Abu (a-aminobutyric acid), Tie (tert-butylglycine), p-aianine, 3-aminomethyl benzoic acid, anthranilic acid) or natural amino acids which are not encoded by the genetic code, e.g. hydroxyproline, γ-carboxyglutamate, ornithine, phosphoserine, D-alanine and D-glutamine or by a modification of the peptide backbone by alternative peptide backbone arrangements.

In the following preferred modifications of amino acids of the inventive peptide (which - as defined above - also comprises variants, analogues or fragments of the fusion peptide) are disclosed, which may occur in an inventive peptide at any site (any amino acid), e.g. positioned in component (1), (II) and/or (III).

Cysteinyl residues, if present in any form of an inventive peptide, e.g. an analogue of an inventive peptide, most commonly are reacted with alpha-haloacetates (and corresponding amines), such as chloroacetic acid or chloroacetamide, to give carboxylmethyl orcarboxyamidomethyl derivatives. Cysteinyl residues also are derivatized by reaction with bromotrifluoroacetone, alpha-bromo-beta-(5-imidazoyl)propionic acid, chloroacetyl phosphate, N-alkylmaleimides, 3-nitro-2-pyridyl disulfide, methyl-2-pyridyl disulfide, p-chloromercuribenzoate, 2-chloromercuri-4-nitrophenol, orchloro-7-nitrobenzo-2-oxa-1,3-diazole.

Histidyl residues in an inventive peptide may be derivatized by reaction with diethylprocarbonate at pH 5.5-7.0 because this agent is relatively specific for the histidyl side chain. Parabromophenacyl bromide is also useful; the reaction is preferably performed in 0.1 M sodium cacodylate at pH 6.0. In particular, the N-terminal histidine residue (His7) of GLP-1(7-37) as contained in component (I) and/or (III) of the inventive peptide is very important to the insulinotropic activity of GLP-1 peptides as shown by Suzuki et. al. (Diabetes Res.; Clinical Practice 5 (Supp. 1): S30 (1988)). Correspondingly, the inventive peptide may be modified at His7 of its GLP-1 as part of component (I) and/or (III) by alkyl or acyl (C1-C6) groups, or replacement of His with functionally-equivalent C5-C6 ring structures. A preferred modification is the introduction of a hydrophobic moiety at the amino terminus of His7 or its histidyl side chain.

Lysinyl and amino terminal residues of an inventive peptide may e.g. be reacted with succinic or other carboxylic acid anhydrides. Derivatization with these agents has the effect of reversing the charge of the lysinyl residues. By acyl (C12-C18) modifications of the epsilon-amino group of lysine residue(s) in the inventive peptide, their half-life in circulation is increased. Arginyl residues may e.g. be modified by reaction with one or several conventional reagents, among them phenylglyoxal; and ninhydrin. Derivatization of arginine residues requires that the reaction be performed in alkaline conditions because of the high pKa of the guanidine functional group. Furthermore, these reagents may react with the groups of lysine, as well as the arginine epsilon-amino group.

The specific modification of tyrosyl residues per se has been studied extensively. Most commonly, N-acetylimidazole and tetranitromethane may be used to form O-acetyl tyrosyl species and e-nitro derivatives, respectively.

Carboxyl side groups (aspartyl or glutamyl) may be selectively modified by reaction with carbodiimides (R'N-C-N-R') such as 1-cyclohexyl-3-[2-morpholinyl-(4-ethyl)] carbodiimide or 1-ethyl-3-(4-azonia-4,4-dimethylpentyl)carbodiimide.

Furthermore, aspartyl and glutamyl residues may be converted to asparaginyl and glutaminyl residues by reaction with ammonium ions.

Glutaminyl and asparaginyl residues may be deamidated to the corresponding glutamyl and aspartyl residues. Alternatively, these residues may be deamidated under mildly acidic conditions. Either form of these residues falls within the scope of this invention. Deamidated inventive peptides may undergo an altered susceptibility to proteolysis with protease or peptidase enzymes, suggesting that deamidation may have physiological significance in directing proteolytic cleavage of an inventive peptide. It is noted that biosynthetic inventive peptides may degrade under certain storage conditions, resulting in deamidation at one more positions in the inventive peptide. Methionine residues in the inventive peptides may be susceptible to oxidation, primarily to the sulfoxide. As the other derivatives mentioned above, both desamide inventive peptides and/or sulfoxide inventive peptides may be used to exhibit full biological activity.

Other suitable reagents for derivatizing alpha-amino acid-containing residues include imidoesters such as methyl picolinimidate; pyridoxal phosphate; pyridoxal; chloroborohydride; trinitrobenzenesulfonic acid; O-methyliosurea; 2,4-pentanedione; and transaminase-catalyzed reaction with glyoxylate.

The terminal amino acid residues of an inventive peptide with their carboxy (C-terminus) and their amine (N-terminus) groups (as well as carboxy or amide amino acid side chain groups, see above) may be present in their protected (e.g. the C terminus by an amide group) and/or unprotected form, using appropriate amino or carboxyl protecting groups. Also, acid-addition salts of the inventive peptide may be provided. Common acid addition salts are hydrohalic acid salts, i.e., HBr, HI, or more preferably, HCl.

Other modifications resulting in derivatives of inventive peptides are based on carbohydrates and/or lipids which may be covalently coupled to the inventive peptide. It is preferred to couple lipids and/or carbohydrates to serine, threonine, asparagine, glutamine or tyrosine or glutamate or aspartate via their reactive side chain moieties. Alternatively, carbohydrates and/or lipids may also be linked to the terminal moieties of the inventive peptide. Furthermore, an inventive peptide may be coupled to a functionally different peptide or protein moiety, which may also stabilize the inventive peptide and/or may serve to improve the transport properties of an inventive peptide in body fluids, in particular blood. Suitable peptides or proteins may e.g. be selected from albumin, transferrin etc., which are directly coupled (as component IV) to the inventive peptide or via a peptide or organic linker sequence (see also above). Preferably, these peptides or protein are linked to one of the termini of the inventive peptide.

In order to circumvent the problem of degradation of the inventive peptide another embodiment of the present invention provides a retro-inverso isomer of the inventive peptide composed of D amino acids or at least partially composed of D amino acids. The term "retro-inverso isomer" refers to an isomer of a linear peptide in which the direction of the sequence is reversed and the chirality of each amino acid residue is inverted (see, e.g., Jameson et al., Nature, 368, 744-746 (1994); Brady et al., Nature, 368, 692-693 (1994)).

With respect to the parent peptide, the retro-inverso peptide is assembled in reverse order of amino acids, typically with F-moc amino acid derivatives. Typically, the crude peptides may be purified by reversed phase HPLC.

Other modifications, which may be introduced into the inventive peptides relate to modifications of the peptide backbone. Preferably, the modified inventive peptides are scaffold mimetics. Their backbone is different from the natural occurring backbone, while their side-chain structures are identical with the inventive peptides or their fragments, variants, derivatives or analogs. In general, scaffold mimetics exhibit a modification of one or more of the backbone chain members (NH, CH, CO), either as substitution (preferably) or as an insertion. Substituents are e.g. (I) -O-, -S-, or -CH₂- instead of -NH-; (II) -N-, C-Alkyl-, or -BH- instead of -CHR- and (III) -CS-, -CH₂-, -SOₙ-, -P=O(OH)-, or -B(OH)- instead of - CO-. A peptide mimetic of an inventive peptide may be a combination of each of these modifications. In particular, modifications of each the groups I, II and III may be combined. In a peptide mimetic each backbone chain member may be modified or, alternatively, only a certain number of chain members may be exchanged for a non-naturally occurring moiety. Preferably, all backbone chain members of an inventive peptide of either -NH-, -CHR- or CO are exchanged for another non-naturally occurring group. In case the amide bond (-NH-CO-) of the inventive peptide backbone is substituted (in the entire molecule or at least in one single position), preferable substitution moieties are bioisosteric, e.g. retro-inverse amide bonds (-CO-NH-), hydroxyl ethylene (-CH(OH)-CH2-), alkene (CH2=CH-), carba (CH2-CH2-) and/or -P=O(OH)-CH2-). Alternatively, backbone chain elongation by insertions may occur in a scaffold mimetic of the inventive peptide, e.g. by moieties flanking the C-alpha atom. On either side of the C-alpha atom e.g. - O-, -S-, -CH-, -NH- may be inserted.

Particularly preferred are oligocarbamate peptide backbone structure of the inventive peptides. The amide bond is replaced by a carbamate moiety. The monomeric N-protected amino alkyl carbonates are accessible via the corresponding amino acids or amino alcohols. They are converted into active esters, e.g. p-nitro phenyl ester by using the F-moc moiety or a photo sensitive nitroatryloxycarbonyl group by solid phase synthesis.

Inventive peptides are protected against proteolytic cleavage as outlined above. They are in particular protected against dipeptidyl aminopeptidase-4 (DPP-IV). The term "DPP-IV protected" as used herein refers to a peptide according to claim 1. Inventive peptides as well as their derivatives, analogs, fragments and variants render GLP-1(7-35, 36 or 37) as part of component (I) and/or (III) of the inventive peptide resistant to the plasma peptidase (DPP-IV).

Resistance of a peptide to degradation by dipeptidyl aminopeptidase IV is determined e.g. by the following degradation assay: Aliquots of the peptides are incubated at 37 C with an aliquot of purified dipeptidyl aminopeptidase IV for 4-22 hours in an appropriate buffer at pH 7-8 (buffer not being albumin). Enzymatic reactions are terminated by the addition of trifluoroacetic acid, and the peptide degradation products are separated and quantified using HPLC or LC-MS analysis. One method for performing this analysis is: The mixtures are applied onto a Zorbax300SB-C18 (30 nm pores, 5 µm particles) 150 x 2.1 mm column and eluted at a flow rate of 0.5 ml/min with a linear gradient of acetonitrile in 0. 1 % trifluoroacetic acid (0%-100% acetonitrile over 30 min). Peptides and their degradation products may be monitored by their absorbance at 214 nm (peptide bonds) or 280 nm (aromatic amino acids), and are quantified by integration of their peak areas. The degradation pattern can be determined by using LC-MS where MS spectra of the separated peak can be determined. Percentage intact/degraded compound at a given time is used for estimation of the peptides DPP-IV stability.

An inventive peptide is defined as DPP-IV stabilised when it is 10 times more stable than the GLP-1 (7-37) based on percentage intact compound at a given time. Thus, a DPP-IV stabilised inventive peptide is preferably at least 10, more preferably at least 20 times more stable than GLP-1 (7-37) as such. Stability may be assessed by any method known to the skilled person, e.g. by adding DPP-IV to a solution of the peptide to be tested and by determining the degradation of the peptide, e.g. over time, by e.g. a spectroscopic method, Western-Blot analysis, antibody screening etc. In parallel, an inventive peptide is defined as a compound, which exerts the effect of GLP-1 (7-37) by e.g. binding to its native receptor (GLP-1 receptor). Preferably, an inventive peptide has a binding affinity to the GLP-1 receptor, which corresponds to at least 10%, preferably at least 50% of the binding affinity of the naturally occurring GLP-1 peptide. The binding affinity may be determined by any suitable method, e.g. surface plasmon resonance etc. Moreover, it is preferred, if the inventive peptide evokes formation of intracellular cAMP by its binding to its extracellular receptor, which transmits the signal into the cell.

The peptides of the invention may be produced synthetically, using solid phase peptide synthesis techniques, similar to the manner of production of GLP-1 (7-36) amide and GLP-1 (7-37) in the art and can be purified afterwards on a laboratory scale e.g. by a single purification step on a reversed-phase HPLC column or suitable chromatography methods.

However, it is preferably formed in engineered cells, either in microbial cells or in animal cell lines to produce the inventive peptide. The inventive peptide may be isolated from the cells from which it is expressed, for instance using conventional separation techniques. Thus cells may be grown under appropriate conditions, for instance including support and nutrients, in vitro, and secreted protein, i.e. the inventive peptide, is recovered from the extracellular medium. The sequences engineered into cells thus preferably include leader sequences and signal peptide sequences directing secretion of the inventive peptide. The cells preferably express protease capable of cleaving the leader and signal sequences, either endogenously or by engineered gene sequences. In an alternative, the engineered gene sequences encoding an inventive peptide do not include such leader and signal peptide sequences, whereby the intracellularly expressed inventive peptide will not be secreted, and is recovered from cells by processes involving cell lysis. In such methods the coding sequences may include purification tags allowing efficient extraction of the product peptide from the medium, which tags may be cleaved to release isolated inventive peptide.

According to a further aspect of the invention there is provided a method of treatment of an animal, preferably a human being, by administration of an inventive conjugate complex comprising components (I) and (II) and the synthetic polymer and/or the protein and eventually component (III). It is also provided corresponding use of such inventive conjugate molecule in the manufacture of a product for the treatment or prevention of a disease or condition associated with glucose metabolism. Non-limiting examples of glucose disorder include: diabetes mellitus type I or type II (NIDDM), or insulin resistance, weight disorders and diseases or conditions associated thereto, wherein such weight disorders or associated conditions include obesity, overweight-associated conditions, satiety deregulation, reduced plasma insulin levels, increased blood glucose levels, or reduced pancreatic beta cell mass. Preferably, use of inventive conjugate molecules for the manufacture of a medicament for the treatment of type 2 diabetes (NIDDM) is disclosed herewith. As a consequence, the present invention relates to a use of the inventive conjugatew molecule e.g. for lowering weight of a subject, for reducing satiety of a subject, for post-prandially increasing plasma insulin levels in a subject, for reducing fasting blood glucose level in a subject, for increasing pancreatic beta cell mass in a subject or for treating diabetes type I or II in a subject.

Patients with other diseases or disorders may be treated by inventive conjugate molecules, i.e. fusion peptides or its analogs, fragments, variants or derivatives coupled to at least one polymer constituent, as well. Inventive conjugate molecules may be used for the preparation of a medicament for the treatment of neurodegenerative disorders and diseases or conditions associated thereto and for the treatment of disorders and diseases or conditions associated to apoptosis. The use of the inventive conjugate molecule for treating these disorders results from the following: GLP-1 receptors, which are coupled to the cyclic AMP second messenger pathway, are expressed throughout the brains of rodents and humans. The chemoarchitecture of receptor distribution in the brain does not only correlate with a central role for GLP-1 in the regulation of food intake and response to aversive stress. It was also shown that GLP-1 binding at its GLP-1 receptor exerts neurotrophic properties, and offer protection against glutamate-induced apoptosis and oxidative injury in cultured neuronal cells. Furthermore, GLP-1 was shown to modify processing of the amyloid β-protein precursor in cell culture and dose-dependently reduces amyloid β-peptide levels in the brain *in vivo.* GLP-1 is therefore also known as regulator of the central nervous system. Inventive conjugate molecule containing peptides mimicking the biological activity of physiologically active GLP-1 have therapeutic relevance to the treatment of e.g. Alzheimer's disease (AD) and other central and peripheral neurodegenerative conditions (e.g. amyotrophic lateral sclerosis (ALS), Alexander disease, Alper's disease, Ataxia telangiectasia, Canavan disease, Cockayne syndrome, Creutzfeldt-Jakob disease, Multiple Sclerosis, Sandhoff disease, Pick's disease, Spinocerebellar Ataxia, Schilder's disease and Parkinson's disease).

Moreover, it was shown that physiologically active GLP-1 exerts anti-apoptotic action on various cells, e.g. GLP-1 is beneficial to the preservation of mass and function of freshly isolated human islets or other cell types. Insofar, the biologically active fusion peptide of a conjugate molecule may be used to treat disorders, which are caused by cell or tissue apoptosis.

The use of an inventive conjugate molecule may be for the manufacture of a composition which is administered exogenously, and comprises the isolated inventive conjugate molecule. The resulting composition may be used as well for the treatment of the above disorders.

Preparation of formulations which contain inventive conjugate molecules having the fusion peptide sequences as active ingredients is generally well understood in the art, as exemplified by US Patents 4,608,251; 4,601,903 ; 4,599,231; 4,599,230; 4,596,792; and 4,578,770, all incorporated herein by reference. Typically, such formulations are prepared as injectables either as liquid solutions or suspensions, preferably containing water (aqueous formulation) or may be emulsified. The term "aqueous formulation" is defined as a formulation comprising at least 50 % w/w water. Likewise, the term "aqueous solution" is defined as a solution comprising at least 50% w/w water, and the term "aqueous suspension" is defined as a suspension comprising at least 50 % w/w water.

For intravenous, cutaneous or subcutaneous injection, or injection at the site of affliction, the active ingredient will be in the form of a parenterally acceptable aqueous solution which is pyrogen-free and has suitable pH, isotonicity and stability. Liquid pharmaceutical compositions generally include a liquid vehicle such as water. Preferably, the liquid vehicle will include a physiological saline solution, dextrose ethanol or other saccharide solution or glycols such as ethylene glycol, propylene glycol or polyethylene glycol or combinations thereof may be included. Further examples are other isotonic vehicles such as Ringer's Injection or Lactated Ringer's Injection.

If the invention relates to a pharmaceutical formulation comprising an aqueous solution of a compound according to the present invention, and a buffer, wherein said compound is present in a concentration from 0.1 mg/ml or above, and wherein said formulation has a pH from about 2.0 to about 10.0, preferably from about 7.0 to about 8. 5. Preferably, the pH of the formulation is at least 1 pH unit from the isoelectric point of the compound according to the present invention, even more preferable the pH of the formulation is at least 2 pH unit from the isoelectric point of the compound according to the present invention.

Solid forms suitable for solution in, or suspension in, liquid prior to injection may also be prepared. The pharmaceutical formulation may be a freeze-dried formulation, whereto the physician or the patient adds solvents and/or diluents prior to use. In other words, the formulation once prepared, is not immediately administered to a subject. Rather, following preparation, it is packaged for storage in a frozen state, or in a dried form for later reconstitution into a liquid form or other form suitable for administration to a subject. In another embodiment the pharmaceutical formulation is a dried formulation (e.g. freeze-dried or spray-dried) ready for use without any prior dissolution. By "dried form" is intended the liquid pharmaceutical composition or formulation is dried either by freeze drying (i.e. lyophilization ; see, for example, Williams and Polli (1984) J. Parenteral Sci. Technol. 38: 48-59), spray drying (see Masters (1991) in Spray-Drying Handbook (5th ed; Longman Scientific and Technical, Essez, U. K. ), pp. 491-676; Broadhead et al. (1992) Drug Devel. Ind. Pharm. 18: 1169-1206; and Mumenthaler et al. (1994) Pharm. Res. 11: 12-20), or air drying (Carpenter and Crowe (1988) Cryobiology 25: 459-470; and Roser (1991) Biopharm. 4: 47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

It is possible that other ingredients may be present in the peptide pharmaceutical formulation of the present invention. Such additional ingredients may include wetting agents, emulsifiers, antioxidants, bulking agents, pH buffering agents (e.g. phosphate or citrate or maleate buffers), preservatives, surfactants, stabilizers, tonicity modifiers, cheating agents, metal ions, oleaginous vehicles, proteins (e.g. human serum albumin, gelatin or proteins) and/or a zwitterion (e.g. an amino acid such as betaine, taurine, arginine, glycine, lysine and histidine).

With regard to stabilizers for inventive formulations these may preferably be selected from the group of high molecular weight polymers or low molecular compounds. In a further embodiment of the invention the stabilizer is selected from polyethylene glycol (e.g. PEG 3350), polyvinylalcohol (PVA), polyvinylpyrrolidone, carboxy-hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention. The pharmaceutical compositions may also comprise additional stabilizing agents, which further enhance stability of a therapeutical active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

With regard to surfactants for inventive formulations these may preferably be selected from a detergent, ethoxylated castor oil, polyglycolyzed glycerides, acetylated monoglycerides, sorbitan fatty acid esters, polyoxypropylene-polyoxyethylene block polymers (e.g. poloxamers such as Pluronie F68, poloxamer 188 and 407, TritonX-100), polyoxyethylene sorbitan fatty acid esters, starshaped PEO, polyoxyethylene and polyethylene derivatives such as alkylated and alkoxylated derivatives (tweens, e.g. Tween-20, Tween-40, Tween-80 and Brij-35), polyoxyethylenehydroxystearate, monoglycerides or ethoxylated derivatives thereof, diglycerides or polyoxyethylene derivatives thereof, alcohols, glycerol, lecitins and phospholipids (e.g. phosphatidyl serine, phosphatidyl choline, phosphatidyl ethanolamine, phosphatidyl inositol, diphosphatidyl glycerol and sphingomyelin), derivates of phospholipids (e.g. dipalmitoyl phosphatidic acid) and lysophospholipids (e.g. palmitoyllysophosphatidyl-L-serine and 1-acyl-sn-glycero-3-phosphate esters of ethanolamine, choline, serine or threonine) and alkyl, alkoxyl (alkyl ester), alkoxy (alkyl ether)-derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (e.g. cephalins), glyceroglycolipids (e.g. galactopyransoide), sphingoglycolipids (e.g. ceramides, gangliosides), dodecylphosphocholine, hen egg lysolecithin, fusidic acid derivatives (e.g. sodium tauro-dihydrofusidate etc.), long-chain fatty acids and salts thereof C6-C12 (e.g. oleic acid and caprylic acid), acylcarnitines and derivatives, N'X-acylated derivatives of lysine, arginine or histidine, or side-chain acylated derivatives of lysine or arginine N-acylated derivatives of dipeptides comprising any combination of lysine, arginine or histidine and a neutral or acidic amino acid, N-acylated derivative of a tripeptide comprising any combination of a neutral amino acid and two charged amino acids, DSS (docusate sodium, CAS registry no [577-11- 7]), docusate calcium, CAS registry no [128-49-4]), docusate potassium, CAS registry no [7491-09-0]), SDS (sodium dodecyl sulfate or sodium lauryl sulfate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N, N-dimethyl-3-ammonio-1-propanesulfonate, anionic (alkyl-aryl-sulphonates) monovalent surfactants, zwitterionic surfactants (e.g. N-alkyl-N, N-dimethylammonio-1-propanesulfonates, 3-cholamido-1-propyldimethylammonio-1-propane-sulfonate, cationic surfactants (quaternary ammonium bases) (e.g. cetyl-trimethylammonium bromide, cetylpyridinium chloride), non-ionic surfactants (e.g. Dodecyl-D-glucopyranoside), poloxamines (e.g. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention. The use of a surfactant in pharmaceutical compositions is well-known to the skilled person. For convenience reference is made to Remington: The Science and Practice of Pharmacy, 19th edition, 1995.

With regard to pharmaceutically acceptable preservative these may preferably be selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, ethanol, chlorobutanol, and thiomerosal, bronopol, benzoic acid, imidurea, chlorohexidine, sodium dehydroacetate,chlorocresol, ethyl p-hydroxybenzoate, benzethonium chloride, chlorphenesine (3p-chlorphenoxypropane-1,2-diol) or mixtures thereof. Alkylparabens may be introduced as well. Alkylparaben refers to a Cl to C4 alkylparaben, or mixtures thereof. Examples of alkylparabens include methylparaben, ethylparaben, propylparaben, or butylparaben. Preservative refers to a compound that is added to the formulation to act as an antimicrobial agent. The concentrations must be sufficient to maintain preservative effectiveness by retarding microbial growth. Preferably, the preservative is a phenol derivative. More preferably the preservative is a cresol. Even more preferably the preservative is metacresol. A preferred concentration of a preservative in the final mixture is about 1.0 mg/mL to about 20.0 mg/mL. More preferred ranges of concentration of preservative in the final mixture are about 2.0 mg/mL to about 8.0 mg/mL, about 2.5 mg/mL to about 4.5 mg/mL and about 2.0 mg/mL to about 4.0 mg/mL. A most preferred concentration of preservative in the final mixture is about 3.0 mg/mL.

With regard to isotonic agents these may preferably be selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. *Isotonicity agents refer to compounds that are tolerated physiologically and impart a suitable tonicity to the for mulation to prevent the net flow of water across cell membranes.* Any sugar such as mono-, di-, or polysaccharides, or water-solubleglucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galacititol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. One or more isotonicity agents may be added to adjust the ionic strength or tonicity. The preferred isotonicity agent is NaCl. The concentration of the NaCl is preferably between about 10 mM and 200 mM, more preferred is between about 50 mM and 150 mM, and most preferred is about 100 mM.

With regard to cheating agents these may preferably be selected from salts of ethylenediaminetetraacetic acid (EDTA), citric acid, and aspartic acid, and mixtures thereof.

With regard to buffers these are preferably selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethane, hepes, bicine, tricine, malic acid, succinate, maleic acid, fumaric acid, tartaric acid, aspartic acid or mixtures thereof. Each one of these specific buffers constitutes an alternative embodiment of the invention.

The administration composition of the present invention may also include one or more enzyme inhibitors. Such enzyme inhibitors include, but are not limited to, compounds such as actinonin or epiactinonin and derivatives thereof. Other enzyme inhibitors include, but are not limited to, aprotinin (Trasylol) and Bowman-Birk inhibitor.

The administration composition may optionally contain additives such as phosphate buffer salts, citric acid, glycols, or other dispersing agents. Stabilizing additives may be incorporated into the solution, preferably at a concentration ranging between about 0.1 and 20% (w/v).

The use of all of the afore-mentioned additives in pharmaceutical compositions containing the inventive therapeutic conjugate molecule is well-known to the skilled person, in particular with regard to concentration ranges of the same. For convenience reference is made to Remington: The Science and Practice of Pharmacy, 19th edition, 1995.

The formulations containing the inventive conjugate molecule are conventionally administered parenterally, by injection, for example, either subcutaneously, intradermally, subdermally or intramuscularly. A composition for parenteral administration of the inventive conjugate molecule may, for example, be prepared as described in WO 03/002136.

Additional formulations which are suitable for other modes of administration include suppositories and, in some cases, oral, pulmonary, nasal, buccal, sublinqual, intraperitoneal, intravaginal, anal and intracranial formulations. For suppositories, traditional binders and carriers may include, for example, polyalkalene glycols or triglycerides; such suppositories may be formed from mixtures containing the active ingredient in the range of 0.5% to 10%, preferably 12%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%. In addition , depending on the form, the duration of action may further be enhanced by controlled release preparations. The inventive conjugate molecules are incorporated into particles of a polymeric material such as polyesters, polyamino acids, hydrogels, poly(lactic acid) or ethylene vinylacetate copolymers.

The choice of drug is often influenced by the way it is administered, as this can make the difference between a drug's success and failure. So the choice of a delivery route can be driven by patient acceptability, important properties of the drug (e.g. solubility), the ability to target the disease location, or effectiveness in dealing with the specific disease. In a preferred embodiment of the conjugate molecule of the present invention, it is contained in a medicament formulated for oral, nasal or pulmonary application or by injection.

The most important drug delivery route is the peroral route. Peptide-based drugs do not easily cross mucosal surfaces and biological membranes, since they are easily denatured or degraded due to rapid clearance in the liver and other body tissues. Furthermore, they require precise dosing. While the fusion peptide as such will typically be administered by injection, the inventine conjugate molecule will be accessible for oral administration. So, despite the barriers to successful drug delivery that exist in the gastrointestinal tract (e.g. acid-induced hydrolysis in the stomach, enzymatic degradation throughout the gastrointestinal tract, bacterial fermentation in the colon), the peroral route is for the inventive conjugate molecule is available.

Alternatively, pulmonary delivery is also important for the administration of the present conjugate molecule and is effected in a variety of ways - via aerosols, metered dose inhaler systems, powders (dry powder inhalers) and solutions (nebulizers), which may contain nanostructures such as liposomes, micelles, nanoparticles and dendrimers. Pulmonary drug delivery of the present conjugate molecule offers a viable option for the delivery of the fusion peptide systemically. The inventive conjugate molecule paves the way for pulmonary delivery of the fusion peptide, since its degradation by proteases in the lung, which reduce their overall bioavailability is diminished. Moreover, the inventive comjugate molecule crosses the barrier between the capillary blood and alveolar air (the air-blood barrier).

Furthermore, it is possible to deliver the conjugate molecule systemically using the nasal route through the association of active fusion peptide with polymeric constituents. It is preferred to combine the inventive fusion peptide with bioadhesive materials or absorption enhancers in order to provide a nasally administerred formulation. The choice of the material will depend on various parameters, e.g. different physical and chemical characteristics of drugs and the different target areas of the drug within the body. The effectiveness will also depend up un whether the nasally administered formulation will be provided as a liquid, powder, gel or microsphere. A nasal solution formulation of the inventive fusion peptide or an inventive conjugate molecule combined with the cationic material polysaccharide chitosan may considerably enhance the absorption of the inventive fusion peptide across the nasal mucosa. Chitosan is a bioadhesive excipient. It is derived from the shells of crustaceans. It is synthesised by the deacetylation of chitin and has been found to be both biocompatible and biodegradable. It demonstrates no allergic effects and is biocompatible with both healthy and infected human skin. It is assumed that its positively charged molecules interact with the negatively charged sialic acid residues present in mucus, thereby retaining drug compound at the mucosal surface for an extended period of time. The nasal formulation may be prepared by ionotropic gelation of chitosan glutamate and tripolyphosphate pentasodium and by simple complexation of the inventive peptide and chitosan. The chitosan molecule may also be covalently linked to the fusion peptide to form an inventive conjugate molecule.

Another approach to provide a nasal formulation for the inventive fusion peptide or the inventive complex is the combination with an alkylglykosid, preferably a medium chain length alkylglycoside or a long chain alkylglycoside, e.g. octylmaltoside or tetradecylmaltoside. Bioavailability of nasally administered inventive peptide or conjugate complex may be considerably enhanced by the combination with an alkylglykoside.

The fusion peptides as part of the inventive conjugate molecule may be formulated as neutral or salt forms. A peptide of the present invention may be sufficiently acidic or sufficiently basic to react with any of a number of organic and inorganic bases, and organic and inorganic acids, to form an (addition) salt, e.g. formed with the free amino groups of the peptide. Acids commonly employed to form acid addition salts are inorganic acids such as hydrochloric acid, hydrobromic acid, hydroiodic acid, sulfuric acid, phosphoric acid, and the like, and organic acids such tartaric, asp-toluenesulfonic acid, methanesulfonic acid, oxalic acid, mandelic, p-bromophenyl-sulfonic acid, carbonic acid, succinic acid, citric acid, benzoic acid. Examples of such salts include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleat, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolat, tartrate, methanesulfonate, propanesulfonate, Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like. Acid addition salts, carboxylate salts, lower alkyl esters, and amides of the fusion peptides of the inventive conjugate molecule may be formulated according to WO 91/11457 (1991); EP 0 733 644 (1996); and U.S. 5,512,549 (1996).

Formulations containing conjugate molecule having the fusion peptide sequences are administered in a manner compatible with the dosage formulation, and in such amount as will be therapeutically effective. The quantity to be administered depends on the subject to be treated, including, e.g., the severity of the patient's disease. Suitable dosage ranges are e.g. of the order of several hundred micrograms active ingredient per therapeutic dose with a preferred range from about 0.1 µg to 2000 µg (even though higher amounts in the 1-10 mg range are contemplated), such as in the range from about 0.5 µg to 1000 µg, preferably in the range from 1 µg to 500 µg and especially in the range from about 10 µg to 100µg.

Formulations containing the inventive conjugate molecules plus e.g. additional excipients, e.g., glycine and mannitol or other additives, may be marketed in a lyophilized form as vials. A companion diluent vial is provided, allowing the patient to reconstitute the product to the desired concentration prior to administration of the dose. Inventive formulations can also be marketed in other well known manners, such as prefilled syringes, etc.

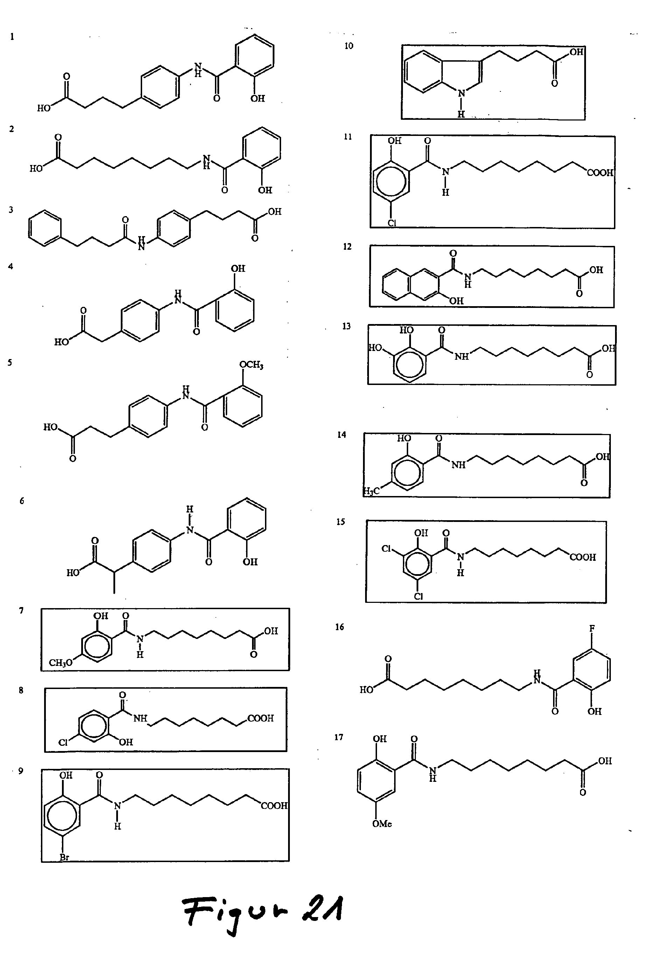

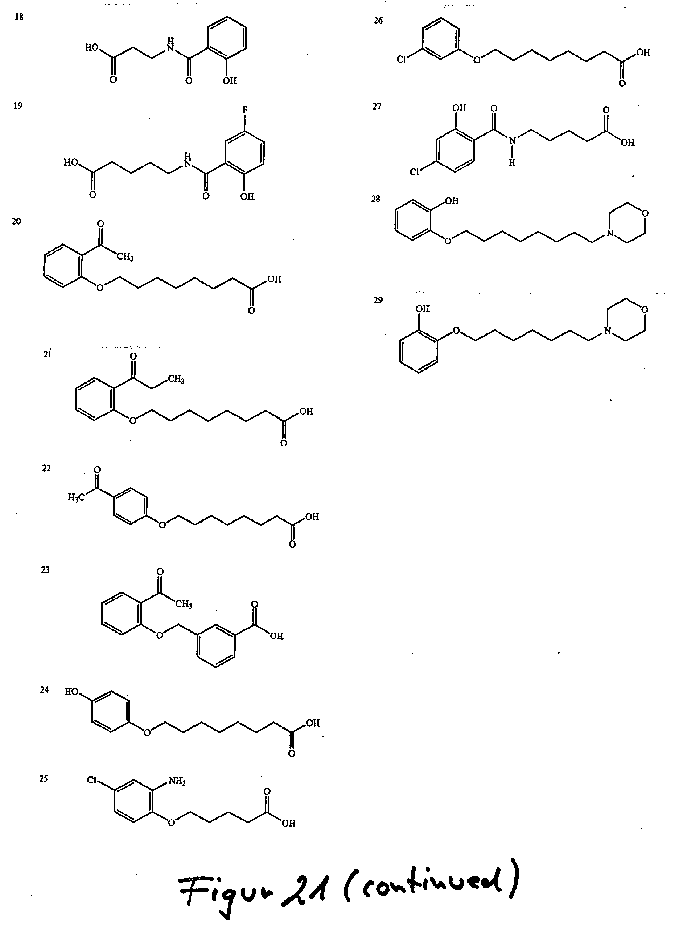

A pharmaceutical composition, in particular for oral application, may comprise in addition to the inventive conjugate molecule at least one delivery agent. These pharmaceutical compositions facilitate the oral delivery of the inventive conjugate molecule, providing a still further improved (e.g. increased) bioavailability of the inventive conjugate molecule compared to administration without a delivery agent. The delivery agent may be selected from the compounds of figure 21, including salts thereof.

The delivery agents of the present invention include those disclosed in WO 96/21464, WO 96/30036, WO 00/06534, WO 98/34632, WO 00/07979, WO 01/44199, WO 1/32596, WO 02/18969, WO 03/045306, and WO 03/072195. Each of these prior applications are incorporated by reference.

Many of the delivery agents uselful for a composition of the present invention can be readily prepared from amino acids including, but not limited to, aminocaprylic acid, butyrylhydroxaminic acid, aminophenylbutyric acid, aminophenylhexanoic acid, aminophenylpropionic acid, aminosalicylic acid, aminophenylsuccinic acid, aminononanic acid, aminonicotinic acid, aminovalenic acid, aminophenylacetic acid, aminocaproic acid, aminoundecanoic acid, aminoheptanoic acid, aminohydroxybenzoic acid, and aminodecanoic acid.

For example, these delivery agents may be prepared by reacting the single acid with the appropriate agent which reacts with free amino moiety present in the amino acids to form amides. Protecting groups may be used to avoid unwanted side reactions as would be known to those skilled in the art.

The delivery agent compounds may be in the form of the free base or salts thereof. Suitable salts include, but are not limited to, organic and inorganic salts, for example sodium, potassium, ammonium, acetate salt, citrate salt, halide (preferably hydrochloride), hydroxide, sulfate, nitrate, phosphate, hydrochloric acid, sulfuric acid, phosphoric acid, chloride, bromide, iodide, propionate, hydrobromic acid, alkoxy, perchlorate, tetrafluoroborate, carboxylate, mesylate, fumerate, carbonate, malonate, succinate, tartrate, acetate, gluconate, and maleate. Preferred salts include, but are not limited to, citrate and mesylate salts. The salts may also be solvates, including ethanol solvates, and hydrates.

Salts of the delivery agent compounds used in the present invention may be prepared by methods known in the art. For example, citrate salts and mesylate salts may be prepared in ethanol, toluene and citric acid.

The delivery agents may be purified by recrystallization or by fractionation on solid column supports. Suitable recrystallization solvent systems include acetonitrile, methanol and tetrahydrofuran. Fractionation may be performed on a suitable solid column supports such as alumina, using methanol/n-propanol mixtures as the mobile phase; reverse phase column supports using trifluoroacetic acid/acetonitrile mixtures as the mobile phase; and ion exchange chromatography using water as the mobile phase.

When anion exchange chromatography is performed, preferably a subsequent 0-500 mM sodium chloride gradient is employed.

The delivery agent may contain a polymer conjugated to it such as described in WO 03/045306. For example, the delivery agent and polymer may be conjugated by a linkage group selected from the group consisting of -NHC(O)NH-, -C(O) NH-, -NHC(O),-OOC-,-COO-,-NHC(O)O-,-OC(O)NH-,-CH2NH-NHCH2-,-CH2NHC(O)O-,-OC(O)NHCH2-, - CH2NHCOCH2O-,-OCH2C(O)NHCH2-,-NHC(O)CH20-,-OCH2C(O)NH-, -NH-,-O-, and carbon-carbon bond, with the proviso that the polymeric delivery agent is not a polypeptide or polyamino acid. The polymer may be any polymer including, but not limited to, alternating copolymers, block copolymers and random copolymers, which are safe for use in mammals.

Preferred polymers for modifying the delivery agent include, but are not limited to, polyethylene; polyacrylates; polymethacrylates; poly (oxyethylene); poly (propylene); polypropylene glycol; polyethylene glycol (PEG); and derivatives thereof and combinations thereof. The molecular weight of the polymer typically ranges from about 100 to about 200,000 daltons. The molecular weight of the polymer preferably ranges from about 200 to about 10,000 daltons. In one embodiment, the molecular weight of the polymer ranges from about 200 to about 600 daltons and more preferably ranges from about 300 to about 550 daltons.

Delivery agents of the present invention are described in U.S. Pat. Nos. 6,663,898, 6,663,887, 6,646,162, 6,642,411, 6,627,228, 6,623,731, 6,610,329, 6,558,706, 6,525,020, 6,461,643, 6,461,545, 6,440,929, 6,428,780, 6,413,550, 6,399,798, 6,395,774, 6,391,303, 6,384,278, 6,375,983, 6,358,504, 6,346,242, 6,344,213, 6,331,318, 6,313,088, 6,245,359, 6,242,495, 6,221,367, 6,180,140, 5,541,155, 5,693,338, 5,976,569, 5,643,957, 5,955,503, 6,100,298, 5,650,386, 5,866,536, 5,965,121, 5,989,539, 6,001,347, 6,071,510, and 5,820,881. Delivery agents of the present invention are also described in U.S. Published Application Nos. 20030232085, 20030225300, 20030198658, 20030133953, 20030078302, 20030072740, 20030045579, 20030012817, 20030008900, 20020155993, 20020127202, 20020120009, 20020119910, 20020102286, 20020065255, 20020052422, 20020040061, 20020028250, 20020013497, 20020001591, 20010039258, 20010003001. Delivery agents of the present invention are also described in International Published Application Nos. 2003/057650, 2003/057170, 2003/045331, 2003/045306, 2003/026582, 2002/100338, 2002/070438, 2002/069937, 02/20466, 02/19969, 02/16309, 02/15959, 02/02509, 01 /92206, 01 /70219, 01 /51454, 01 /44199, 01 /34114, 01 /32596, 01 /32130, 00/07979, 00/59863, 00/50386, 00/47188, 00/40203, 96/30036. Each of the above listed U.S. Patents, U.S. Pulished Applications, and Published International Applications are herein incorporated by reference.

An "effective amount of delivery agent" refers to an amount of the delivery agent that promotes the absorption of a therapeutically effective amount of the fusion peptide as part of the inventive conjugate molecule from the gastrointestinal tract. Typical amounts of delivery agents and the inventive conjugate molecule used in testing with male Sprague-Dawly rats range from 0 to 3000 [mu]g/kg of, the inventive conjugate molecule and 0 to 200 [mu]g/kg of delivery agent.

The administration composition comprises one or more delivery agent compounds and the conjugate molecule of the invention. In one embodiment, one or more of the delivery agent compounds, or salts of these compounds, or polyamino acids or peptides of which these compounds or salts form one or more of the units thereof, may be used as a delivery agent by mixing with the inventive conjugate molecule prior to administration to form an administration composition.

The oral administration compositions are preferably in the form of a solid, such as a tablet, capsule or particle, such as a powder or sachet. Solid dosage forms may be prepared by mixing the solid form of the compound with the solid form of the active agent. Alternately, a solid may be obtained from a solution of compound and active agent by methods known in the art, such as freeze-drying (lyophilization), precipitation, crystallization and solid dispersion.

The various oral formulations may optionally encompass a suspending agent. Some delivery agents require a suspending agent due to their solubility characteristics. An example of a suspending agent is hydroxypropylmethylcellulose. Preferably, the final concentration of hydroxypropylmethylcellulose is between about 2% and about 10% (weight/volume). Even more preferably, the concentration is between about 2% and about 5% (w/v). Most preferably the concentration is about 3.9% (w/v).

However, because the compositions of the invention may deliver the fusion peptide more efficiently than compositions containing the inventive fusion peptide alone (without a covalently coupled polymer constituent(s)), lower amounts of the inventive fusion peptide than those used in prior dosage unit forms or delivery systems can be administered to the subject, while still achieving the same blood levels and therapeutic effects.

The administration composition may also be in the form of a liquid. The solution medium may be water, 25% aqueous propylene glycol, and/or a phosphate buffer. Other dosing vehicles include polyethylene glycol. Dosing solutions may be prepared by mixing a solution of the delivery agent with a solution of the conjugate molecule active agent, just prior to administration. Alternately, a solution of the delivery agent compound (or the inventive conjugate molecule) may be mixed with the solid form of the the inventive conjugate molecule active agent (or delivery agent compound). The delivery agent compound and the inventive conjugate molecule may also be mixed as dry powders. The delivery agent compound and the inventive conjugate molecule can also be admixed during the manufacturing process. The administration composition of the present invention may optionally comprise a cosolvent. Some delivery agents require cosolvents due to their solubility characteristics. Examples of cosolvents include ethanol, N-methylpyrrolidone, N,N-dimethylacetamide,N,N-dimethylformamide, glycofurol, ethoxydiol, propylene glycol, polyethylene glycol 300 and polyvinylpyrrolidone. Preferably, the final concentration of the cosolvents is between about 5% and about 30% (volume/volume). Even more preferably, the concentration is between about 10% and about 25% (v/v). Most preferably the concentration is about 20% (v/v).

In aqueous liquid form, pH of the administration composition may be adjusted to provide stability and to be acceptable for oral administration. The pH may be adjusted to between about 7.0 and about 9.0. More particularly, the pH may be adjusted between about 7.4 and 8.4, or between 7.8 and 8.4, or between about 7.8 and 8.1

### Examples

### Example 1

### Creation of genetic constructs

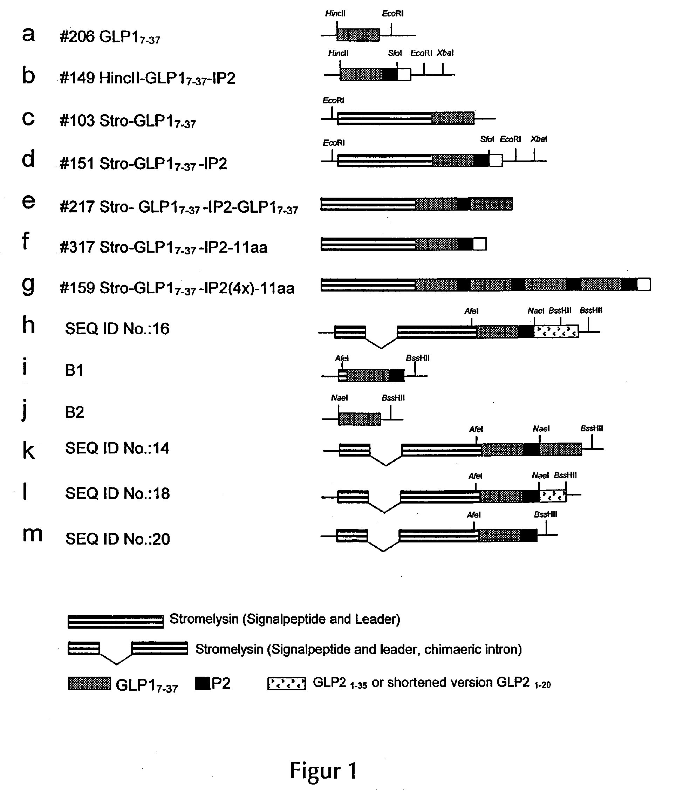

The coding sequence for GLP-1(7-37) cDNA was synthesised synthetically, in a sequence including HincII and EcoRI sites as indicated in Fig. 1a. Separately the cDNA illustrated in Fig. 1b was synthesised, including the coding sequences for GLP-1(7-37), IP2 and restriction sites for Sfol, EcoRI and Xbal, as illustrated in Fig. 1 b. To direct GLP-1 to the secretory pathway, the heterologous signal sequence of stromelysin 3 (Acc.No. NM_005940) was used. Therefore the cDNA, encoding stromelysin signal and leader sequence was reverse transcriptase PCR amplified from human RNA, and used with the construct of Fig. 1a or Fig. 1b to form the construct shown in Fig. 1c and Fig. 1d, respectively.

The HincII/EcoRI fragment of the Fig. 1a construct is cloned into the Sfol site of the sequence of Fig. 1d to form the construct Fig. 1e. Similarly, the EcoRI fragment of Fig. 1d is cloned into the EcoRI site of an eukaryotic expression plasmid, to produce the construct shown in Fig. 1f. To form the construct shown in Fig. 1g, the Hincll/Xbal fragment of the construct shown in Fig. 1b is repetitively cloned into the Sfol/Xbal site of the construct shown in Fig. 1d. Figure 1h shows a synthesized, codon optimized sequence encoding the stromelysin leader and signal sequences interrupted by a shortened endogenous intron sequence, fused to sequences encoding human GLP-1(7-37), IP2 and GLP-2(1-35). The DNA sequence of the construct Fig. 1h is SEQ ID No.:16, while SEQ ID No.:15 also shows the sequence of the translated peptide.

Also synthesised are the sequences in Figs 1i and 1j. These are then used to form the construct in Fig. 1k, by cloning the Nael/BssHII fragment of Fig. 1j into the Nael/BssHII linearised sequence of Fig. 1h. The DNA sequence of the construct Fig. 1k is SEQ ID No.: 14, while SEQ ID No.:13 also shows the sequence of the translated peptide. The construct of Fig. 1l is formed by BssHll digest and religation of the sequence of Fig. 1h. The DNA sequence of the construct Fig. 1l is SEQ ID No.: 18, while SEQ ID No.:17 also shows the sequence of the translated peptide. The construct of Fig. 1 m is formed by cloning the Afel/BssHII fragment of the sequence of Fig. 1i into the Afel/BssHII linearised sequence of Fig. 1h. The DNA sequence of the construct Fig. 1m is SEQ ID No.: 20, while SEQ ID No.:19 also shows the sequence of the translated peptide.

The above constructs may be made by a person skilled in the art using routine techniques.

### Example 2

### Transfection, clonal selection and GLP-1 expression of mammalian cells

Source of the cells: HEK293 (human embryonic kidney cell line, # ACC 305, DSMZ Cell Culture Collection, Germany), AtT20 (Mouse LAF1 pituitary gland tumor cell line, #87021902, European Cell Culture Collection, UK), hTERT-MSC cells are generated by Prof. Kassem, University Hospital of Odense, Denmark.

For transfection of 10⁶ cells 0,5-2 µg plasmid DNA with different GLP-1 constructs was used. The constructs were generated as described in Example 1. HEK293 cells were transfected by standard calcium phosphate co-precipitation method as described in Current Protocols in Molecular Biology (Ausubel et al. 1994ff Harvard Medical School Vol2., Unit 9.1). AtT20 cells were transfected using FuGene (Roche) as described in current Protocols in Molecular Biology (Ausubel et. al. 1994ff, Harvard Medical School Vol 2., Unit 9.4). Transfection of hTERT-MSC cells was performed using the Nucleofector technology (Amaxa), a non-viral method which is based on the combination of electrical parameters and cell-type specific solutions. Using the Nucleofector device (program C17) and the Nucleofector solution VPE-1001 transfection efficiencies >60% have been achieved. 48 hours after transfection selection of cell clones with stable integration of DNA into the chromosome was performed by adding the selective agent blasticidin (2 µg/ml) into the culture medium. 12-15 days later, stable transfected cell clones could be isolated and expanded for characterisation.

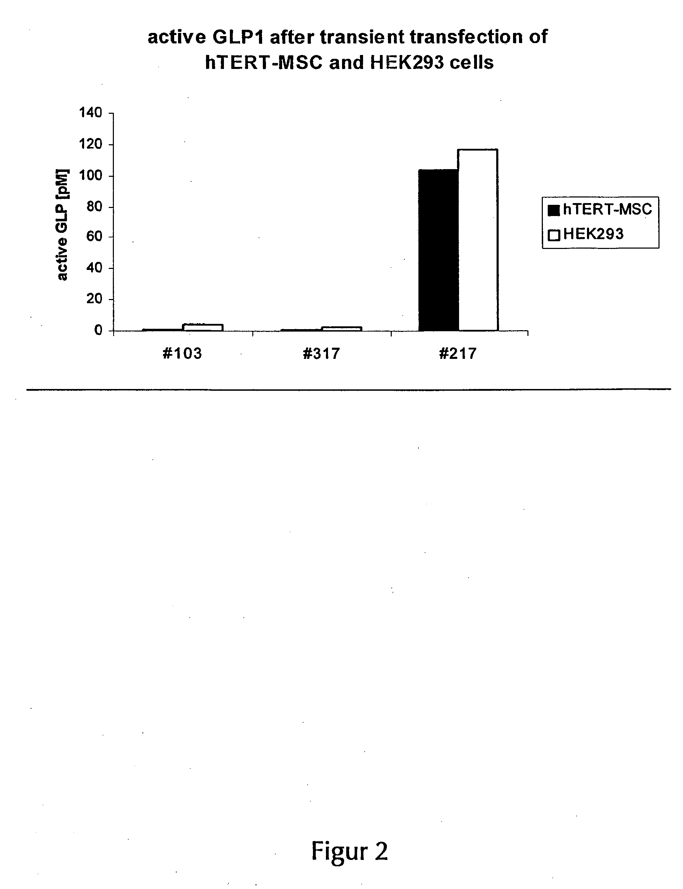

Transient expression of different GLP-1 constructs was measured in hTERT-MSC and HEK293 cells. Whereas only marginal active GLP-1 level can be found in the monomeric GLP-1 constructs #103 and #317 (having just one copy of GLP-1 (7-37) an enormous gain in expression can be found in the dimeric GLP-1 construct #217 (having GLP-1(7-37) as component (I) and as component (III)) both in hTERT-MSC and in HEK293 cells. Results are summarized in Figure 2. An elongation of the construct to the GLP-1 construct #159 (having four IP2 copies as component (II)) results in no further significant increase (not shown). After transfection of hTERT-MSC cells with different constructs clones were selected, which stably express GLP-1. The expression levels are shown in Table 1 .

**Table 1**

| construct | cell clone | active GLP per 10⁶ cells and hour [pmol] |
|---|---|---|
| #103 -GLP1₍₇₋₃₇₎ | 49TM113/13 | 0,4 |
| #317-GLP1₍₇₋₃₇₎-IP2-11aa | 71TM169/1 | 0,3 |
| #217 -GLP1₍₇₋₃₇₎-IP2-GLP1₍₇₋₃₇₎ | 79TM217/13 | 2,7 |

### Example 3

### Western Blot Analysis of GLP-1 peptides, secreted from mammalian cells

Cell culture supernatant from GLP-1 secreting cells was separated in a 10%-20% gradient SDS PAGE (120V, 90 minutes) and transferred to a PVDF membrane (immobilon-P Membrane 0,45 µm Millipore IPVH 00010) by semi-dry blotting (2.0 mA/cm2, 60 minutes). After methanol fixation and blocking (3% (w:v) BSA, 0.1 % (v:v) Tween-20 in TBS) the membrane was immunoblotted with 1 µg/ml anti-GLP-1 antibody (HYB 147-12, Antibodyshop) at 4°C o/n. After washing and incubation with 0.02 µg/ml detection antibody (Anti Mouse IgG, HRP conjugated, Perkin Elmer PC 2855-1197) at RT for 4 hours, chemiluminescence detection reveals the location of the protein.

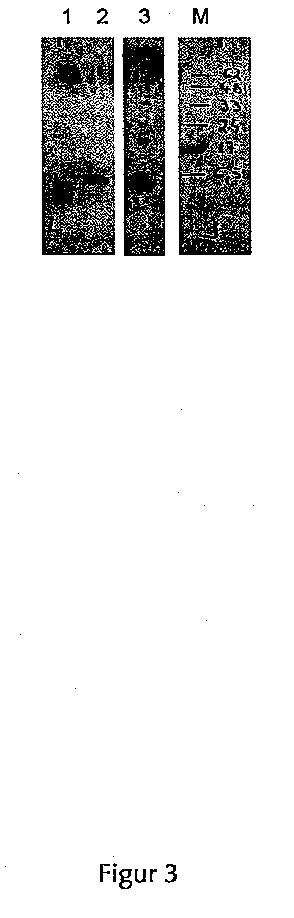

Western Blot Analysis is shown in Figure 3 (1: 100 ng synthetic GLP-1(7-37) dissolved in supernatant of mock transfected hTERT-MSC cells, 2: supernatant of hTERT-MSC cells (clone 79TM217/13) secreting dimeric GLP-1 from construct #217, 3: supernatant of AtT20 cells (clone 81-A-217/3) secreting dimeric GLP-1 from construct #217; M: prestained protein marker [kDa]). The results show that inventive peptides containing GLP-1(7-37) and a C-terminal appendix (2 and 3 in Fig. 3) are secreted from the transfected cell lines and can be detected using an anti-GLP-1 antibody, which binds to the mid-molecular epitopes of GLP-1 (7-37).

### Example 4

### In vitro plasma stability of GLP-1 peptides secreted from human cells

HEK293 and hTERT-MSC cells were transiently transfected with constructs, encoding the heterologous stromelysin signal sequence, which is linked to GLP-1 variants encoding the following peptides:
1: GLP-1 (7-37)
2: GLP-1(7-37)-IP2-extended with 11 AA
3: GLP1(7-37)-IP2-GLP1(7-37)

Cell culture supernatant, containing GLP-1 peptides secreted from cells or synthetic GLP-1(7-37) (Bachem) was incubated with human lymphocyte enriched plasma containing dipeptidylpeptidase activity at 37°C and 5% CO₂, for 3 or 4 hours. Synthetic GLP-1(7-37) in supernatant from mock transfected cells was used as a positive control for DPP-IV activity, which was shown to be inhibited by addition of an DPP-IV inhibitor (#DPP4, Biotrend). Active GLP was measured using the GLP-1 (Active) ELISA (#EGLP-35K, Biotrend), using an antibody which binds to the N-terminal epitope of GLP-1(7-37) discriminating the DPP-IV degraded, inactive GLP-1 (9-37) peptide.

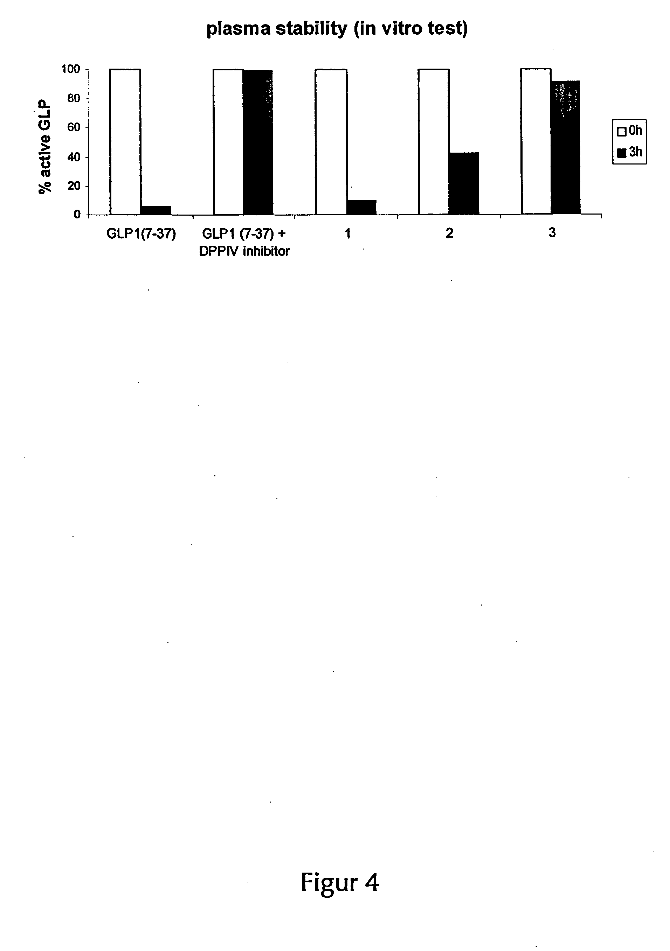

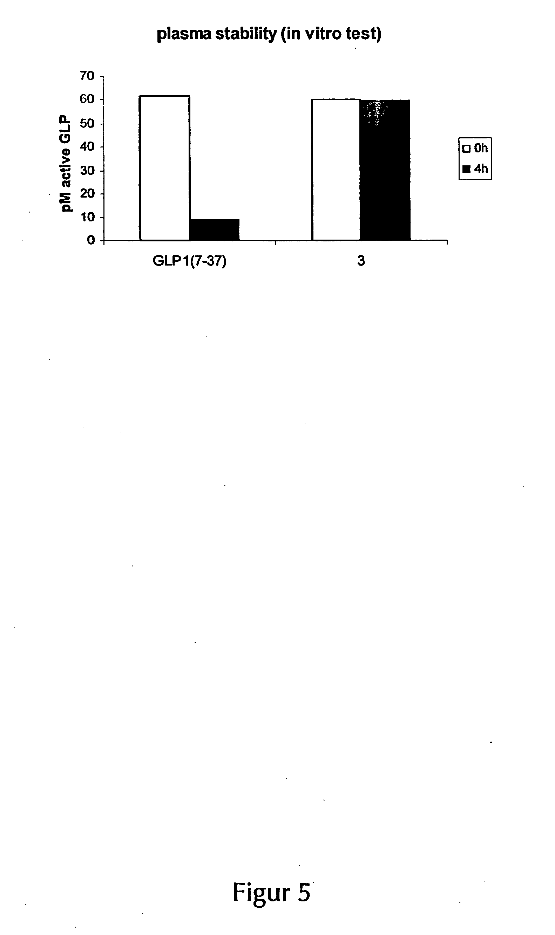

The results are shown in Figures 4 (HEK293 cells) and 5 (hTERT-MSC cells). HEK293 and hTERT-MSC cells are both effective hosts for the gene construct. The numbering of the results for the transfected cells of types 1 to 3 is as with Example 3 (1: 100 ng synthetic GLP-1(7-37) dissolved in supernatant of mock transfected hTERT-MSC cells, 2: supernatant of hTERT-MSC cells (clone 79TM217/13) secreting dimeric GLP-1 from construct #217,3: supernatant of AtT20 cells (clone 81-A-217/3) secreting dimeric GLP-1 from construct #217). While construct 1 produces wild type GLP-1 which is inactivated by DPP-IV in a similar way to synthetic GLP-1, the inventive C-terminally elongated GLP-1 forms (2 and 3 in Figure 4, 3 in Figure 5) are more resistant to degradation and maintain at least 40% activity. The C-terminal extended GLP-1 peptides are significantly stabilised in human plasma in vitro. The peptide with the dimeric GLP-1 sequence (3) is nearly fully stabilised to DPP-IV degradation in vitro.

### Example 5

### Western Blot Analysis of GLP-1 peptides

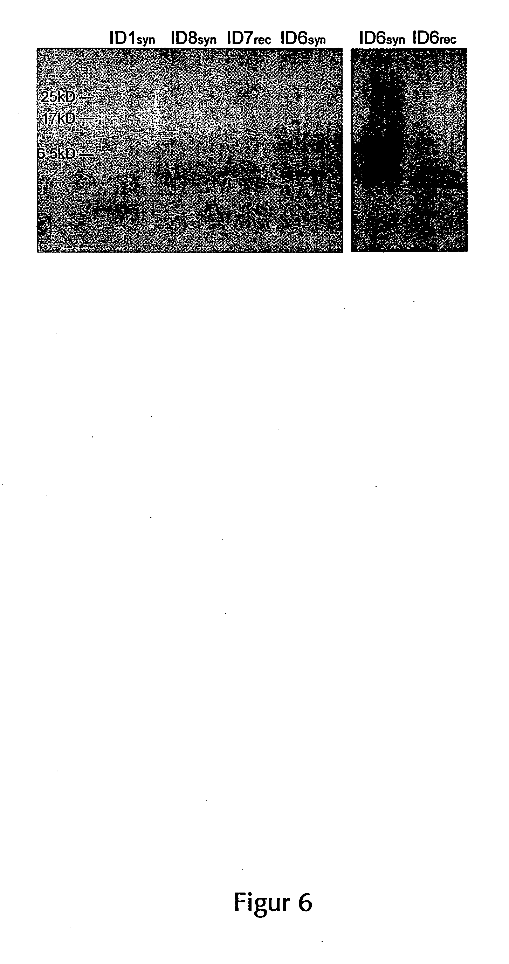

Various GLP-1 peptides were produced synthetically by solid phase *(syn)* or recombinant using E.coli (rec). GLP-1 peptides (31 ng SEQ ID No:1 and 10 ng of each SEQ ID No:6, SEQ ID No:7, SEQ ID No:8) were separated in a 10%-20% gradient SDS PAGE (120V, 90minutes) and transferred to a PVDF membrane (Immobilon-P Membran 0,45 µm Millipore IPVH 00010) by semi-dry blotting (2.0 mA/cm², 60 minutes). After methanol fixation and blocking (3% (w:v) BSA, 0.1% (v:v) Tween-20 in TBS) the membrane was immunoblotted with 1 µg/ml anti-GLP-1 antibody (HYB 147-12, Antibodyshop) at 4°C o/n. After washing and incubation with 0.02 µg/ml detection antibody (Anti Mouse IgG, HRP conjugated, Perkin Elmer PC 2855-1197) at RT for 4 hours, chemiluminescence detection reveals the location of the protein. Fig. 6 shows a Western Blot for the peptides indicated. The following values can be given: SEQ ID No.: 1 (ID1syn) corresponds to GLP-1(7-37), 31 aa, 3,3 kD; SEQ ID No.:8 (ID8 syn, CM3) corresponds to GLP-1(7-37)-IP2, 46 aa, 5,1 kD; SEQ ID No.: 7 (ID7rec, CM2) corresponds to GLP-1(7-37)-IP2-RR-GLP2, 83 aa, 9,4 kD; SEQ ID No.: 6 (ID6syn, CM1) corresponds to GLP-1(7-37)-IP2-RR-GLP1(7-37), 79 aa, 8,7 kD.

### Example 6

### In vitro human plasma stability of GLP-1^{CM} peptides

Synthetic GLP-1 peptides (SEQ ID No:1_{syn}, SEQ ID No:6_{syn}, SEQ ID No:7_{rec}, SEQ ID No:8_{syn}) were incubated at concentrations of 20 ng/ml with human plasma at 37°C and 5% CO₂ for 3 hours. Dipeptidylpeptidase activity of the plasma was inhibited by an DPP-IV inhibitor (#DPP4, Biotrend). Active GLP was measured using the GLP-1 (Active) ELISA (#EGLP-35K, Biotrend).

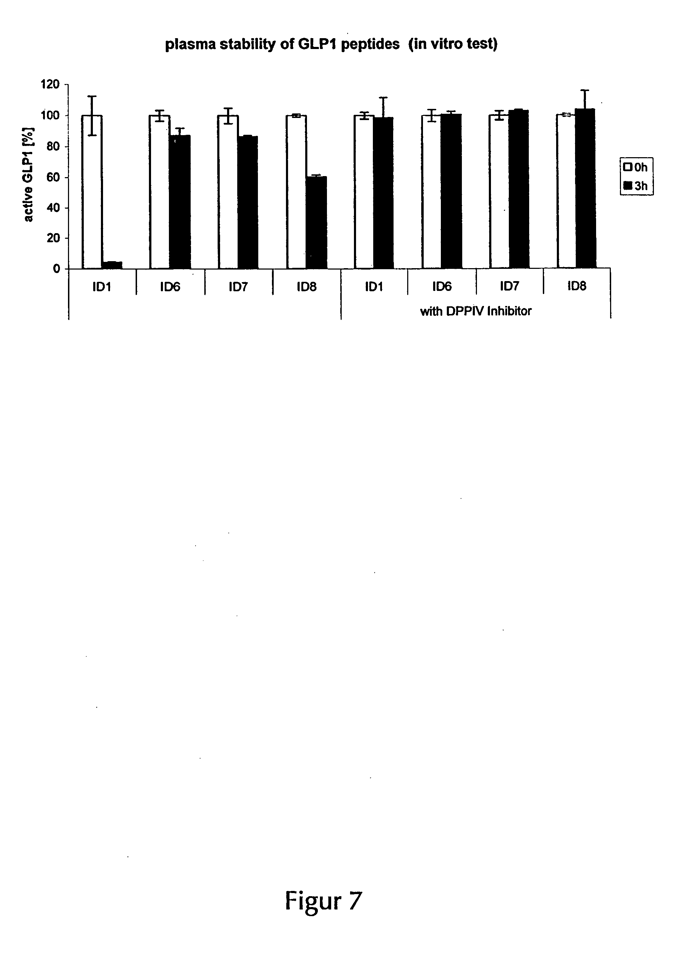

In contrast to the native GLP-1₍₇₋₃₇₎ (SEQ ID No:1) the inventive C-terminal elongated GLP-1 peptides SEQ ID No:6, SEQ ID No:7, and SEQ ID No:8 are significantly stabilized in human plasma in vitro (Fig. 7). As control (on the right hand side) the results obtained for experiments with addition of DPP-IV are shown. GLP-1 activity is completely maintained in these control experiments.

### Example 7

### Bioassay in vitro

### Cyclic AMP Production

RIN₋5F cells (rat islet cell tumor; ECACC No. 95090402) were grown in 24-well plates for 4 days reaching 70% confluence. Cells were washed twice with DMEM (E15-009, PAA) before addition of 0.5 ml DMEM (E15-009, PAA) supplemented with 1% HSA (Aventis), 0.2 mM IBMX (858455, Sigma) and the test peptides. After a 20 minute incubation at 25°C, cells were washed twice with ice cold PBS. Cellular cAMP was extracted by addition of 0.1 N HCl containing 0.5% Triton X-100. Cyclic AMP was quantified using the cAMP (low pH) EIA (Cat. DE0355, R&D). For stimulation 3*10⁻⁸ M SEQ ID No:1, SEQ ID No:6_{syn}, SEQ ID No:6_{rec}, SEQ ID No:7_{rec}, SEQ ID No:8_{syn} have been used.

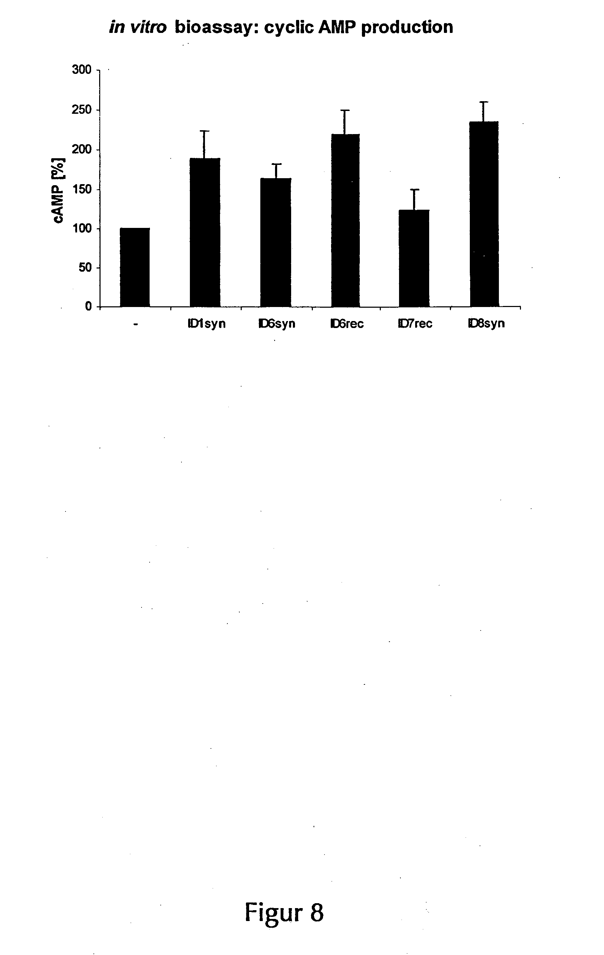

Results are shown in Fig. 8. 100% cAMP production corresponds to the basal production in the absence of GLP-1. GLP-1 binds to G protein-coupled receptors and stimulates cAMP production. All molecules tested increase the cellular cAMP production.

### Example 8

### in vivo bioactivity

11-week-old type II diabetic mice (C57BUKs-Lepr^{db/db} , Harlan) were treated with 5 µg peptide by subcutaneous injection twice a day at 9 a.m. and 5 p.m. (n=5 per group). Blood glucose was measured before (day 0) and after treatment with GLP^{CM} peptides (Day 2, 4, 7, 10) at 10 a.m. after an overnight fastening period. Data were presented in relation to blood glucose levels measured at day 0.

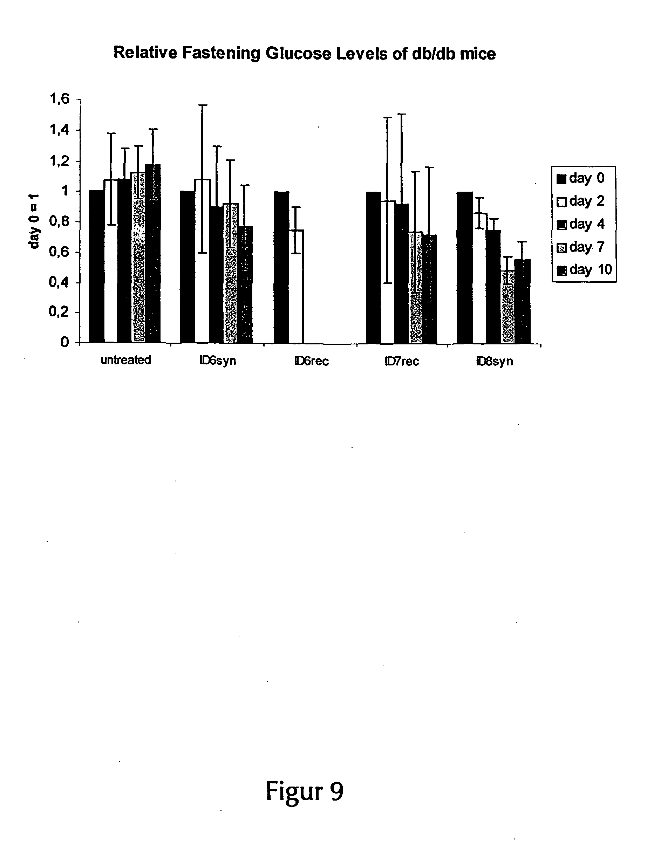

All inventive peptides tested (SEQ ID No.:6 (synthetic or recombinant) and SEQ ID No.:7 (synthetic or recombinant)) have an anti-hyperglycemia effect. Best results were obtained with recombinant SEQ ID No.:6 (CM1) and synthetic SEQ ID No.:8 (CM3). In Fig. 9 (y-axis) the relative effect of the treatment is shown. Blood glucose at day = 0 was set to 1. Untreated animals undergo continuous increase in blood glucose level over time, whereas animals treated with inventive peptides display *grosso modo* a continuous decrease of the blood glucose level over time.

### Example 9

### Measurement of in vitro plasma stability of GLP1^{CM} peptides (kinetic test method)

Aliquots of 1 µM peptide in incubation buffer (50mM Triethanolamin-HCl (pH 7,8), 0.2% HSA) from CM3, Alanin substituted CM3 analogs (CM3-ANA01, CM3-ANA02, CM3-ANA03, CM3-ANA04), C-terminally shortened CM3 analogs (CM3-ANA06, CM3-ANA07) or C-terminally elongated CM3 analogs (CM3-ANA09) were incubated with 10% human plasma at 37°C and 5% CO₂ for 0, 3, 6 and 9 hours. Dipeptidylpeptidase activity was stopped by addition of DPPIV inhibitor (#DPP, Biotrend) and active GLP-1 levels determined using the GLP-1 (active) ELISA (#EGLP-35K, Linco). Results are from triplicates in at least two independent experiments.

Inventive peptides having at least nine amino acids added to the C-terminus of GLP-1 are CM3 (murine, GLP-1(7-37)-IP2) and derivatives thereof with modified sequences in component (II) (IP2), i.e. CM3-ANA01, CM3-ANA01, CM3-ANA02, CM3-ANA03, CM3-ANA04, CM3-ANA05, CM3-ANA07, CM3-ANA07. Peptides GLP-1 and CM3-ANA06 reflect control or reference substances.

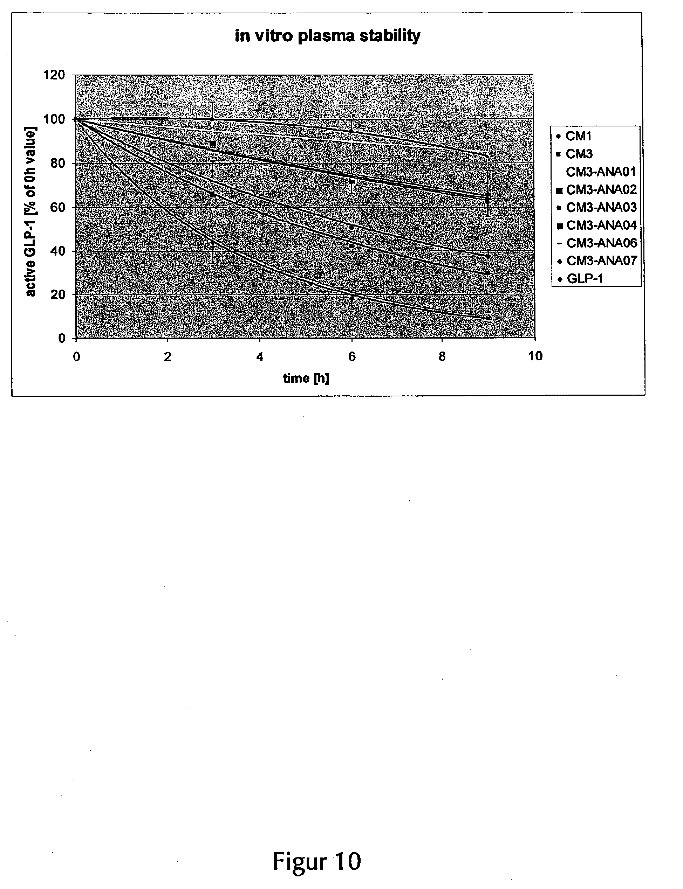

Fig. 10 shows the active portion of active GLP-1 as a % of the 0h value (0h = 100%). It is clearly seen that GLP-1 has the worst plasma stability with only 9% left after 9h plasma exposure. CM3-ANA01 shows the best stability values with 84% material left after 9h. Inventive peptides have a remainder of at least 30% after 9h, whereas peptides with shorter extensions do not reach these values. From the kinetics, a time can be determined, which is needed to degrade active GLP-1 to 80% of the 0h value (DT₈₀: 80% degradation time) for the substances tested.

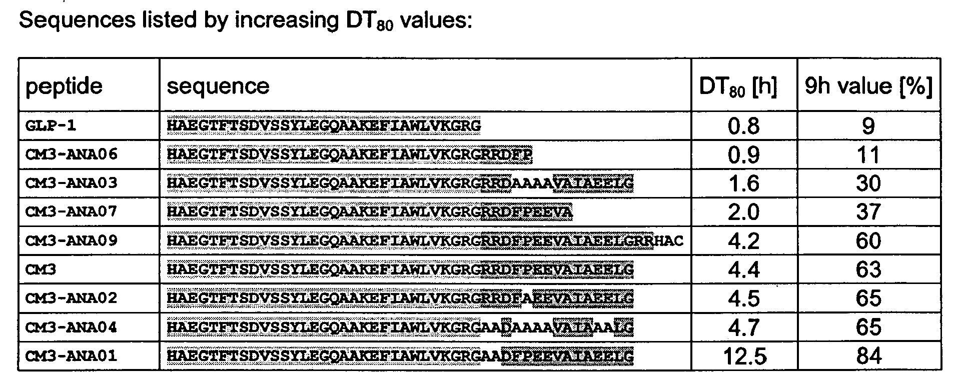

The inventive substances show a considerably longer in vitro plasma stability as compared to the reference substances. Without being bound to any theory, the C-terminal elongation of GLP-1 introduces a steric hindrance, preventing these analogs to enter the active site of the DPPIV, and thus escape degradation, whereas shorter reference substances do not show this protecting effect for GLP-1 stability. This hypothesis is supported by the continuously increasing stability of cumulative C-terminal elongations of the peptides CM3-ANA06 (36aa), CM3-ANA07 (40aa) and CM3 (46aa). Nevertheless this phenomenon is not only dependent on the pure number of amino acids, which has been shown by Alanin substitutions in the IP2 region of CM3. The CM3-ANA03 peptide, which has the same number of amino acids like CM3, has a strongly reduced stability compared to CM3. On the other hand CM3-ANA01, which also consists of 46 amino acids, has a higher plasma stability than CM3. This may indicate that beside the number of amino acids, additional steric effects may influence the stability. Particularly preferred are peptides having an elongation comprising IP2 at the C-terminus of GLP-1 or an elongation having a certain degree of homology with IP2, which may result from a specific conformation, which hinders the N-terminal region from entering the active site of DPPIV.

### Example 10

### In vivo study - Immunogenicity

To elucidate potential immunogenicity of test substance CM1 a mouse study was performed at Parabioscience (Groningen, Germany). The trial was done in BALB/c mice receiving 5 repeated injections (70µg peptide / dose, i.v.) over 22d (n=5).

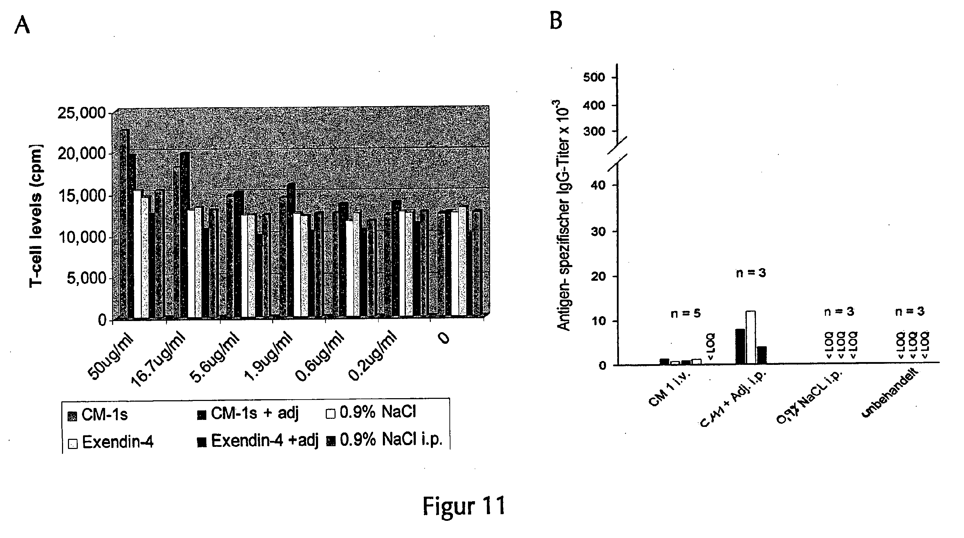

CM1_{syn} induced no toxicity and only a minimal T-cell antibody response and antibody titer as shown in the following figure. An immunologic effect of by-products (purity of the peptide only 95%) cannot be excluded. Fig. 11 shows studies for evaluation of potential immunogenic effects of CM1syn. On the left hand side (Fig. 11A), results of a T-cell recall response assay are shown. Spleens of the treated and control mice were explanted, T-cells isolated and stimulated with increasing amounts of antigene. Proliferative recall response is measured. On the right hand side (Fig. 11 B), the antigen specific IgG antibody titer in the immune sera of the animals was determined.

### Example 11

### Dose-efficacy study in diabetic mice

After a two-hour fastening period diabetic C57BL/KsJ@Rj-db (db/db) mice were treated with five different concentrations of GLP-1, CM1, CM3, CM3-ANA01 and exendin-4. A sixth group was treated with saline only. 7 animals were treated per group. Blood glucose was determined from tail bleeds directly before, 1 hour and 4 hours after injection.

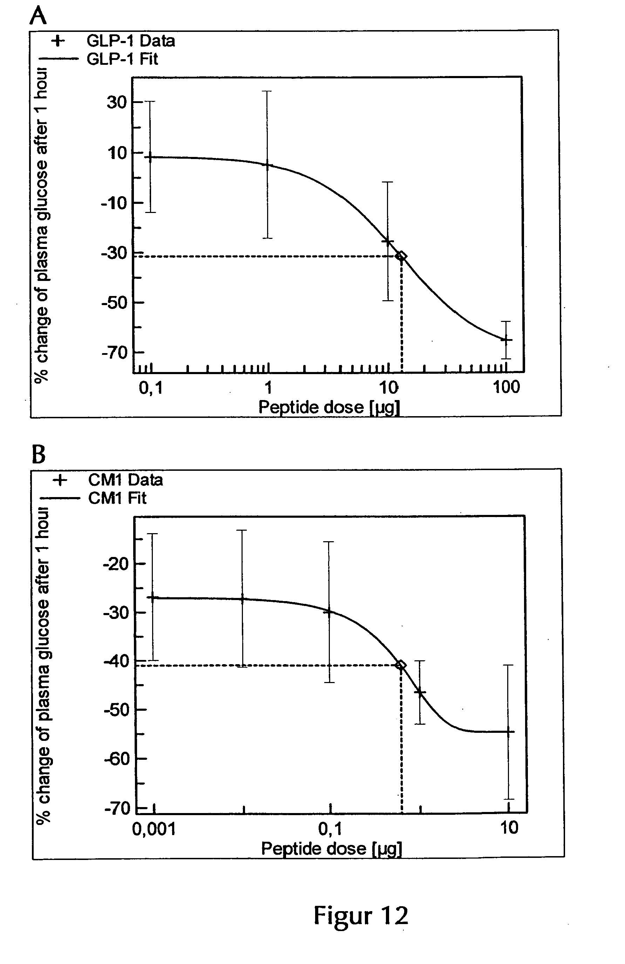

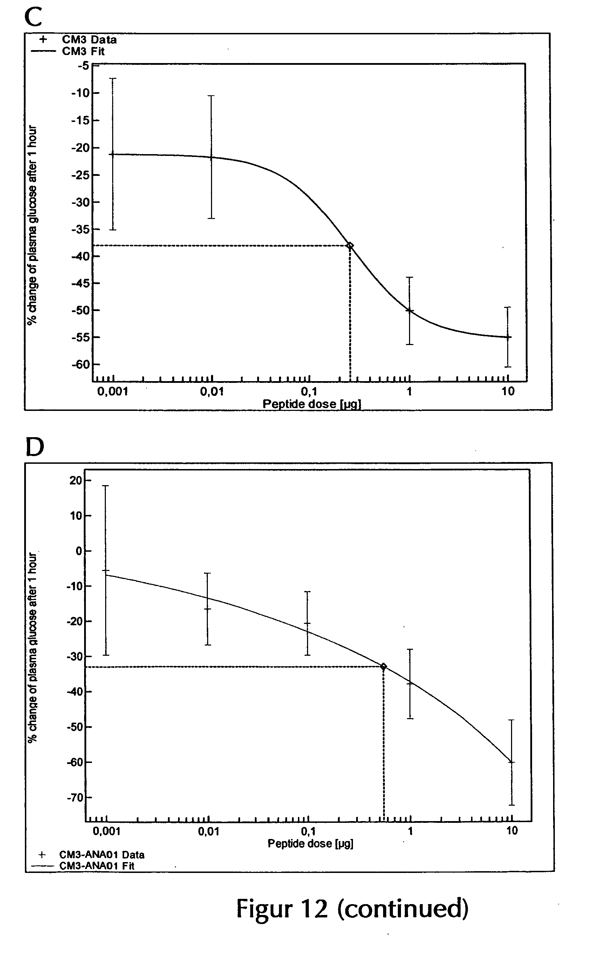

The results are shown in dose-response curves according to Fig. 12 (Fig. 10A (GLP-1), Fig. 12B (CM1), Fig. 12C (CM3) and Fig. 12D (CM3-ANA01)). For every test substance a graph was prepared with the percentage difference of the blood glucose in relation to the start value for the different concentrations. The ED₅₀ value for the different test substances was determined by preparing a dose response curve with the values of the 1 h measurements as a percentage reduction in comparison to the basal value. To evaluate the ED₅₀ value for GLP-1, CM3-ANA01, CM3_{rec} and Exendin-4 the Morgan-Mercer-Flodin (MMF) model was used, for CM1 the Richards model was used. Both models are sigmoidal fit models suitable for dose response evaluation. For every peptide the plasma glucose ED₅₀ values (single s.c. dose in µg / mouse) have been determined from the percentage decrease of blood glucose. They are summarized in table 1.

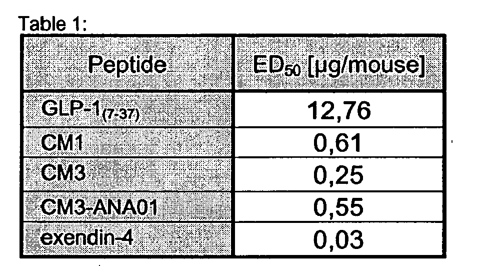

Deduced from the ED₅₀ values, the GLP-1 analogs CM1, CM3 and CM3-ANA01 reveal a more than 20fold better *in vivo* bioactivity, which is most likely a result of the enhanced plasma stability.

All peptides tested lead to a significant decrease of blood glucose levels in hyperglycaemic db/db mice at least for the highest concentrations. The fall in plasma glucose after a single s.c. injection of 1.1 - 3.0 nmol peptide (versus control) is summarized in table 2. Table 2 exhibits the glucose lowering effects of the test substances (versus control). The fall in plasma glucose and the corresponding significance levels are given for the time period 1 hour (@1h) and 4 hours (@4h) after peptide injection. Differences have been observed in the long-term efficacy of the peptides. Only exendin-4 and CM3 revealed a significant decrease of the blood glucose levels after 4 hours.

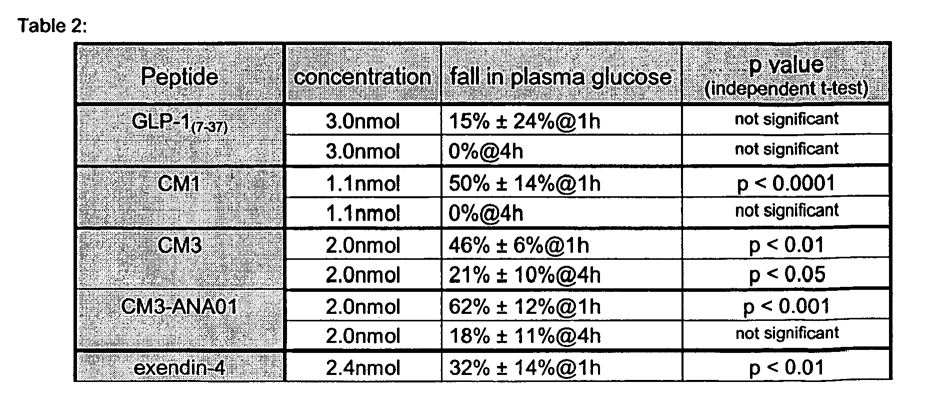

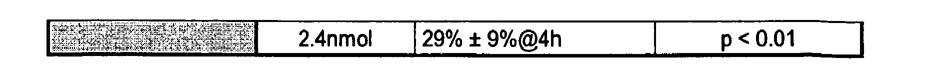

### Example 12

### Long-term treatment of db/db mice

Four groups with n=12 C57BL/KsJ@Rj-db (db/db) mice have been investigated:
- Group A: treatment with **vehicle** (0.9% saline) once daily
- Group B: treatment with 24nmol/kg test substance 1: **CM1rec** once daily
- Group C: treatment with 24nmol/kg test substance 2: **CM3-ANA01** once daily
- Group D: treatment with 24nmol/kg reference substance **exendin-4** (known in the art and approved (however, exendin-4 is not an GLP-1 analog)) once daily

Peptides or vehicle will be given once daily (group A-D) between 2°° - 3°°p.m. subcutaneously in the skin fold of the back. The regimen was continued for 18 weeks. From week 12 to 18 treatment was done twice a day in 6 of 12 animals per group.

Various parameters of the mice were investigated, i.e. health status (1), body weight (2), food consumption (3), blood glucose (4), glucose tolerance test (5), insulin data (6), glycosylated hemoglobin (7), pathology (8), and restimulation of T cells (9).

Health status (1) was good in all groups, no side effects of the treatment have been seen.

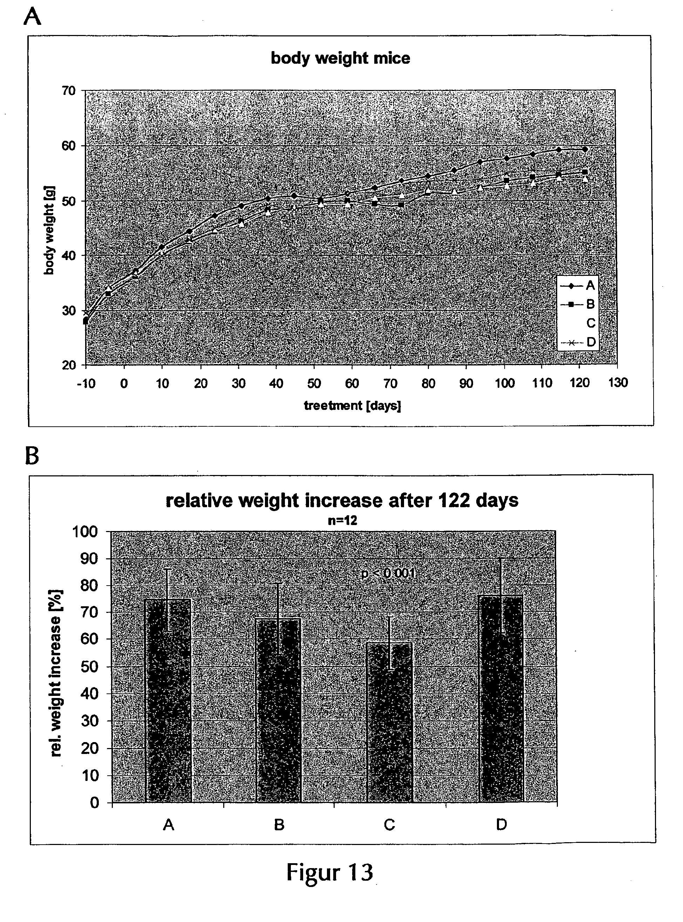

Body weight (2) was lower in all treated groups after 18 weeks of treatment (Fig.13). Relative body weight increase is significantly lower in the CM3-ANA01 group. Fig. 13A shows the effect of a treatment with saline (A), CM1 (B), CM3-ANA01 (C) or exendin (D) on the body weight of db/db mice. The mean values of 12 animals per group are plotted. Fig. 13B shows the effect of a treatment with saline (A), CM1 (B), CM3-ANA01 (C) or exendin (D) on the body weight of db/db mice. The relative body weight increase of 12 animals per group after a treatment of 122 days is plotted. Only the treatment with test substance CM3-ANA01 (group B) revealed a significantly lower weight increase (p<0.001).

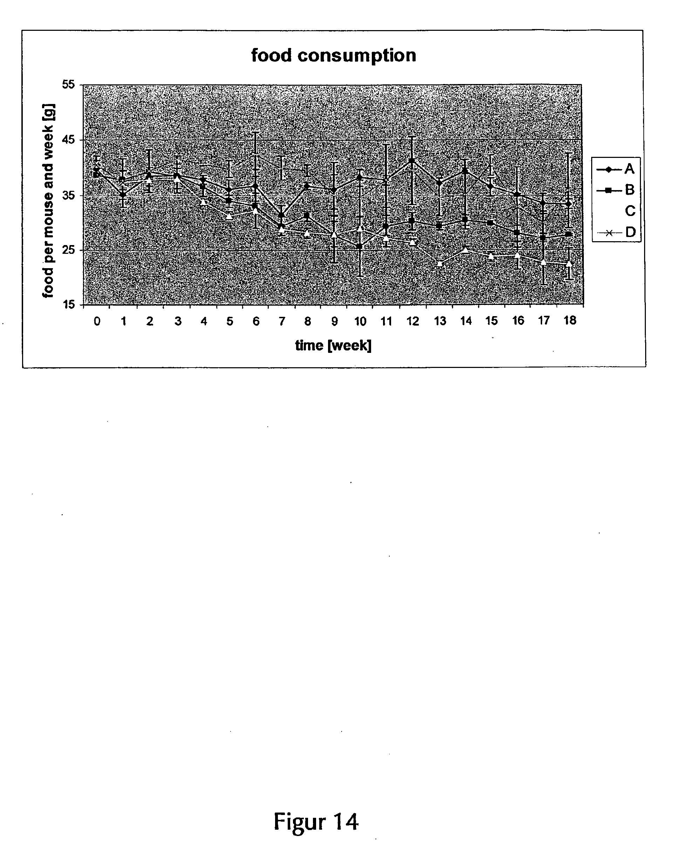

Food consumption (3) was significantly lower in the CM1 and CM3-ANA01 group compared to the control. Fig. 14 shows the effect of a treatment with saline (A), CM1 (B), CM3-ANA01 (C) or exendin (D) on the weekly food consumption of db/db mice during a 122 day treatment period. The relative food consumption per mouse is significantly decreased in group B and C (p<0.001).

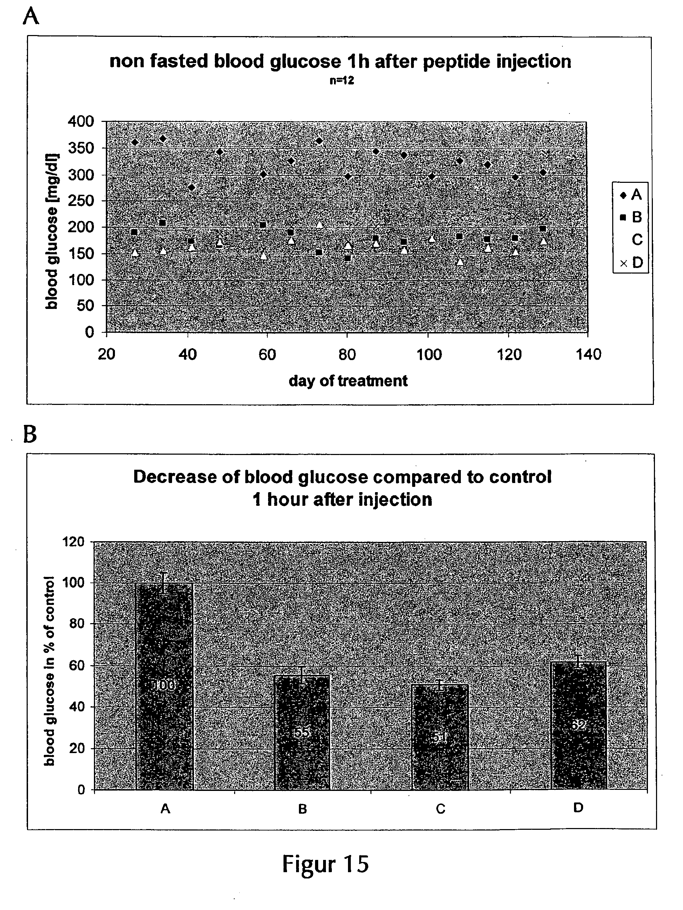

Blood glucose (4) levels were determined in a variety of situations. *Non-fasted blood glucose level* (1 hour after peptide injection) is shown in Fig. 15A, i.e. the effect of a treatment with saline (A), CM1 (B), CM3-ANA01 (C) or exendin (D) on the non-fasted blood glucose levels. Blood glucose was measured from a tail bleed 1 hour after s.c. injection of saline (A) or the peptides CM1 (B), CM3-ANA01 (C) or exendin (D) in a concentration of 24nmol/kg. Compared to control non fasted blood glucose level 1h after s.c. peptide injection is reduced to 55% in group B, 51% in group C and 60% in group D (Fig. 15B). The blood glucose drecrease in the treated groups treated groups is highly significant (p < 0.0001).

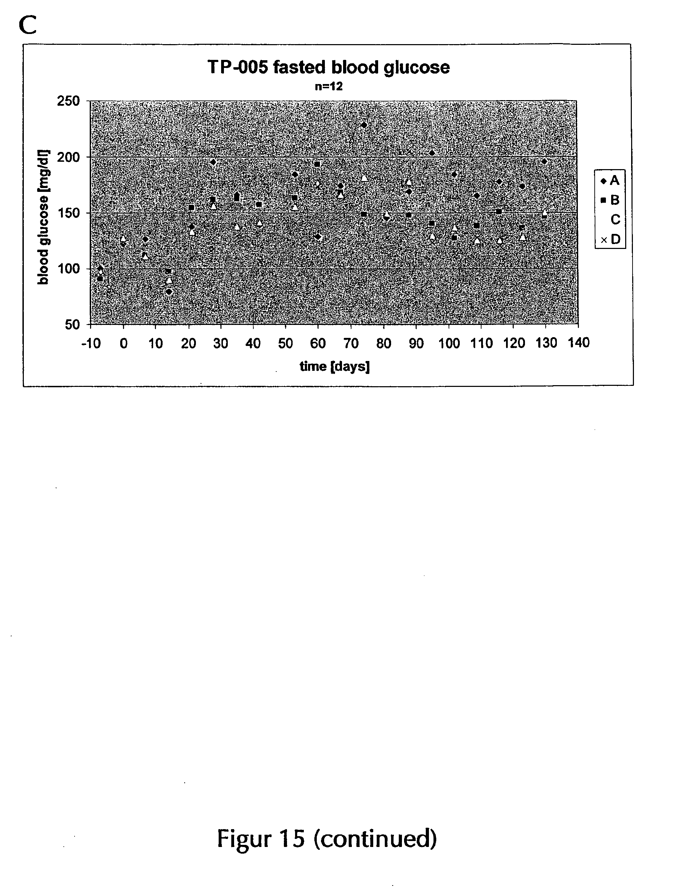

Fasted blood glucose level (18 hours after peptide injection) are shown in Fig. 15C, i.e. the effect of a treatment with saline (A), CM1 (B), CM3-ANA01 (C) or exendin (D) on the fasted blood glucose levels. Blood glucose was measured from a tail bleed after a 12-hour overnight fast 18 hours after s.c. injection of the test substances. Fasted blood glucose level 18h after peptide injection is reduced in the CM1 and CM3-ANA01 group.

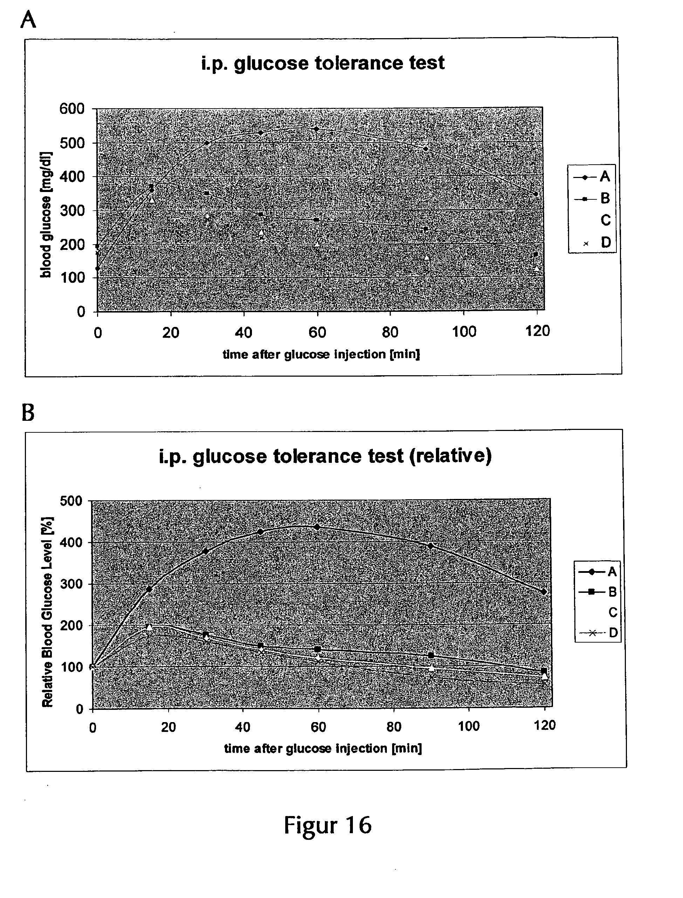

An i.p. glucose tolerance test (5) (IPGTT) was conducted after 8 weeks of treatment on mice of the groups A-D. The basal blood glucose value (0 min) of 12 hour fasted animals has been determined by tail bleed followed by an s.c. injection of the test substance and an i.p. injection of 20% glucose solution (1g glucose per kg). Blood glucose has been further on determined 15, 30, 45, 60, 90 and 120 minutes after glucose injection. A significant normalization of glucose tolerance was shown in all treated groups (Fig. 16A). In Fig. 16A absolute blood glucose levels during an i.p. glucose tolerance test (IPGTT) after 8 weeks of treatment with saline (A), CM1 (B), CM3-ANA01 (C) or exendin (D) is shown (mean values of each group (n=12)).

Another presentation of the data presented by Fig. 16A of the blood glucose tolerance test is given in Fig. 16B. *Relative* blood glucose levels during an i.p. glucose tolerance test (IPGTT) after 8 weeks of treatment with saline (A), CM1 (B), CM3-ANA01 (C) or exendin (D) normalized on the starting blood glucose level (100%).

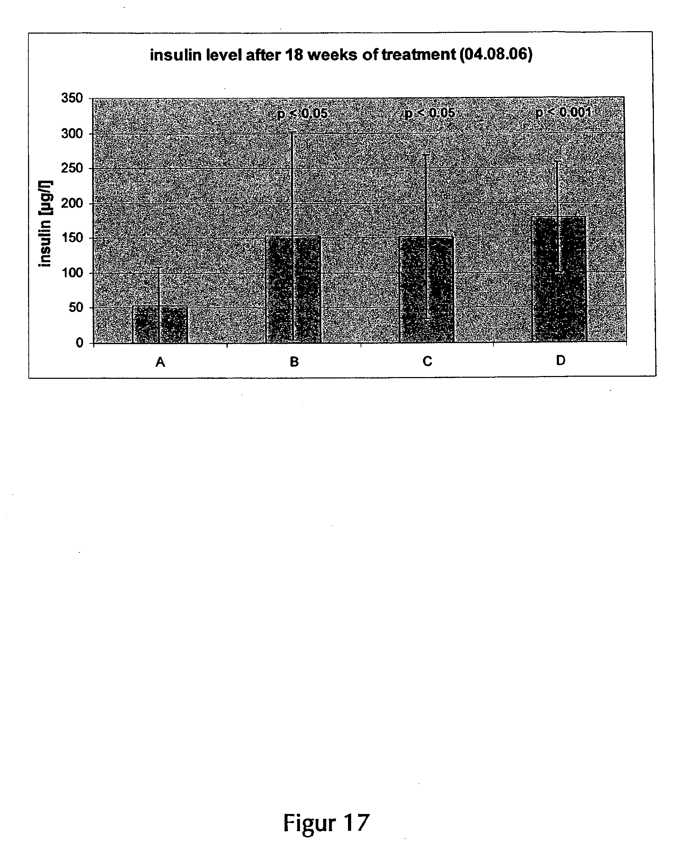

Insulin levels (6) were determined from mice after a 12 hour overnight fastening period. After 18 weeks of treatment, all mice of the treated groups produce significantly more insulin than the non-treated control. Fig. 17 presents the data for serum insulin levels in mice after 18 week treatment with 0.9% saline (Group A), CM1 peptide (group B), CM3-ANA01 peptide (group C) or exendin-4 (group D). Directly after sampling blood samples have been treated with a protease inhibitor cocktail to avoid insulin degradation. The serum was analysed for insulin with the *Insulin Mouse Uitrasensitive ELISA* (Cat.# EIA-3440, DRG). Significance has been determine with Student's t-test.

Glycosylated hemoglobin (7) is formed by excess plasma glucose binding to hemoglobin in red blood cells (RBC). Since RBCs have a 120-day lifespan, measurements of the glycolylated hemoglobin gives a longer-term indication of glucose control and is seen as a better indicator than plasma glucose levels. Whole blood was collected from the retro-orbital sinus and analyzed with the *Enzymatic HbA1c Test Kit* (#DZ121A, Diazyme) to determine the glycosylated hemoglobin levels. Using glycosylated hemoglobin as a parameter, a significant reduction in the increase was seen in all treated groups.

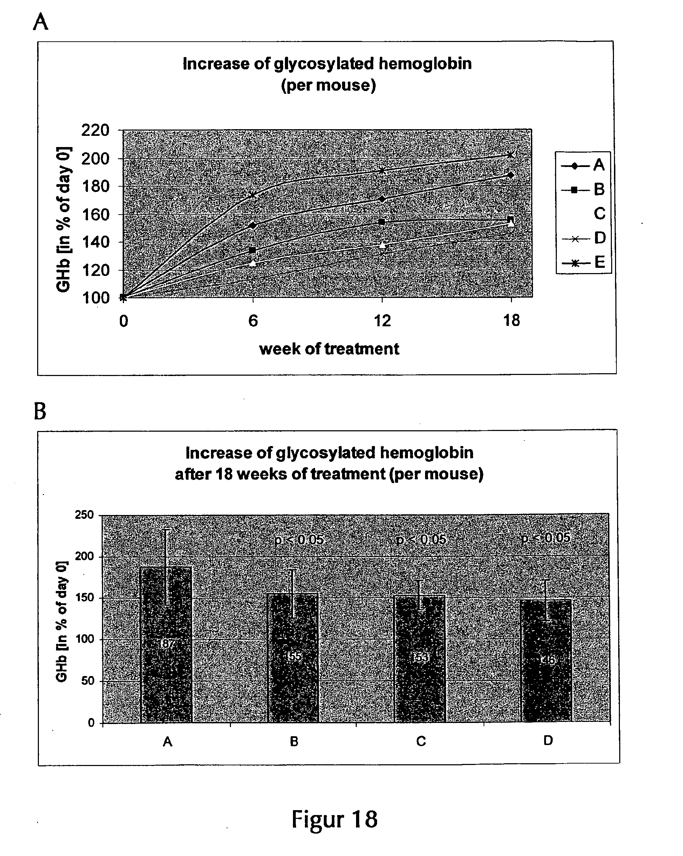

Fig. 18A shows the relative increase of glycosylated hemoglobin (GHb) in whole blood samples after 6, 12 and 18 weeks of treatment with saline (A), CM1 (B), CM3-ANA01 (C) or exendin (D). Fig. 18B shows the relative increase of glycosylated hemoglobin (GHb) in whole blood samples after 18 weeks of treatment with saline (A), CM1 (B), CM3-ANA01 (C) or exendin (D). The mean value of all animals per group (n=12) is given. The increase of the glycosylated hemoglobin levels of all treated groups are significantly lower than the control.

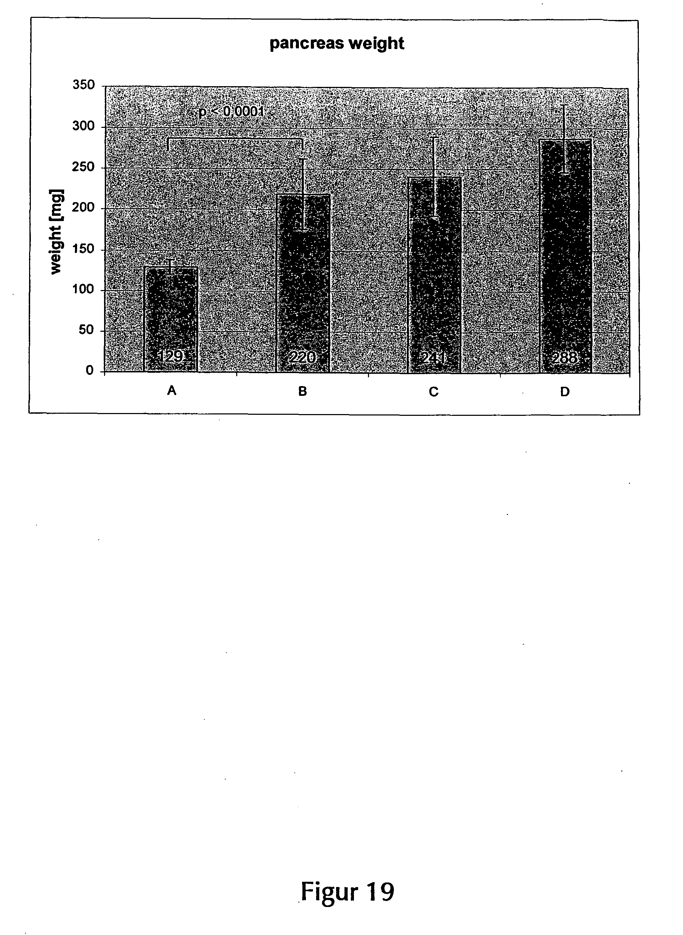

Pathology (8) of the mice treated was conducted. Necropsy revealed no differences in the organs of the treated groups compared to the non-treated group A, except the pancreas volume. Pancreas weight was determined and revealed to be highly significant higher in all treated groups. Figure 19 presents the pancreas weight determined at day of scarification of the animals after 18 weeks of treatment with 0.9% saline (Group A, n=11), CM1 peptide (group B, n=12), CM3-ANA01 peptide (group C, n=12) or exendin-4 (group D, n=12).

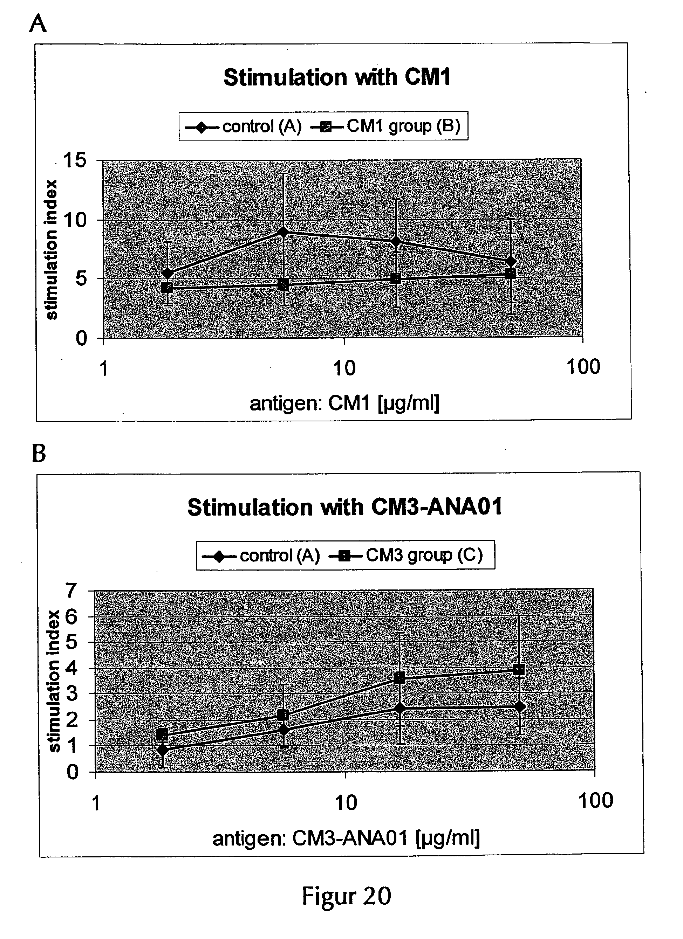

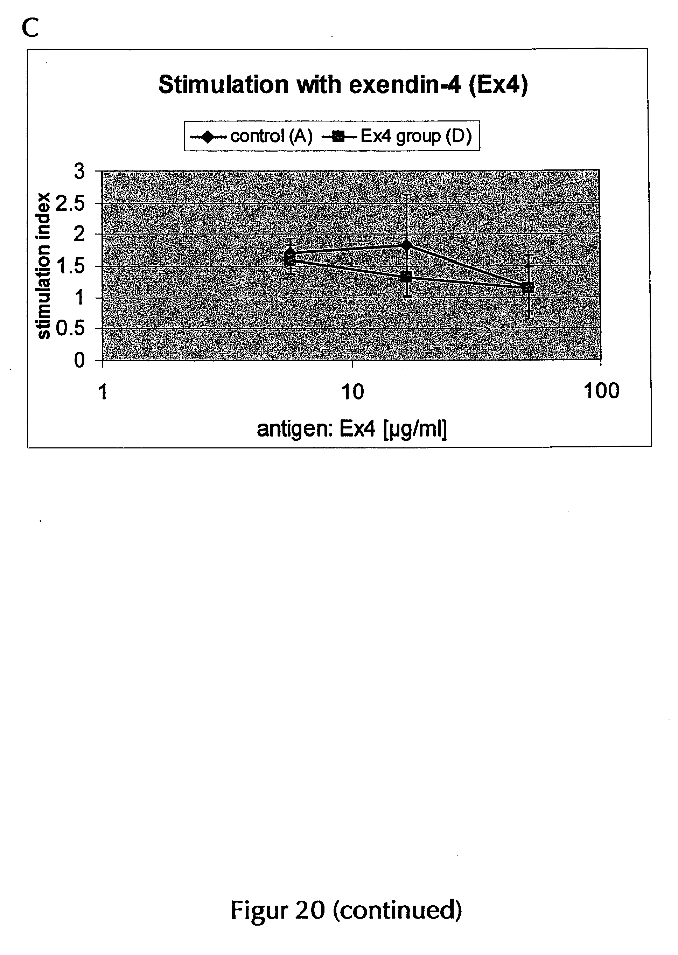

To evaluate potential immunological effects of the tested substances, type IV immunogenicity (9) was examined by T cell restimulation. Therefore, the spleen of 8 animals per group was explanted, T-cells isolated and restimulated with different concentrations of the corresponding test substance. Compared to non-treated controls no significant increase in peptide restimulated T-cell proliferation was found. Figure 20 shows the *in vitro* recall response of spleen cells to different concentrations of the test substances CM1 (Fig. 20A) and CM3-ANA01 (Fig. 20B) and the reference substance exendin-4 (Fig. 20C). Using a non-radioactive cell proliferation assay (Cell Proliferation ELISA, BrdU, chemiluminescent) the *in vitro* recall response of spleen cells of mice of group A to D to 3-fold dilutions of the test substances CM1 and CM3-ANA01 and the reference substance exendin-4, starting at 50 µg/ml, were measured in triplicates of individual mice. The stimulation index (SI) was calculated as the quotient of the response in the presence of the respective peptide and the response in the absence of peptides. Mean values ± standard deviations of the respective treated group and control group are shown (group A: n=3, group B: n=6, group C: n=7, group D: n=6). For the calculation of the mean values only mice, with a SI of more than 6.5 in the positive control cultures with 5 µg/ml Con A were analysed.

The long-term study in diabetic mice further supports the efficacy of the inventive C-terminally elongated peptides. In all parameters tested (body weight, food consumption, blood glucose levels, glycosylated hemoglobin, glucose tolerance, insulin secretion, pancreas weight) the C-terminally elongated peptides revealed a significant therapeutic effect. Taking in account the homology of the C-terminally elongated CellMed peptides with the endogenously occurring sequence it was shown that these inventive peptides are advantageous in terms of immunogenicity compared to non-mammalian exendin-4. Data from an immunogenicity study in mice support the absence of any clinically relevant immunogenicity of the CM1 peptide.

### Example 13

### Stability testing

The GLP-1 analogue CM3 (GLP-1 elongated by IP2, SEQ ID No. 8) has a molecular weight of only 5 kDa. Serum levels therefore rapidly decrease due to a high renal clearance rate. In order to decrease renal clearance PEG polymers are coupled C-terminally to a cysteine elongation of the peptide.

For the PEGylation, the peptide CM3-ANA08 was based on the following amino acid sequence: CM3 (46 aa):
HAEGTFTSDVSSYLEGQAAKEFIAWLVKGRGRRDFPEEVAIAEELG (SEQ ID No. 8),
which was then C-terminally elongated by a cysteine residue according to standard methods in the protein chemistry resulting in CM3-ANA08 (HAEGTFTSDVSSYLEGQA-AKEFIAWLVKGRGRRDFPEEVAIAEELGC).

CM3-ANA08 was covalently conjugated to an unbranched 40 kDa mPEG maleimido propionamide (Dow Chemicals, 008,016) using free thiol group of the C-terminal cysteine. The coupling was done according to standard methods. The obtained fusion peptide according to the present invention is designated PEG-CM3-ANA08.

### In vitro plasma stability testing (40 kDa PEG-CM3-ANA-08)

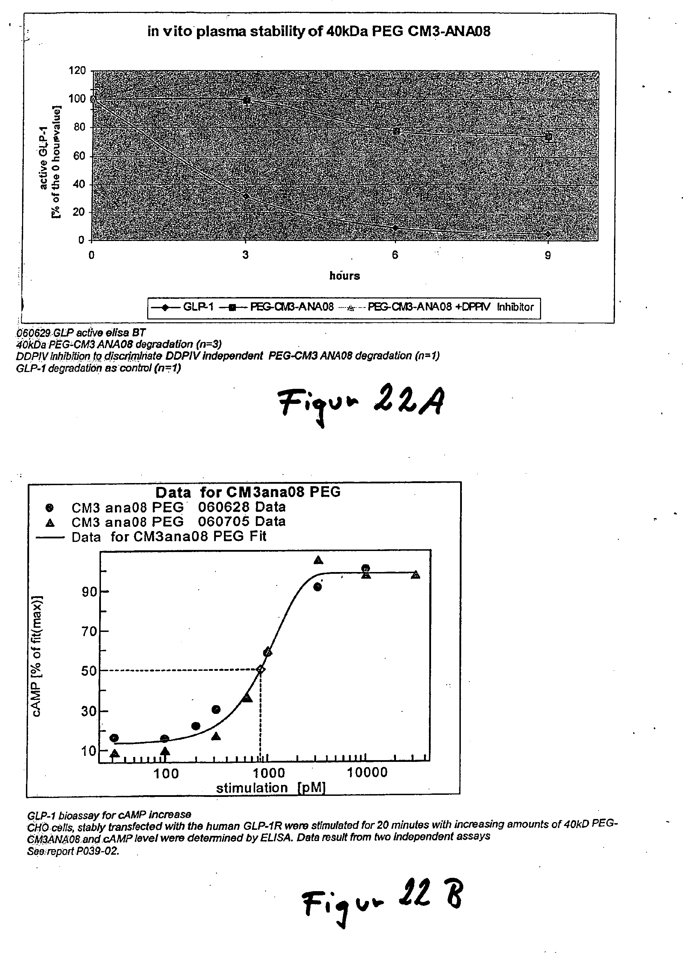

In vitro plasma stability testing was done according to standard methods, the results are shown in FIG. 22A.

The in vitro plasma stability of the PEGylated CM3-ANA08 is slightly higher than for the unmodified CM3 (9h value: 63 %) and slightly lower than CM3-ANA01 (HAEGTFTSDVSSYLEGQAAKEFIAWLVKGRGAADFPEEVAIAEELG) (9h value: 84 %). The slightly elevated 3 hour values may represent a termperature and buffer dependent remodelling of the PEGylated CM3-ANA08 peptide resulting in a better cognition of the ELISA antibody.

T 0 hours only 19 % of the peptide can be detected in the GLP-1 active ELISA - an indication that the PEG is masking or sterically hindering ELISA antibodies epitope.

### GLP-1 Bioassay cAMP increase

The in vitro bioactivity was tested in a GLP-1 receptor transfected CHO cell line by determining the half maximum effective dose (ED₅₀).

### The data are represented in FIG. 22B

The ED₅₀ of 847 pM is approximately 5 times higher than CM3 (IC₅₀ = 187 pM) or CM3-ANA01 (IC₅₀ = 159 pM)

Summing up, a 40 kDa PEG CM3 ANA08 peptide has shown to have a comparable in vitro plasma stability like the unmodified peptide but a 5 times lowered in vitro bioactivity. In vivo bioactivity has been shown in diabetic db/db mice, leading to a significant blood glucose decrease in the treated versus non-treated group. No signs of toxicity have been observed.

### Example 14

### Oral formulation

### Preparation of Dosing Solutions

The PEG-CM3-ANA08 (i.e. an inventive conjugate complex formed by an inventive fusion peptide coupled to PEG) of Example 13 was used. To prepare stock solutions, this conjugate complex was dissolved in deionized water (pH6.5) to obtain a concentration of 8 mg/ml. Stock solutions were kept frozen at -20°C. in 0.5-ml aliquots until used. For dosing solutions, the delivery agents of figure 21 were dissolved in deionized water to obtain a final concentration of 200 mg/ml (oral dosing) or 100 mg/ml (intracolonic dosing). The free acid form of delivery agent was converted to the sodium salt by adding one equivalent of sodium hydroxide. Solutions were vortexed, sonicated, and heated, and if necessary, additional sodium hydroxide was added in [mu] quantities to achieve uniform solubility. A specified quantity of rhe conjugate complex stock was then added to the delivery agent solution to obtain a final concentration of 1 or 0.3 mg/ml (oral) or 0.6 mg/ml (intracolonic). After solubilization and "drug" addition, solutions were brought to final volume by the addition of deionized water.

### Animals and Dosing

The conjugate complex was administered to male, Sprague-Dawley rats either alone or in combination with a delivery agent. Typically, rats were fasted for 18-24 hours prior to dosing. On the day of the experiment, rats were weighed and then anesthestized with a combination of ketamine (44 mg/kg) and thorazine (1.5 mg/kg). Anesthesia was administered by intramuscular (IM) injection (26-gauge needle) into the hindleg in a volume of 0.5 mls/kg body weight. A tail or toe pinch was used to determine the level of anesthesia. Once anesthetized, rats were administered the inventive conjugate molecule (s. above) alone or in combination with a delivery agent. Routes of delivery were either oral (PO) or intracolonic (IC). For PO & IC dosing, a Rusch 8 French catheter was cut to 11 cm in length and adapted to fit a 1-ml syringe. The syringe was filled with dosing solution and the catheter was wiped dry of excess solution. For PO dosing, the catheter was inserted into the rat mouth and fed down the esophagus (10.0 cm). The dosing solution was delivered by pressing the syringe plunger while holding the rat in an upright position. The doses of the delivery agent and GLP-1 analogue were 200 mg/kg and 1 or 0.3 mg/kg, respectively. The dose volume was 1 ml/kg. For IC dosing, the catheter was inserted into the rectum and fed into the colon (7.5 cm). The dosing solution was delivered by pressing the syringe plunger while holding the rat up by the tail. The doses of delivery agent and conjugate molecule were 50 mg/kg and 0.3 mg/kg, respectively. The dose volume was 0.5 ml/kg.

### Sample Collection and Handling

During blood sample collection, rats may have received additional injections of ketamine/thorazine in order to maintain anesthesia. For blood sampling, a 22-gauge needle was inserted into the tail artery. Typically, blood samples were collected prior to dosing (time 0) and at 5, 15, 30, 45, and 60 minutes after dosing. Samples were collected into capiject tubes containing EDTA and a DDP-IV inhibitor, and placed on wet ice until centrifugation. After all samples were collected, tubes were centrifuged at 10,000 rpm for 4 minutes at 4 C. in order to separate the plasma. Plasma was collected into eppendorf tubes and frozen at -20 °C. until assayed.

### Bioanalytical Method and Data Analysis

Briefly, concentrations of the conjugate complex were quantified in rat plasma using a radioimmunoassay (RIA). The % B/B0 (% bound drug/unbound drug) was derived for the assay standards and plotted on a log/log scale. A 4PL logistic fit was used to fit the standard curve. The sample concentrations were calculated by reading the % B/B0 of the samples off the standard curve. % B/B0 values from each time point were averaged (n=5) and plotted versus concentration. Finally, data were expressed as mean plasma concentration of conjugate complex versus time. The range of the assay was 0.1-12.8 ng/ml.

The results of the experiments have demonstrated that the oral application of PEG-CM3-ANA08 (conjugate complex) together with the delivery agents of figure 21 provides an improved (i.e. increased) bioavailability compared to the administration of PEG-CM3-ANA08 without delivery agent.

## Claims

1. The conjugate molecule containing a fusion peptide comprising as component (I) N-terminally a sequence according to formula II
Xaa7-XaaB-Glu-Gly-Thr-Phe-Thr-Ser-Asp-Xaal6-Ser-Xaa18-Xaa19-Xaa20-Glu-Xaa22-Xaa23-Ala-Xaa25-Xaa26-Xaa27-Phe-Ile-Xaa3o-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohis6dine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is is Ser, Lys or Arg; Xaa19 is Tyr or Gln ; Xaa20 is Leu or Met; Xaa22 is Gly, Glu or Aib; Xaa23 is Gln, Glu, Lys or Arg ; Xaa25 is Ala or Val ; Xaa26 is Lys, Glu or Arg; Xaa27 is Glu or Leu; Xaa30 isAla, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent.
or formula III
Xaa7-XaaB-Glu-Gly-Thr-Phe-Thr-Ser-Asp-Val-Ser XaaB-Tyr-Leu-Glu-Xaa22-Xaa23-Ala-Ala-Xaa26-Glu-Phe-lie-Xaa3O-Trp-Leu-Val-Xaa34-Xaa35-Xaa36-Xaa37,
wherein Xaa7 is L-histidine, D-histidine, desamino-histidine, 2-amino-histidine, - hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gin, Glu, Lys or Arg ; Xaa26 is Lys, Glu or Arg;Xaa30 isAla, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent.
as component (II) C-terminally a peptide sequence of at least 9 amino acids or a functional fragment, variant or derivative thereof, and a synthetic or natural polymer.

2. The conjugate molecule according to claim 1, wherein the synthetic polymer is prepared from a momomer of the following formula 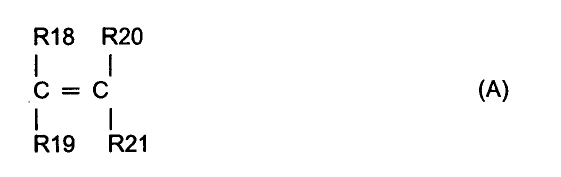 in which
R18 is selected from hydrogen, halogen, C1-4 alkyl and groups COOR22 in which R22 is hydrogen and C1-4 alkyl;
R19 is selected from hydrogen, halogen and C1-4 alkyl;
R20 is selected from hydrogen, halogen, C1-4 alkyl and groups COOR22 provided that R18 and R20 are not both COOR22; and
R21 is a C1-10 alkyl, a C1-20 alkoxycarbonyl, a mono- or di-(C1-20 alkyl) amino carbonyl, a C6-20 aryl (including alkaryl), a C7-20 aralkyl, a C6-20 aryloxycarbonyl, a C1-20 aralkyloxycarbonyl, a C6-20 arylamino carbonyl, a C7-20 aralkyl-amino, a hydroxyl or a C2-10 acyloxy group, any of which may have one or more substituents selected from halogen atoms, alkoxy, oligo-alkoxy, aryloxy, acyloxy, aclamino, amine (including mono and di-alkyl amino and trialkylammonium), carboxyl, sulphonyl, phosphoryl, phosphino, (including mono- and di-alkyl phosphine and tri-alkylphosphonium), zwitterionic and hydroxyl groups;or
R21 and R20 or R21 and R19 may together form -CONR23C0 in which R23 is a C1-20 alkyl group.

3. The conjugate molecule according to claim 1, wherein the synthetic polymer has the following general formula (B) or (C) 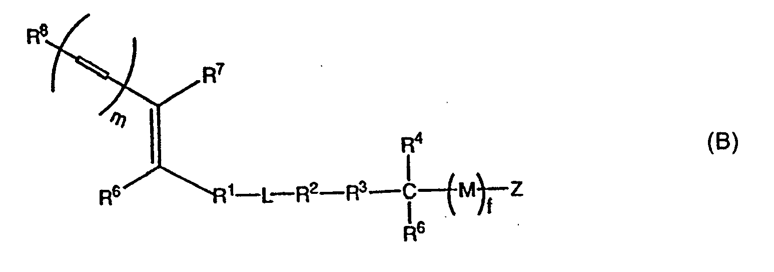 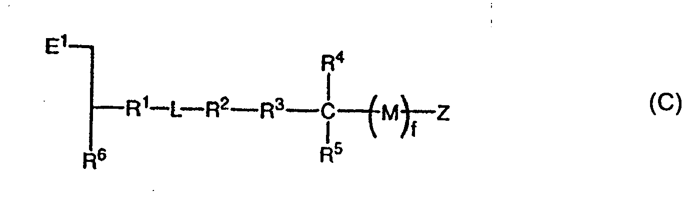 wherein
R1 is a electron withdrawing group known in the art;
R2 is selected from C=O, C(=O)NR9 or a bond; wherein R9 is H or a C1-4 alkyl;
R3 is selected from the group consisting of a C1-C20 alkylene; C3-C8 cycloalkylene; C(=C)R10, C(=O)NR11, C1-C20 alkoxy, C2-C20 alkenylene, C2-C20 alkenyl oxiranylene; arylene, heterocyclene, aralkylene; in which 1 to all of the hydrogen atoms are replaced with halogen; C1-C6 alkyl susbtituted with 1 to 2 substituents selected from the group consisting of C1-C4 alkoxy, aryl, heterocyclyl, C(=O)R10, C(=0)NR11R12. oxiranyl and glycidyl;
R10 is alkylene of from 1 to 20 carbon atoms, alkoxy from 1 to 20 carbon atoms, oligo(alkoxy) in which each alkoxy group has from 1 to 3 carbon atoms, aryloxy or heterocycyloxy; and of which groups may have substituents selected from optionally substituted alkoxy, oligoalkoxy, amino (including mono- and di-alkyl amino and trialkyl ammonium, which alkyl groups, in turn, may have substituents selected from acyl, alkoxycarbonyl, alkenoxycarbonyl, aryl and hydroxy) and hydroxyl groups;
R11 and R12 are independently H or alkyl of from 1 to 20 carbon atom or R11 and R12 may be joined together to form an alkanediyl group of from 2 to 5 carbon atoms, thus froming a 3- to 6-membered ring;
R4 and R5 are each independently selected from H, Z, halogen, C1-C20 alkyl, C3-C8 cycloalkyl, OH, CN, C2-C20 alkenyl, C2-C20 alkenyl oxiranyl, C(=O)R13, glycidyl, aryl, heterocyclyl, arylkyl, aralkenyl, in which 1 to all of the hydrogen atoms are replaced with halogen, C1-C6 alkyl substituted with 1 to 2 substituents selected from the group consisting of C1-C4 alkoxy, aryl, heterocyclyl, C(=O)R10, C(=O)NR11R12, oxiranyl and glycidyl;
where R13 is alkyl of from 1 to 20 carbon atoms, alkoxy of from 1 to 20 carbon atoms, oligo(alkoxy) in which each alkoxy group has 1 to 3 carbon atoms, aryloxy or heterocyclyloxy any of which groups may have substituents selected from optionally susbstituted alkoxy, oligoalkoxy, amino (including mono- and di-alkyl amino and trialkyl ammonium, which alkyl groups, in turn may have susbtituents selevted from acyl, alkoxycarbonyl, alkenoxycarbonyl, and aryl and hydroxy) and hydroxyl groups; and
L is a linking group;
Z is selected from the group consisting of Cl, Br, I, OR14, SR15, SeR15, OP(=O)R15, OP(=O)(=OR15)₂, O-N(R15)₂, and S-C(=S)N(R15)₂, where R14 is alkyl of from 1 to 20 carbon atoms in which each of the hydrogen atoms may be independently replaced by halide, R15 is aryl or a straight or branched C1-C20 alkyl group, and where an N(R15)₂ group is present, the two R15 groups may be joined to form a 5- or 6-membered heterocyclic ring;
R6 is CH₂E2 , H or C1-C4 alkyl;
R7 and R8 are each independently selected from H and C1-C4 alkyl;
m is 0-4;
f is 1 to 500, preferably 5 to 50; and
groups M are the same or different and are residues derived from ethylenically unsaturated monomers, in particular the monomers as defined in claim 2.

4. The conjugate molecule according to claims 1 to 3, wherein the synthetic polymer is selected from alkylene glycols, such as polyethylene glycol (PEG), polypropylene glycol (PPG), copolymers of ethylene glycol and propylene glycol, polyoxyethylated polyol, polyolefinic alcohol, polyvinylpyrrolidone, polyhydroxyalkyl methacrylamide, polyhydroxyalkyl methacrylate, such as polyhydroxyethylen methycrylate, polyacrylate, polysaccharides, poly([alpha]-hydroxy acid), polyvinyl alcohol, polyphosphazene, polyoxazoline, poly(N-acryloylmorpholine), polyvinylethyl ether, polyvinlyacetale, polylactic glycolic acid, polylactic acid , lipid polymer, chitin, hyaluronuic acid, polyurethyne, polysialic acid cellulose triacetat, cellulose nitrate and combinations of any of the foregoing.

5. The conjugate molecule according to claims 1 to 4, wherein the synthetic polymer has a average molecular weigth from 100 to 200 000 Dalton.

6. The conjugate molecule according to claim 5, wherein the average molecular weigth is 1 000 to 40 000 Dalton.

7. The conjugate molecule according to claims 1 to 6, wherein the naturally occuring polymer is a protein or a peptide.

8. The conjugate molecule according to claim 7, wherein the protein is albumin or transferrin.

9. The conjugate molecule according to claims 1 to 8, wherein the polymer is covalently bound to either component (I), component (II) or both of them.

10. The conjugate molecule according to claims 1 to 9, wherein the polymer is bound to the N-terminus, the C-terminus and/or to amino acid side chains of the fusion peptide.

11. The conjugate molecule according to claims 1 to 10, wherein the conjugate molecule contains 1 to 3, preferably 1, polymer constituent.

12. The conjugate molecule according to any of the preceding claims comprising as component (I) N-terminally a GLP-1(7-35, 7-36 or 7-37) sequence and as component (II) C-terminally a peptide sequence of at least 9 amino acids or a functional fragment, variant or derivative thereof.

13. The conjugate molecule according to any of the preceding claims comprising as component (I) N-terminally a sequence according to formula I
His-Ala-Glu-Gly-Tfir-Phe-Thr-Ser-Asp-Val-Ser-8.er-Tyr-Leu-Glu-Gly-Gin-Ala-Ala-Lys-Glu-Phe-Ile-Ala-Trp-Leu-Val-Lys-Gly-Arg-X (I), wherein X is NH2 or Gly-OH,
and as component (II) C-terminally a peptide sequence of at least 9 amino acids or a functional fragment, variant or derivative thereof.

14. The conjugate molecule according to claims 12 or 13, wherein component (I) contains a sequence having at least 80 % sequence homology with SEQ ID No.: 1.

15. The conjugate molecule according to any of the preceding claims, wherein component (II) is a peptide sequence forming a β-turn like structure.

16. The conjugate molecule according to any of the preceding claims, wherein component (II) is a peptide sequence containing at least one alanine or proline residue.

17. The conjugate molecule according to any of the preceding claims, wherein component (II) is a peptide sequence containing a tetramer with β turn forming properties, e.g. having a proline residue at position 2 of that tetramer.

18. The conjugate molecule according to any of the preceding claims, wherein component (II) is a peptide sequence containing a sequence motif selected from the group consisting of VAIA, IAEE, PEEV, AEEV, EELG, AAAA, AAVA, AALG, DFPE, AADX, AXDX, and XADX, wherein X represents any amino acid.

19. The conjugate molecule according to any of the preceding claims, wherein component (II) is a peptide sequence being linked to the C-terminus of component (I) by its N-terminal sequence motif selected from the group consisting of AA, XA, AX, RR, RX, and XR.

20. The conjugate molecule according to any of the preceding claims, wherein component (II) is a peptide sequence containing the sequence motif of SEQ ID No.: 25 (DFPEEVA) or containing a sequence having at least 80% sequence homology with the SEQ ID No.: 25.

21. The conjugate molecule according to any of the preceding claims, wherein component (II) is a peptide sequence containing a sequence selected from the group consisting of SEQ ID No.: 22 (RRDFPEEVAI) and SEQ ID No.: 26 (AADFPEEVAI) or containing a sequence having at least 80% sequence homology with SEQ ID No.: 22 or SEQ ID No.: 26.

22. The conjugate molecule according to any of the preceding claims, wherein component (II) is a peptide sequence containing a sequence selected from a group consisting of SEQ ID No.: 23 (RRDFPEEVAIVEEL), SEQ ID No. 24 (RRDFPEEVAIAEEL), SEQ ID No.: 27 (AADFPEEVAIVEEL), and SEQ ID No.: 28 (AADFPEEVAIAEEL), or a sequence having at least 80% sequence homology with any of SEQ ID Nos.: 23, 24, 27, or 28.

23. The conjugate molecule according to any of the preceding claims, wherein component (II) is a peptide sequence containing a sequence selected from the group consisting of SEQ ID No.: 2 (RRDFPEEVAIVEELG), SEQ ID No. 3 (RRDFPEEVAIAEELG), SEQ ID No.: 29 (AADFPEEVAIVEELG), and SEQ ID NO.: 30 (AADFPEEVAIAEELG), or a sequence having at least 80% sequence homology with SEQ ID Nos.: 2, 3, 29 or 30.

24. The conjugate molecule according to any of the preceding claims, wherein component (II) is a peptide sequence having 9 to 30, preferably 9 to 20, and most preferably 9 to 15 amino acids.

25. The conjugate molecule according to any of the preceding claims, wherein component (I) and component (II) are directly linked or linked via a linker sequence.

26. The conjugate molecule according to claim 25, wherein the linker sequence has a length of 1 to 10 amino acids.

27. The conjugate molecule according to any of the preceding claims, wherein the fusion peptide contains a sequence selected from the group consisting of SEQ ID No.: 8 (HAEGTFTSDVSSYLEGQAAKEFIAWLVKGRGRRDFPEEVAIAEELG), SEQ ID No. 12 (HAEGTFTSDVSSYLEGQAAKEFIAWLVKGRGRRDF-PEEVAIVEELG), SEQ ID No.: 31 (HAEGTFTSDVSSYLEGQAAKEFIAWLVKGRGAADFPEEVAIAEELG), SEQ ID No.: 32 (HAEGTFTSDVSSYLEGQAAKEFIAWLVKGRGRRDFAEEVAIAEELG), SEQ ID No.: 33 (HAEGTFTSDVSSYLEGQAAKEFIAWLVKGRGRRDAAAAVAIAEELG), SEQ ID No.: 34 (HAEGTFTSDVSSYLEGQAAKE-FIAWLVKGRGAADAAAAVAIAAALG), SEQ ID No.: 35 (HAEGTFTSDVSSYLEGQAAKEFIAWLVKGRGRRDFP), SEQ ID No.: 36 (HAEGTFTSDVSSYLEGQAAKEFIAWLVKGRGRRDFPEEVA), SEQ ID No.: 37 (HAEGTFTSDVSSYLEGQAAKEFIAWLVKGRGRRDFPEEVAIAEELGRRHAC), SEQ ID No.: 38 (HAEGTFTSDVSSYLEGQAAKEFIAWLVKGRGAADFPEEVAIVEELG), SEQ ID No.: 39 (HAEGTFTSDVSSYLEGQAAKEFIAWLVKGRGRRDFAEEVAIVEELG), SEQ ID No.: 40 (HAEGTFTSDVSSYLEGQAAKEFIAWLVKGRGRRDAAAAVAIVEELG), SEQ ID No.: 41 (HAEGTFTSDVSSYLEGQAAKEFIAWLVKGRGAADAAAAVAIVAALG), and SEQ ID No.: 42 (HAEGTFTSDVSSYLEGQAAKEFIAWLVKGRGRRDFPEEVAIVEELGRRHAC), or a sequence having at least 80% sequence homology with SEQ ID Nos.: 8, 12.

28. The conjugate molecule according to any of the preceding claims, wherein the fusion peptide contains another component (III) linked to the C-terminus of component (II) and/or to the N-terminus of component (I).

29. The conjugate molecule according to claim 28, wherein component (III) comprises at least four amino acid residues, preferably at least 10 additional amino acid residues, more preferably at least 20, or at least 30.

30. The conjugate molecule according to claim 28 or 29, wherein component (III) comprises at least 4, preferably at least 10, more preferably at least 20 additional amino acid residues of the N-terminal sequence of GLP-2 as in proglucagon or of GLP-1 (7-37).

31. The conjugate molecule according to any of claims 28 to 30, wherein component (III) contains the sequence of SEQ ID Nos.: 4 or 5 or a sequence having at least 80% sequence homology with SEQ ID Nos.: 4 or 5.

32. The conjugate molecule according to any of claims 28 to 31, wherein the fusion peptide contains a peptide sequence selected from a group consisting of: SEQ ID No. 6, SEQ ID No. 7, SEQ ID No. 10 and SEQ ID No. 11 or a sequence having at least 80% sequence homology with SEQ ID Nos.: 6, 7, 10, or 11.

33. The conjugate molecule according to any of the preceding claims, wherein at least one of the amino acids of the fusion peptide is derivatized by a covalent modification of a side chain of a naturally occurring amino acid, by a modification of the peptide backbone, by modification of the NH₂ or carboxy terminal groups.

34. The conjugate molecule according to claim 33, wherein at least one of the amino acids of the fusion peptide is derivatized by a lipyl or carbohydrate group.

35. The conjugate molecule according to claim 33 or 34, wherein the N-terminal His residue of GLP-1 (GLP-1(7)) of component (I) of the fusion protein is chemically modified at its NH2 terminus and/or at its histidyl side chain, in particular by a hydrophobic moiety.

36. The conjugate molecule according to any of the preceding claims, wherein the amino acid sequence of components (I), (II) and/or (III) is reversed and wherein said amino acid sequence(s) is at least partially composed of D amino acid isomers.

37. Method of producing a conjugate molecule according to any of the preceding claims by (a) solid state peptide synthesis of the fusion peptide, (b) reacting the product of step (a) with one or more polymer constituent(s) and (c), optionally, derivatizing the fusion peptide by an additional reaction.

38. A method for producing a fusion peptide as portion of the conjugate molecule according to any of the preceding claims 1 to 32 in which a micro-organism transformed to include a nucleic acid encoding the fusion peptide is fermented and the fusion peptide is recovered.

39. The method of producing a fusion peptide according to claim 38 in which animal cells are grown under conditions in which the protein is exported from the cells.

40. Conjugate molecule according to any of the preceding claims 1 to 36 as a medicament for use in human or animal therapy.

41. Use of a conjugate molecule according to any of the preceding claims 1 to 36 for the manufacture of a medicament for the treatment of diabetes mellitus type I or type II, insulin resistance, weight disorders and diseases or conditions associated thereto.

42. Use of a conjugate molecule according to any of the preceding claims 1 to 36 for the manufacture of a medicament for the treatment of neurodegenerative disorders and diseases or conditions associated thereto.

43. Use of a conjugate molecule according to any of the preceding claims 1 to 36 for the manufacture of a medicament for the treatment of disorders and diseases or conditions associated to apoptosis.

44. Use of a conjugate molecule according to any of claims 41 to 43, wherein the medicament is formulated for oral, nasal or pulmonary application.

45. Use of conjugate molecule according to claim 44, wherein the medicament for oral application contains a delivery agent.
